(19) Europäisches Patentamt
European Patent Office
Office européen des brevets

(11) **EP 2 947 074 A1**

(12) **EUROPEAN PATENT APPLICATION**
published in accordance with Art. 153(4) EPC

(43) Date of publication:
**25.11.2015 Bulletin 2015/48**

(21) Application number: **14740983.3**

(22) Date of filing: **15.01.2014**

(51) Int Cl.:
***C07D 281/16*** *(2006.01)*
***C07D 495/14*** *(2006.01)*
***C07K 14/555*** *(2006.01)*
***C12P 21/08*** *(2006.01)*
***A61K 31/554*** *(2006.01)*
***A61K 31/5517*** *(2006.01)*
***A61K 38/02*** *(2006.01)*
***A61P 35/00*** *(2006.01)*
***A61P 37/00*** *(2006.01)*

(86) International application number:
**PCT/RU2014/000015**

(87) International publication number:
**WO 2014/112898 (24.07.2014 Gazette 2014/30)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **16.01.2013 RU 2013101884**

(71) Applicants:
- **"Biointegrator" Limited Liability Company (OOO "Biointegrator") g. Khimki, Moscow Region 141400 (RU)**
- **Alla Chem, LLC. Carson City, NV 89701 (US)**

(72) Inventors:
- **DEMIN, Alexandre Viktorovich Moscow 119607 (RU)**
- **TKACHENKO, Sergey Evgenievich California, San Diego 92122 (US)**

(74) Representative: **Henrion, Oliver et al Patentanwälte Zellentin & Partner Rubensstrasse 30 67061 Ludwigshafen (DE)**

# (54) CONJUGATES AND SMALL MOLECULES WHICH INTERACT WITH THE CD16A RECEPTOR

(57) The invention is related to medicine, in particular, to oncology and immunology. The novel compounds of the general formula 1 or 2, exhibiting affinity for CD16a receptor have been proposed. There were also proposed novel modified proteins active towards CD16a receptor, selected from antibody or autogen conjugated by a modifying compound selected from compound of the general formula 1 or 2, which stimulate and direct antibody-dependent cellular cytotoxicity. The novel modified proteins (conjugates) could be used for destruction of definite targeted group of cells in organism, for example, cancerous cells or autoimmune lymphocytes. There were also proposed methods for conjugate preparation, pharmaceutical composition, and medicament, comprising modified proteins for treating oncology and autoimmune diseases.

EP 2 947 074 A1

## Description

### Field of invention

The invention relates to medicine, in particular to immunology and oncology, to novel compounds binding to CD16a receptor and to proteins (conjugates) modified by them which are used for inducing antibody-dependent cellular cytotoxicity and as a consequence of this removing cells of a particular target group, such as cancerous cells or autoimmune lymphocytes from the body. The invention also relates to a method for preparation of the above conjugates, pharmaceutical compositions and medicaments comprising modified proteins (conjugates) for treating oncology and autoimmune diseases.

### Background of the invention

Receptor FcγIIIa (CD16a) belongs to a group of receptors responsible for binding of Fc-fragment of antibodies. CD16a is expressed on the surface of NK-cells (killers) and macrophages and is responsible for induction of antibody-dependent cellular cytotoxicity (ADCC), interacting with Fc-fragment of antibody bound to the cell. ADCC, along with complement-dependent cytotoxicity (CDC) and apoptosis, is one of the main mechanisms of the destruction of cancer cells in the body. It is the cause of autoantibody mediated autoimmune diseases such as autoimmune polyendocrinopathy of the first type, autoimmune hemolytic anemia, idiopathic thrombocytopenia, hemolytic disease of newborns, etc. That is, antibody-dependent cellular cytotoxicity may play either positive or negative role in the development of pathologic processes in human body.

Autoimmune diseases - a group of diseases developing as a result of immunologic response generated against healthy tissues of organism and resulting in damage of these tissues. Currently immunosuppressants are used for treating autoimmune diseases which suppress immune system as a whole. Selective suppression of autoimmune response would significantly reduce the incidence of treatment side effects.

Antibodies are used rather long ago for targeted destruction of cancerous cells. Examples include rituximab, trastuzumab, cetuximab, and many other antibodies having a target on the cancer cells surface, and acting through a compliment-dependent cytotoxicity and antibody-dependent cellular cytotoxicity. It allows to use these unconjugated monoclonal antibodies as medicaments for treating cancerous diseases, for example, rituximab - for treating of CD20-positive B-cellular low-grade or follicular non-Hodgkin's lymphoma, trastuzumab - for treating advanced breast cancer. Successful usage of these medicaments relies not only on their efficacy but also on their outstanding safety profile (Grillo-Lopez A.-J et al. Semin. Oncol., 26, 1999, pp. 66-73).

They all have brought the possibility of new type therapy. But, in spite of the pronounced efficacy, about half of patients do not respond to rituximab therapy, and up to 60% of patients became resistant at refresher course of treatment. In the light of success associated with these medicaments there is currently a large interest in obtaining higher specific antibody activity than what is typically afforded by treatment with unconjugated antibodies.

It caused broadening of investigation of directed therapeutic effect of antibodies to surface antigens. The first direction of antibody modification has become the development of modified proteins (conjugates) antibody-drug conjugates for local delivery of cytotoxic or cytostatic remedies, that is medicaments which have already been used for destruction or inhibition of tumor cells at treatment of malignant tumor. [Payne, G. (2003) Cancer Cell 3:207-212; Trail et al. (2003) Cancer Immunol. Immunother. 52:328-337; Syrigos and Epenetos (1999) Anticancer Research 19:605-614; Niculescu-Duvaz and Springer (1997) Adv. Drug Del. Rev. 26:151-172; US 4,975,278]. These conjugates facilitate targeted delivery of medicament to tumors and its accumulation within the cells, adding to cytotoxic action of antibodies antitumor activity of cytotoxic or cytostatic medicaments. Examples of such conjugates are trastuzumab-DM1, the enhanced version of trastuzumab (Herceptin) (WO 201169074) and a series of conjugates with auristatins E (US 20120003248).

An alternative direction was to enhance the inherent cytotoxicity of antibodies by means of enhancing the interaction with receptors stipulating cytotoxicity. Roche company has developed obinutuzumab antibody exhibiting an increased binding to receptor CD16a. The effect was achieved by engineering of glycosylation of antibody. Obinutuzumab has ten times stronger antibody-dependent cellular cytotoxicity. (WO 2005044859, EA 015009).

Antibody-dependent cellular cytotoxicity - one of the main mechanisms of cytotoxic antibody action, which are bound to antigen on the surface of the targeted cell through variable domains, while the constant part is bound to CD16a receptor on the surface of killer cells. This intercellular contact leads to secretion of perforines and granzymes by killers. The first ones form pores in cellular membrane of the targeted cell, while the latter - activate caspases and other molecules of apoptosis. CD16a receptor is a member of a large family of Fc-receptors binding to the constant domain of antibody and differing in localization, function and the affinity for constant domain.

## Disclosure of the invention

In context of the invention, terms are generally defined as follows:

"**Alkyl**" means an aliphatic hydrocarbon straight or branched group with 1-12 carbon atoms in the chain. Branched group means that the alkyl chain has one or more "lower alkyl" substituents. Alkyl group may have one or more substituents of the same or different structure ("alkyl substituent") including halogen, alkenyloxy, cycloalkyl, aryl, heteroaryl, heterocyclyl, aroyl, cyano, hydroxy, alkoxy, carboxy, alkynyloxy, aralkoxy, aryloxy, aryloxycarbonyl, alkylthio, heteroarylthio, aralkylthio, arylsulfonyl, alkylsulfonylheteroaralkyloxy, annelated heteroarylcycloalkenyl, annelated heteroarylcycloalkyl, annelated heteroarylheterocyclenyl, annelated heteroarylheterocyclyl, annelated arylcycloalkenyl, annelated arylcycloalkyl, annelated arylheterocyclenyl, annelated arylheterocyclyl, alkoxycarbonyl, aralkoxycarbonyl, heteroaralkyloxycarbonyl or $R_k^aR_{k+1}^aN$-, $R_k^aR_{k+1}^aNC(=O)$-, $R_k^aR_{k+1}^aNC(=S)$-, $R_k^aR_{k+1}^aNSO_2$-, wherein $R_k^a$ and $R_{k+1}^a$ are, independently from one another, "amino group substituents", the meanings of which are defined elsewhere in this section, for example, hydrogen, alkyl, aryl, aralkyl, heteroaralkyl, heterocyclyl or heteroaryl, or $R_k^a$ and $R_{k+1}^a$ together with the N-atom, they are attached to, form through $R_k^a$ and $R_{k+1}^a$, a four- to seven-membered heterocyclyl or heterocyclenyl. The preferred alkyl groups are methyl, trifluoromethyl, cyclopropylmethyl, cyclopentylmethyl, ethyl, n-propyl, iso-propyl, n-butyl, tert-butyl, n-pentyl, 3-pentyl, methoxyethyl, carboxymethyl, methoxycarbonylmethyl, ethoxycarbonylmethyl, benzyloxycarbonylmethyl and pyridilmethyloxycarbonylmethyl. The preferred "alkyl substituents" are cycloalkyl, aryl, heteroaryl, heterocyclyl, hydroxy, alkoxy, alkoxycarbonyl, aralkoxy, aryloxy, alkylthio, heteroarylthio, aralkylthio, alkylsulfonyl, arylsulfonyl, alkoxycarbonyl, aralkoxycarbonyl, heteroaralkyloxycarbonyl or $R_k^aR_{k+1}^aN$-, $R_k^aR_{k+1}^aNC(=O)$-, annelated arylheterocyclenyl, annelated arylheterocyclyl.

[0001] "**Alkylamino**" means $C_nH_{2n+1}NH$- or $(C_nH_{2n+1})(C_nH_{2n+1})N$- group, where alkyl is defined in this section. The preferred alkylamino groups are methylamino, ethylamino, n-propylamino, iso-propylamino, and n-butylamino.

"**Antibody**" - a protein (immunoglobulin), synthesized by B-lymphocytes in organism in response to being hit by a foreign substance and having specific affinity for this substance. They are the most important factor of specific humoral immunity. Antibodies accomplish two functions: antigen-binding and effector (cause one or another immune response).

"**Autoantigens**" **-** free molecules of compounds or molecules being a part of cells, organs, and tissues, which under certain conditions are recognized by the immune system as foreign and in connection with it cause cellular or humoral immune response on the part of its organism. As a rule, they are normal proteins or protein complexes (also protein complexes with DNA or RNA) which are recognized by immune system at patients with autoimmune diseases. Such antigens normally must not be recognizes by the immune system, but because of genetic factors or environmental conditions immunological tolerance to such antigens could be lost. So-called natural self-antigens (sequestrated) may have properties of autoantigens. These include proteins synthesis of which are beginning after maturation of immune system (sperm, milk); macromolecules of organs, separated from immune system by histohematic barrier; macromolecules which are present in nuclei and cytoplasm of cells; macromolecules with novel foreign determinant groups as a consequence of action of endogenous (immune complexes, necrosis, inflammation) or exogenous (temperature, chemical compounds, including pharmaceutical substances, microbes and their toxins, viruses and others) factors; embrionic proteins with synthesis recommencing at certain conditions (for example, at tumors). They may induce an immune response leading to autoantibodies or sensibilized T-lymphocytes formation and to autoimmune diseases development. As a result of it autoimmune reaction begins to develop. They may lead to the development of the most diversified autoimmune diseases. These include in particular autoimmune polyendocrinopathy of the first type, autoimmune hemolytic anemia, idiopathic thrombocytopenia, hemolytic disease of the newborns, multiple sclerosis, autoimmune thyroiditis, and others.

Autoimmunity - a process and related diseases which is associated with the acquired ability of immune system to identify self-antigens (autoantigens) of the organism and respond to them by forming autoantibodies or autoimmune T-lymphocytes. Autoimmune process - a process and related diseases the base of which is tissue injury caused by after-effects of autoantigens interaction with autoantibodies or autoimmune T- lymphocytes.

"**Conjugate**" - a protein modified by chemical compounds, antigen or antibody. Forming of conjugate - is one of the important step of enzyme immunoassay (ELISA). In forming a conjugate the optimal method for introduction of chemical compound is selected so that a component of conjugate, antigen or antibody, keeps up its biological activity - antigenicity and antigen binding activity, respectively. Ability of foreign compounds and metabolites enter into

reactions of conjugation depends on the presence of determined functional groups in their molecules.

"**Medicament**" **-** is a compound (or mixture of compounds as a pharmaceutical composition) in the form of tablets, capsules, injections, ointments and other ready forms intended for restoration, improvement or modification of physiological functions at humans and animals, and also for treatment and prophylaxis of diseases, diagnostics, anesthesia, contraception, cosmetology and others.

"**Receptors**" (from Latin *recipere*) represent biological macromolecules located either on cytoplasmic membrane of the cell or intracellular, capable specifically interact with restricted number of physiologically active compounds (ligands) and transform the signal of this interaction into definite cellular response.

"**Solvates**" - adducts of solvent to solute; a special case of solvates - hydrates (solvent - water). Solvates are usually formed in solution, but at times (when solution is cooled, or the solvent was evaporated, and others) may be prepared in the form of crystalline phase - crystalline solvates.

"**Pharmaceutical composition**" means a composition comprising an active component (modified protein) and at least one of components selected from the group consisting of pharmaceutically acceptable and pharmacologically compatible fillers, solvents, diluents, carriers, auxiliary, distributing and sensing agents, delivery agents, such as preservatives, stabilizers, excipients, disintegrators, moisteners, emulsifiers, suspending agents, thickeners, sweeteners, flavouring agents, aromatizing agents, antibacterial agents, fungicides, lubricants, and prolonged delivery controllers, the choice and suitable proportions of which depend on the nature and way of administration and dosage. Examples of suitable suspending agents are ethoxylated isostearyl alcohol, polyoxyethene, sorbitol and sorbitol ether, microcrystalline cellulose, aluminum metahydroxide, bentonite, agar-agar and tragacant, and their mixtures as well. Protection against the action of microorganisms can be provided by various antibacterial and antifungal agents, such as, for example, parabens, chlorobutanole, sorbic acid, and similar compounds. Composition may also contain isotonic agents, such as, for example, sugars, sodium chloride, and similar compounds. Prolonged effect of composition may be achieved by agents slowing down absorption of active ingredient, for example, aluminum monostearate and gelatine. Examples of suitable carriers, solvents, diluents and delivery agents include water, ethanol, polyalcohols and their mixtures, natural oils (such as olive oil) and organic esters (such as ethyl oleate) for injections. Examples of fillers are lactose, milk-sugar, sodium citrate, calcium carbonate, calcium phosphate and the like. Examples of disintegrators and distributors are starch, alginic acid and its salts, and silicates. Examples of suitable lubricants are magnesium stearate, sodium lauryl sulfate, talc and polyethylene glycol of high molecular weight. Pharmaceutical composition for peroral, sublingval, transdermal, intramuscular, intravenous, subcutaneous, local or rectal administration of active ingredient, alone or in combination with another active compound may be administered to humans and animals in a standard administration form, or in a mixture with traditional pharmaceutical carriers. Suitable standard administration forms include peroral forms, such as tablets, gelatin capsules, pills, powders, granules, chewing-gums, and peroral solutions or suspensions, sublingval and transbuccal administration forms; aerosols; implants; local, transdermal, subcutaneous, intramuscular, intravenous, intranasal or intraocular forms of introduction and rectal administration forms. Pharmaceutical compositions are usually prepared by means of standard procedures by mixing an active compound with liquid or finely powdered solid carrier.

"**Pharmaceutically acceptable salt**" means a relatively nontoxic both organic and inorganic salts of acids and bases disclosed in this invention. Salts could be prepared *in situ* in processes of synthesis, isolation or purification of compounds or be prepared specially. In particular, bases salts could be prepared starting from purified base of disclosed compound and suitable organic or mineral acid. Examples of salts prepared in this manner include hydrochlorides, hydrobromides, sulfates, bisulfates, phosphates, nitrates, acetates, oxalates, valeriates, oleates, palmitates, stearates, laurates, borates, benzoates, lactates, p-toluenesulfonates, citrates, maleates, fumarates, succinates, tartrates, methane sulphonates, malonates, salicylates, propionates, ethane sulphonates, benzene sulfonates, sulfamates and the like (Detailed description of properties of such salts is given in: Berge S.M., et al., "Pharmaceutical Salts" J.Pharm.Sci., 1977, 66: 1-19). Salts of the disclosed acids could be also specifically prepared by reaction of purified acid with suitable base; moreover, metal salts and amine salts may be synthesized too. Metal salts are salts of sodium, potassium, calcium, barium, zink, magnesium, lithium and aluminum, sodium and potassium salts being preferred. Suitable inorganic bases from which metal salts can be prepared are sodium hydroxide, carbonate, bicarbonate and hydride; potassium hydroxide, carbonate and bicarbonate, lithium hydroxide, calcium hydroxide, magnesium hydroxide, zinc hydroxide. Organic bases suitable for preparation of disclosed acid salts are amines and amino acids of sufficient basicity to produce stable salt and suitable for medical purposes use (in particular, they are to have low toxicity). Such amines include ammonia, methylamine, dimethylamine, trimethylamine, ethylamine, diethylamine, triethylamine, benzylamine, dibenzylamine, dicyclohexylamine, piperazine, ethylpiperidine, tris(hydroxymethyl)aminomethane and the like. Besides, salts can be prepared using some tetraalkylammonium hydroxides, such as holine, tetramethylammonium, tetraethylammonium, and the like. Aminoacids may be selected among main aminoacids - lysine, ornithine and agrinine.

The purpose of the present invention is to provide novel compounds and modified by them proteins (conjugates) capable to interact with CD16a receptor which is used for induction of antibody-dependent cellular cytotoxicity and as a result of it for removal targeted group of cells from the body, for example, such as cancerous cells or autoimmune lymphocytes.

The purpose in view is achieved by novel compounds discovered by the authors and exhibiting affinity for CD16a receptor and containing activated group which is capable to add to amino group of protein, namely, the new substituted 5,5,11-trioxo-10,11-dihydro-5*H*-dibenzo[*b*,*f*][1,4]thiazepines of the general formula **1** or 5,6,7,8,9,10-hexahydro-4*H*-[1]benzothieno[3,2-*f*]pyrrolo[1,2-*a*][1,4]diazepines of the general formula **2**, or pharmaceutically acceptable salts or solvates thereof,

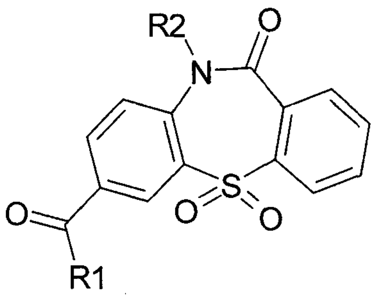

**1**

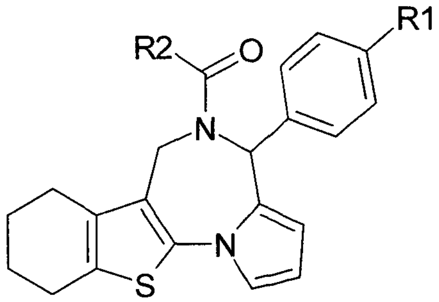

**2**

where R1 represents $(CH_3)_2N$-,

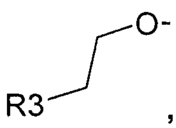 , 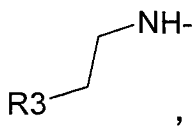 , 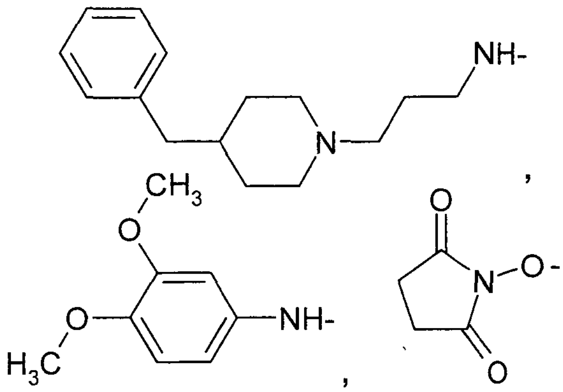 , 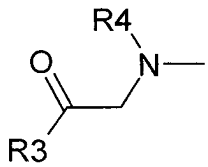 , 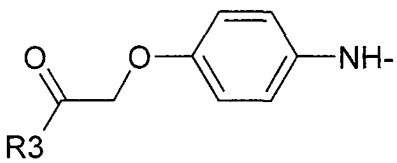 ,

R2 represents

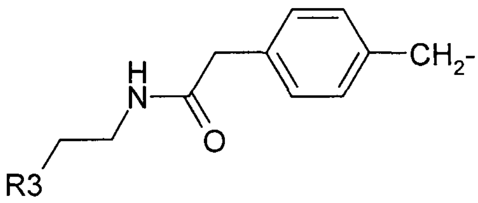, 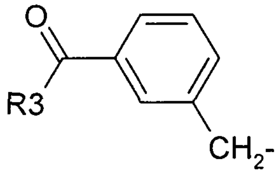, 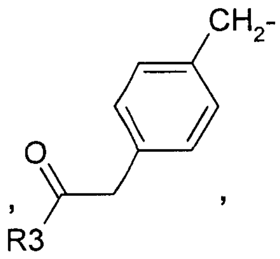,

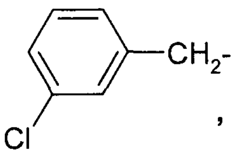, 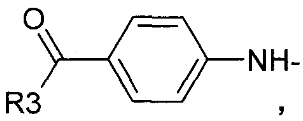, 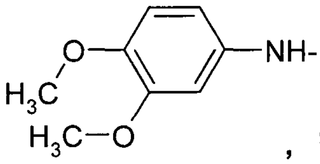, 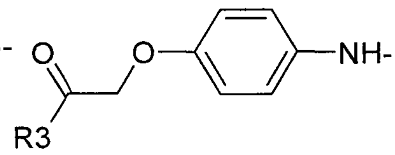

where R3, as a terminal substituent, represents unsubstituted aminoalkyl,

-$NH_2$, 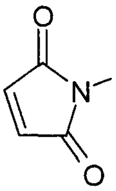, 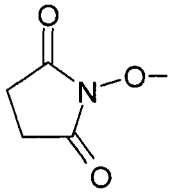, or 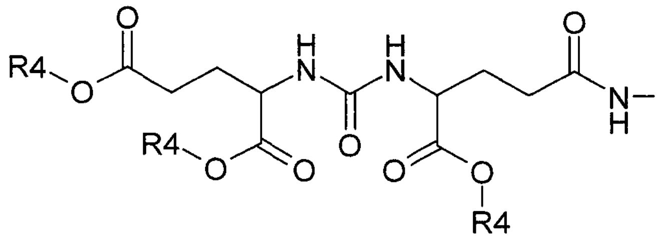,

R4 represents H or $C_1$-$C_3$alkyl.

The preferred compounds are compounds of the general formula **1**, where R1 represents:

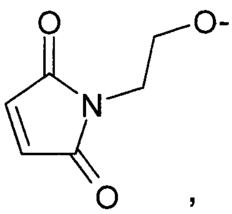, 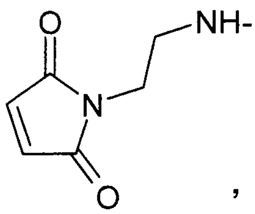, 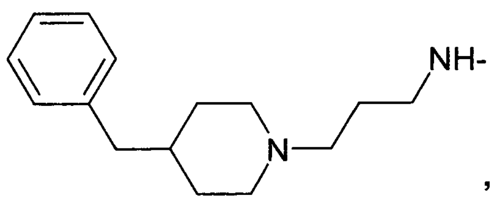, 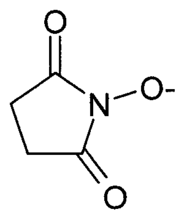,

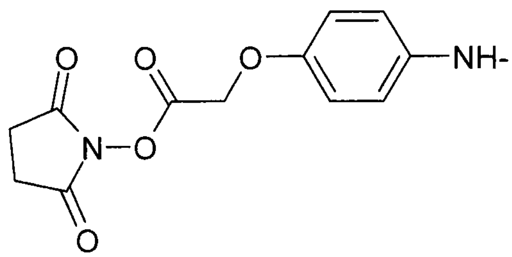, 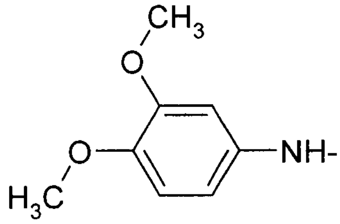,

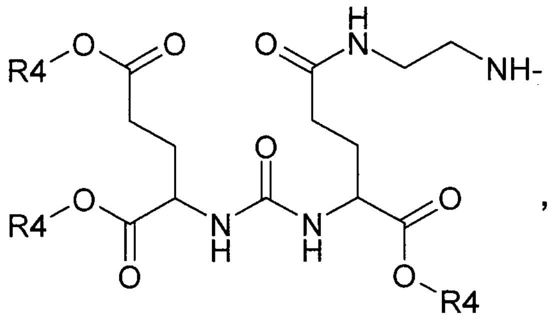

,

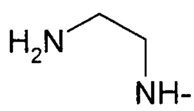

;

R2 selected from the group consisting of:

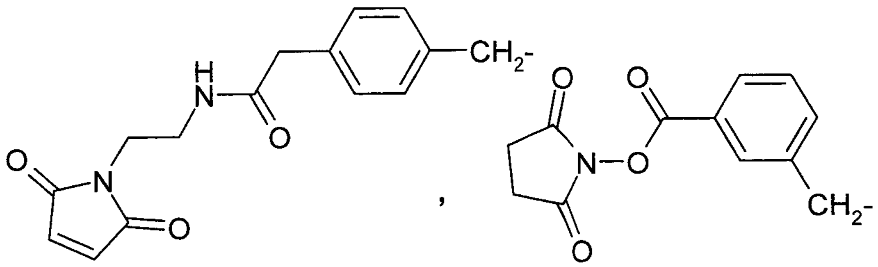

, , ,

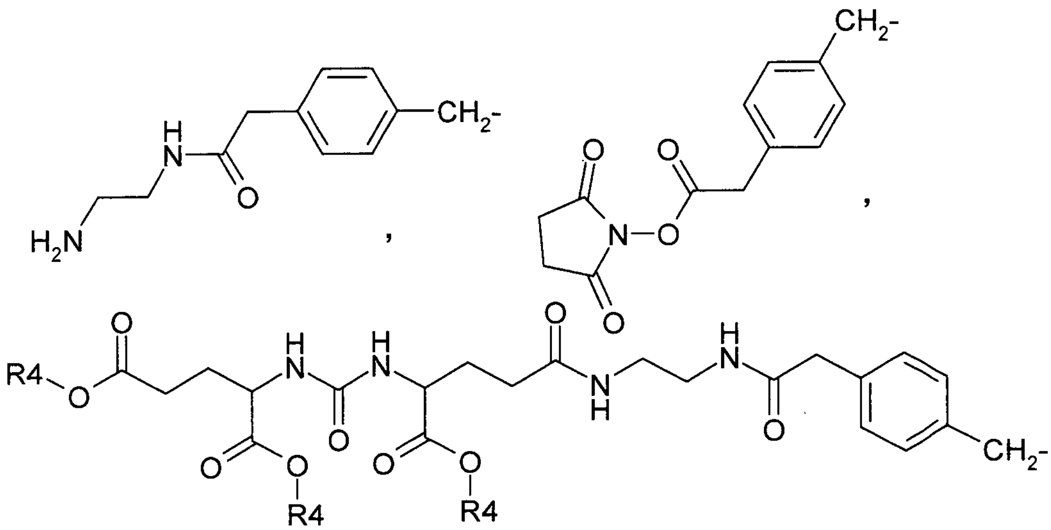

,

,

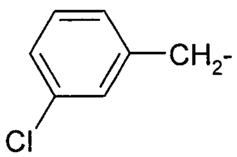

where R4 = H or $C_1$-$C_3$alkyl.

The preferred compounds are also compounds of the general formula **2**, in which R1 represents $(CH_3)_2N$- or

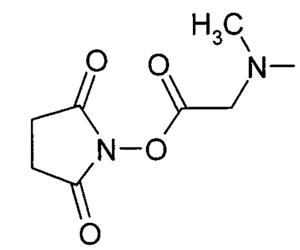

,

R2 represents

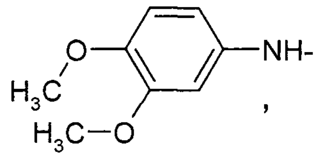, 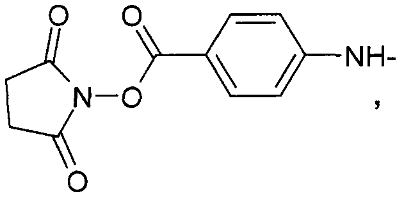, 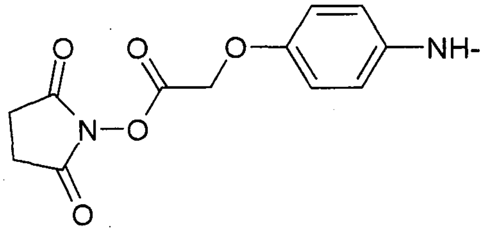

The more preferred compounds are:

2,5-Dioxopyrrolidin-1-yl ester of (3-chlorobenzyl)-5,5,11-trioxo-10,11-dihydro-5*H*-dibenzo[*b*,*f*][1,4]thiazepine-7-carboxylic acid **1(1)**,
2,5-dioxopyrrolidin-1-yl ester of (4-{[10-(3-chlorobenzyl)-5,5,11-trioxo-10,11-dihydro-5*H*-dibenzo[*b*,*f*][1,4]thiazepine-7-carbonyl]-amino}-phenoxy)-acetic acid **1(2)**,
2,5-dioxopyrrolidin-1-yl ester of 4-{[10-(3-chlorobenzyl)-5,5,11-trioxo-10,11-dihydro-5*H*-dibenzo[*b*,*f*][1,4]thiazepine-7-carbonyl]-amino}-phenylcarboxylic acid **1(3)**,
2,5-dioxopyrrolidin-1-yl ester of 3-[8-(3,4-dimethoxyphenylcarbamoyl)-5,5,11-trioxo-5,11-dihydro-dibenzo[*b*,*f*][1,4]thiazepin-10-ylmethyl]-benzoic acid **1(4)**,
2,5-dioxopyrrolidin-1-yl ester of (4-{8-[3-(4-benzylpiperidin-1-yl)-propylcarbamoyl]-5,5,11-trioxo-5,11-dihydrodibenzo[*b*,*f*][1,4]thiazepin-10-ylmethyl}-phenyl)-acetic acid **1(5)**,
10-(3-chlorobenzyl)-5,5,11-trioxo-10,11-dihydro-5*H*-dibenzo[*b*,*f*][1,4]thiazepine-8-carboxylic acid (2-aminoethyl)-amide **1(6)**,
10-{4-[(2-amino-ethylcarbamoyl)-methyl]-benzyl}-5,5,11-trioxo-10,11-dihydro-5*H*-dibenzo[b,f][1,4]thiazepine-8-carboxylic acid [3-(4-benzyl-piperidin-1-yl)-propyl]-amide **1(7)**,
2-(2,5-dioxo-2,5-dihydro-pyrrol-1-yl)-ethyl ester of 10-(3-chlorobenzyl)-5,5,11-trioxo-10,11-dihydro-5*H*-dibenzo[*b*,*f*][1,4]thiazepine-8-carboxylic acid **1(8)**,
10-(4-{[2-(2,5-dioxo-2,5-dihydro-pyrrol-1-yl)-ethylcarbamoyl]-methyl}-benzyl)-5,5,11-trioxo-10,11-dihydro-5*H*-dibenzo[*b*,*f*][1,4]thiazepine-8-carboxylic acid [3-(4-benzyl-piperidin-1-yl)-propyl]-amide **1(9)**,
*N*-[2-({*N*-(methoxycarbonyl)-*N*-[(1,5-dimethoxy-1,5-dioxopentan-2-yl)carbamoyl]-β-alanyl}amino)ethyl]-10-(3-chlorobenzyl)-5,5,11-trioxo-10,11-dihydrodibenzo[*b*,*f*][1,4]thiazepine-7-carboxamide **1(10)**,
$N^5$-(2-{[(4-{[7-{[[3-(4-benzylpiperidin-1-yl)propyl](phenyl)amino]-carbonyl}-5,5,11-trioxo-dibenzo[*b*,*f*][1,4]thiazepin-10(11*H*)-yl]methyl}phenyl)acetyl]-amino}ethyl)-$N^2$-{[(1,3-dicarboxypropyl)amino]carbonyl}glutamine **1(11)**,
4-[4-(dimethylamino)phenyl]-*N*-(4-{[(2,5-dioxopyrrolidin-1-yloxy]carbonyl}phenyl)-7,8,9,10-tetrahydro-4*H*-[1]benzothieno[3,2-*f*]pyrrolo[1,2-*a*][1,4]diazepine-5(6*H*)-carboxamide **2(1)**,
4-[4-(dimethylamino)phenyl]-*N*-(4-{2-[(2,5-dioxopyrrolidin-1-yl)oxy]-2-oxoethoxy}phenyl)-7,8,9,10-tetrahydro-4*H*-[1]benzothieno[3,2-*f*]pyrrolo[1,2-*a*][1,4]diazepine-5(6*H*)-carboxamide **2(2)**,
2,5-dioxopyrrolidin-1-yl *N*-[4-(5-{[(3,4-dimethoxyphenyl)amino]-carbonyl}-5,6,7,8,9,10-hexahydro-4*H*-[1]benzothieno[3,2-*f*]pyrrolo[1,2-*a*][1,4]diazepin-4-yl)phenyl]-*N*-methylglycinate **2(3)**.

For the first time the authors have found modified protein (conjugate), active towards CD16a receptor. That is why the subject of the present invention is a modified protein, active towards CD16a receptor, conjugated by modifying compound exhibiting affinity for CD16a receptor, selected from the compounds of the general formula **1** or **2**.

The more preferred is a modified protein (conjugate), active towards CD16a receptor, prepared by interaction of the protein with modifying compound of the general formula **1** or **2**.

The more preferred is a modified protein (conjugate), prepared from an antibody and a compound of the general formula **1** or **2**, where antibody represents rituximab, trastuzumab, or cetuximab.

The more preferred is also a modified protein (conjugate), representing rituximab, modified by a compound of the general formula **1** or **2**.

The more preferred is also a modified protein (conjugate), representing trastuzumab, modified by a compound of the general formula **1** or **2**.

The more preferred is also a modified protein (conjugate), representing cetuximab, modified by a compound of the general formula **1** or **2**.

The more preferred is also a modified protein (conjugate), prepared from an autoantigen and a compound of the general formula **1** or **2**, where autoantigen represents interferon alfa or myelin basic protein, or complement C1q protein.

The more preferred is also a modified protein (conjugate), representing interferon alfa modified by a compound of the general formula **1** or **2**.

The more preferred is also a modified protein (conjugate), representing myelin basic protein, modified by a compound of the general formula **1** or **2**.

The more preferred is also a modified protein (conjugate), representing complement C1q protein, modified by a compound of the general formula **1** or **2**.

Investigation of comparative effectiveness of proteins and their conjugates towards CD16a receptor revealed that conjugates are 1-3 orders of magnitude more active in comparison with their unmodified proteins.

The subject of the present invention is also a method for preparation of modified protein (conjugate), according to which a protein is subjected to interaction with a compound of the general formula **1** or **2**, dissolved in organic solvent, such as, for example, dimethyl sulfoxide, at mole ratio in interval from 1:3 to 1:100 in medium of PBS solution (pH 7.4) at room temperature at constant stirring.

The subject of the present invention is also a pharmaceutical composition active towards CD16a receptor, comprising a modified protein (conjugate) in therapeutically effective amount and pharmaceutically acceptable diluent, carrier, or excipient.

Pharmaceutical compositions may include pharmaceutically acceptable excipients. Pharmaceutically acceptable excipients mean diluents, auxiliary agents and/or carriers applied in the sphere of pharmaceutics. According to the present invention, pharmaceutical composition in addition to a modified protein (conjugate) prepared by interaction of a protein with a modifying compound of the general formula **1** or **2**, or pharmaceutically acceptable salt or solvate thereof, may include other active ingredients among them possessing influenza activity provided that they do not give rise to undesirable effects.

If necessary, to use a pharmaceutical composition of the present invention in clinical practice, they may be mixed with traditional pharmaceutical carries.

According to the present invention the carriers used in pharmaceutical compositions represent carriers, which are used in the sphere of pharmaceutics for preparation of commonly used forms, among them for peroral forms, for injections, for local forms.

The subject of the present invention is also a medicament active towards CD16a receptor in the form of tablets, capsules or injections placed in a pharmaceutically acceptable packing, intended for treating diseases caused by pathologic cells, comprising a novel modified protein (conjugate) or novel pharmaceutical composition in therapeutically effective amount.

Since at forming a conjugate its component, antigen or antibody, preserve their biological activity - antigenicity and antigenbinding activity, respectively, conjugate, according to the present invention, could be used for treating the same diseases, such as low-grade or follicular non-Hodgkin's lymphoma, breast cancer, for which unconjugated monoclonal antibodies are used as medicaments. It is also known that antibody activity towards CD16a receptor is used for treating autoimmune or oncology diseases (R. L. Ferris, et al. J. Clinical Oncology, 2010, Oct 1, Vol. 28, No 28: 4390-4399), among them lymphoma (K.-H. Heider, et al. Blood, 2011, 118: 4159-4168) or lymphatic leukemia (J. A. Bowels, et al. Blood, 2006, 108: 2648-2654).

The subject of the present invention is a method for treating disease caused by pathologic cells which consists in indirect action on CD16a receptor, according to which therapeutically effective amount of modified protein (conjugate) or pharmaceutical composition, or medicament active towards CD16a receptor are administered to subject.

The preferred is the method for treating autoimmune or oncology diseases defined above in this section, among them lymphoma, lymphocytic leukemia or breast cancer.

The preferred is the method for treating autoimmune polyendocrinopathy of the first type.

Medicaments could be administered peroral or parenterally (for example, intravenous, subcutaneous, intraperitoneally or local). Clinical dose of modified protein (conjugate), or pharmaceutical composition, or medicament, active towards CD16a receptor at patients may be corrected depending on: therapeutic efficiency and bio-accessibility of active ingredients in patients' organism, rate of their exchange and removal from organism, and age, gender, and severity of patient's symptoms. Thus, the daily intake for adults normally is 300~1200 mg, preferably 500~1000 mg in case when protein of the conjugate represents antibody, and 0.01 ~ 100 mg, preferably - 0.1 ~ 10 mg in case when protein in conjugate is autoantigen. Therefore, according to the present invention, while preparing pharmaceutical composition as a dose unit the above effective dose is to be taken into consideration. Following the instructions of physician or pharmacist, the medicaments may be taken several times over specified periods of time (preferably, from one to six times).

**The best mode for carrying out the invention**

The invention is illustrated by the following drawings:

**Fig. 1.** NMR spectrum of 2,5-dioxopyrrolidin-1-yl ester of (3-chlorobenzyl)-5,5,11-trioxo-10,11-dihydro-5*H*-dibenzo[*b*,*f*][1,4]thiazepine-7-carboxylic acid **1(1)**,

**Fig. 2.** NMR spectrum of 2,5-dioxopyrrolidin-1-yl ester of (4-{[10-(3-chlorobenzyl)-5,5,11-trioxo-10,11-dihydro-5*H*-dibenzo[*b*,*f*][1,4]thiazepine-7-carbonyl]-amino}-phenoxy)-acetic acid **1(2)**,

**Fig. 3**. NMR spectrum of 2,5-dioxopyrrolidin-1-yl ester of 4-{[10-(3-chlorobenzyl)-5,5,11-trioxo-10,11-dihydro-5*H*-dibenzo[*b*,*f*][1,4]thiazepine-7-carbonyl]-amino}-phenylcarboxylic acid **1(3)**,

**Fig. 4.** LCMS spectrum of 2,5-dioxopyrrolidin-1-yl ester 3-[8-(3,4-dimethoxyphenyl carbamoyl)-5,5,11-trioxo-5,11-dihydro-5*H*-dibenzo[*b*,*f*][1,4]thiazepin-10-ylmethyl]-benzoic acid **1(4)**,

**Fig 5.** NMR spectrum of 2,5-dioxopyrrolidin-1-yl ester of (4-{8-[3-(4-benzylpiperidin-1-yl)-propylcarbamoyl]-5,5,11-trioxo-5,11-dihydro-dibenzo[*b*,*f*][1,4]thiazepin-10-ylmethyl}-phenyl)-acetic acid **1(5)**,

**Fig. 6.** NMR spectrum of 10-(3-chlorobenzyl)-5,5,11-trioxo-10,11-dihydro-5*H*-dibenzo[b,f][1,4]thiazepine-8-carboxylic acid (2-aminoethyl)-amide **1(6)**,

**Fig. 7.** NMR spectrum of 10-{4-[(2-amino-ethylcarbamoyl)-methyl]-benzyl}-5,5,11-trioxo-10,11-dihydro-5*H*-dibenzo[*b*,*f*][1,4]thiazepine-8-carboxylic acid [3-(4-benzyl-piperidin-1-yl)-propyl]-amide **1(7)**,

**Fig. 8.** NMR spectrum of 2-(2,5-dioxo-2,5-dihydro-pyrrol-1-yl)-ethyl ester 10-(3-chlorobenzyl)-5,5,11-trioxo-10,11-dihydro-5*H*-dibenzo[b,f][1,4]thiazepine-8-carboxylic acid **1(8)**,

**Fig. 9.** NMR spectrum of 10-(4-{[2-(2,5-dioxo-2,5-dihydro-pyrrol-1-yl)-ethylcarbamoyl]-methyl}-benzyl)- 5,5,11-trioxo-10,11-dihydro-5*H*-dibenzo[b,f][1,4]thiazepine-8-carboxylic acid [3-(4-benzyl-piperidin-1-yl)-propyl]-amide **1(9),**

**Fig. 10.** NMR spectrum of *N*-[2-({*N*-(methoxycarbonyl)-*N*-[(1,5-dimethoxy-1,5-dioxopentan-2-yl)carbamoyl]-β-alanyl}amino)ethyl]-10-(3-chlorobenzyl)-5,5,11-trioxo-10,11-dihydrodibenzo[*b*,*f*][1,4]thiazepine-7-carboxamide **1(10),**

**Fig. 11.** NMR spectrum of $N^5$-(2-{[(4-{[7-{[[3-(4-benzylpiperidin-1-yl)propyl](phenyl)amino]-carbonyl}-5,5,11-trioxo-dibenzo[*b*,*f*][1,4]thiazepin-10(11*H*)-yl]methyl}phenyl)acetyl]-amino}ethyl)-$N^2$-{[(1,3-dicarboxypropyl)amino]carbonyl}glutamine **1(11),**

**Fig 12.** LCMS spectrum of 4-[4-(dimethylamino)phenyl]-*N*-(4-{[(2,5-dioxopyrrolidin-1-yl)oxy]carbonyl}phenyl)-7,8,9,10-tetrahydro-4*H*-[1]benzothieno[3,2-*f*]pyrrolo[1,2-*a*][1,4]diazepine-5(6*H*)-carboxamide **2(1),**

**Fig. 13.** NMR spectrum of 4-[4-(dimethylamino)phenyl]-*N*-(4-{2-[(2,5-dioxopyrrolidin-1-yl)oxy]-2-oxoethoxy}phenyl)-7,8,9,10-tetrahydro-4*H*-[1]benzothieno[3,2-*f*]pyrrolo[1,2-*a*][1,4]diazepine-5(6*H*)-carboxamide **2(2),**

**Fig. 14.** LCMS spectrum of 2,5-dioxopyrrolidin-1-yl *N*-[4-(5-{[(3,4-dimethoxyphenyl)amino]-carbonyl}-5,6,7,8,9,10-hexahydro-4*H*-[1]benzothieno[3,2-*f*]pyrrolo[1,2-*a*] [1,4]diazepin-4-yl)phenyl]-*N*-methylglycinate **2(3).**

**Fig. 15.** Chromatogram of conjugate **CR1(1),** on column TSK GEL SUPER SW3000.

**Fig. 16.** Enzyme immunoassay (ELISA) for binding of rituximab conjugates with CD16a receptor. Dependence of optical density at 450 nm on conjugate concentration used in (ELISA) analysis.

**Fig. 17.** Enzyme immunoassay (ELISA) for binding of interferon (**I**) and conjugate **CI1(1)** with CD16a receptor. Dependence of optical density at 450 nm on conjugate concentration used in (ELISA) analysis.

**Fig. 18.** Comparison of rituximab **(R)** and conjugate **CR1(1)** activities in test of antibody-dependent cytotoxicity.

**Fig. 19.** Comparison of rituximab **(R)** and conjugate **CR1(2)** activities in test of antibody-dependent cytotoxicity.

**Fig. 20.** Enzyme immunoassay (ELISA) for binding of rituximab **(R),** trastuzumab **(T)** and their conjugates **CR1(7), CT1(7)** towards CD16a receptor.

The examples given below illustrate, but not limit the scope of the present invention.

**Example 1.** 2,5-Dioxopyrrolidin-1-yl ester of (3-chlorobenzyl)-5,5,11-trioxo-10,11-dihydro-5*H*-dibenzo[*b*,*f*][1,4]thiazepine-7-carboxylic acid **1(1)** was prepared according to the following Scheme 1.

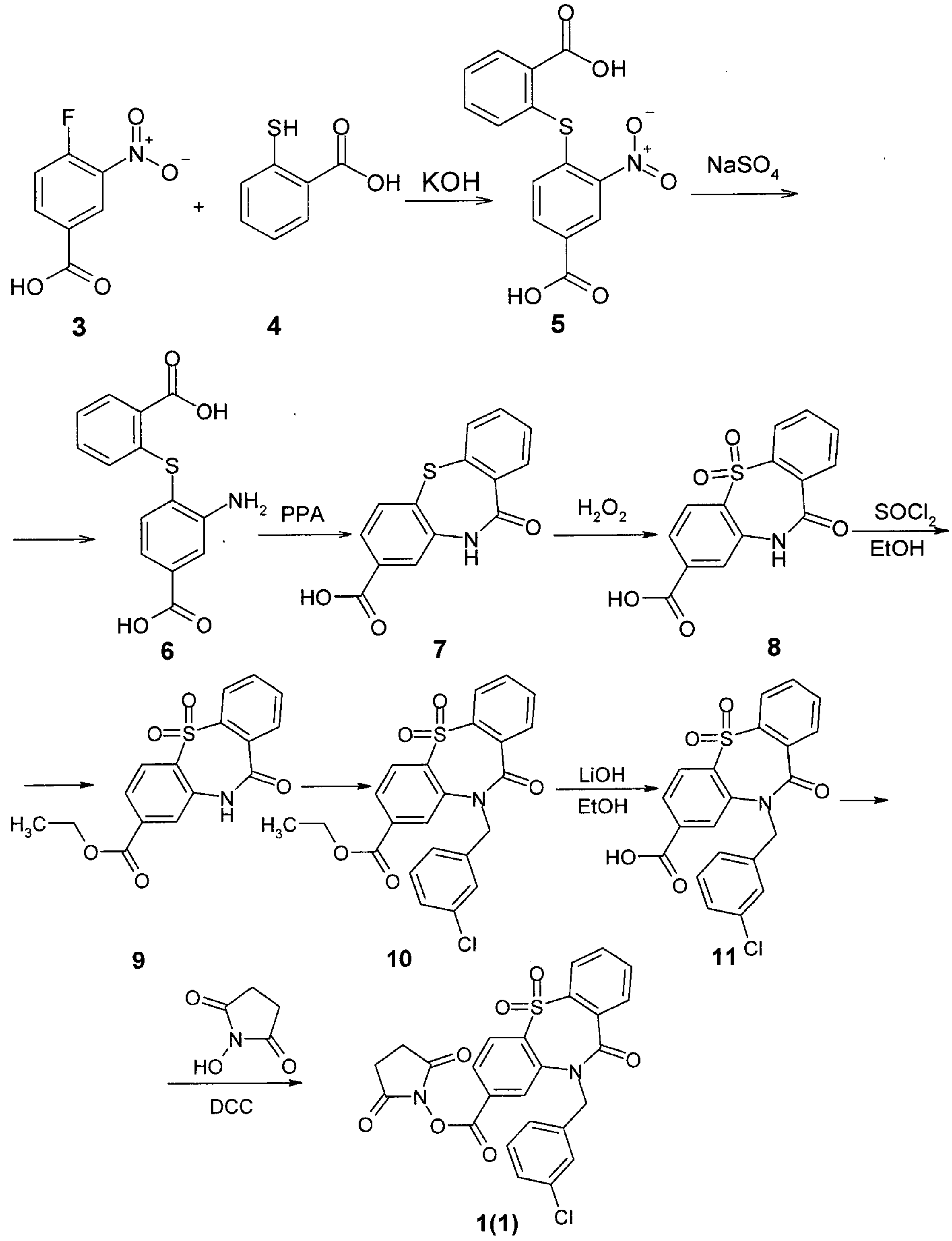


**Scheme 1.**

Compound **4** (25 g) was added in portions to water (170 ml) KOH (31.8 g) solution, after its dissolution compound **3** (30 g) was added and the resultant mixture was stirred at 60° C for 15 h. Then the reaction mixture was cooled, acidified with HCl (10%) to pH=3, filtered, washed with water and dried. It gave compound **5,** yield 70%. Compound **5** (50 g) was dissolved in aqueous ammonia (500 ml) and dithionite (80 g) was added in portions, after that the reaction mixture was refluxed for 1 h, cooled, ammonia was evaporated on rotary evaporator, and water solution was acidified with conc. HCl to pH=1, stirred for 1 h, the solid was filtered off, washed with water and dried. It gave compound **6,** yield 60%. Compound **6** (45 g) was added in portions to polyphosphoric acid (200 ml) at 50°C, then the reaction mixture was stirred for 10 h

at 90 °C, cooled and poured out on ice (500 ml), the solid was filtered off, washed with water and dried. It gave compound **7,** yield 60%. Pactbopяют Compound **7** (30 g) was dissolved in acetic acid (500 ml), 33% hydrogen peroxide (70 ml) was added, stirring was continued for night at 70°C. After cooling acetic acid was evaporated on rotary evaporator and water (600 ml) was added to the residue. The precipitated solid was filtered off, washed with water and dried. It gave compound **8,** yield 70%. To a solution of compound **8** (22 g) in ethanol (300 ml) thionyl chloride (8 ml) was dropped at 10°C, then the mixture was refluxed for 5 h, cooled, evaporated on rotary evaporator, after that water (300 ml) was added. The precipitated solid was filtered off, washed with water and dried. It gave compound **9,** yield 90%. To a solution of compound **9** (8.2 g) in DMF (80 ml) were added in turn potassium carbonate (7 g) and m-chlorobenzyl chloride (5.2 g). The reaction mixture was stirred for night at 50°C, evaporated on rotary evaporator, water (200 ml) was added to the residue. The precipitated solid was filtered off, washed with water and dried. It gave compound **10,** yield 90%. To a solution of compound **10** (11 g) in 50% water ethanol (150 ml) KOH (2.7 g) was added, the resultant mixture was stirred at room temperature for night. Then ethanol was evaporated on rotary evaporator, water solution was acidified with 10% HCl to pH=3. The precipitated solid was filtered off, washed with water and dried. It gave compound **11,** yield 80%. To a solution of compound **11** (1.8 g) in THF (50 ml) at stirring in argon atmosphere N-hydroxysuccinimide (0.48 g) and dicyclohexylcarbodiimide (0.87g) were added. The reaction mixture was stirred for night at room temperature, the precipitated solid was filtered off, mother liquor was evaporated, the residue was purified by flash chromatography on silica (eluent-ethyl acetate). It gave compound **1(1),** yield 50%. NMR Spectrum of compound **1(1)** is given on Fig.1.

**Example 2.** 2,5-Dioxopyrrolidin-1-yl ester of (4-{[10-(3-chlorobenzyl)-5,5,11-trioxo-10,11-dihydro-5*H*-dibenzo[*b*,*f*][1,4]thiazepine-7-carbonyl]-amino}-phenoxy)-acetic acid **1(2)** was prepared according to Scheme 2 given below.

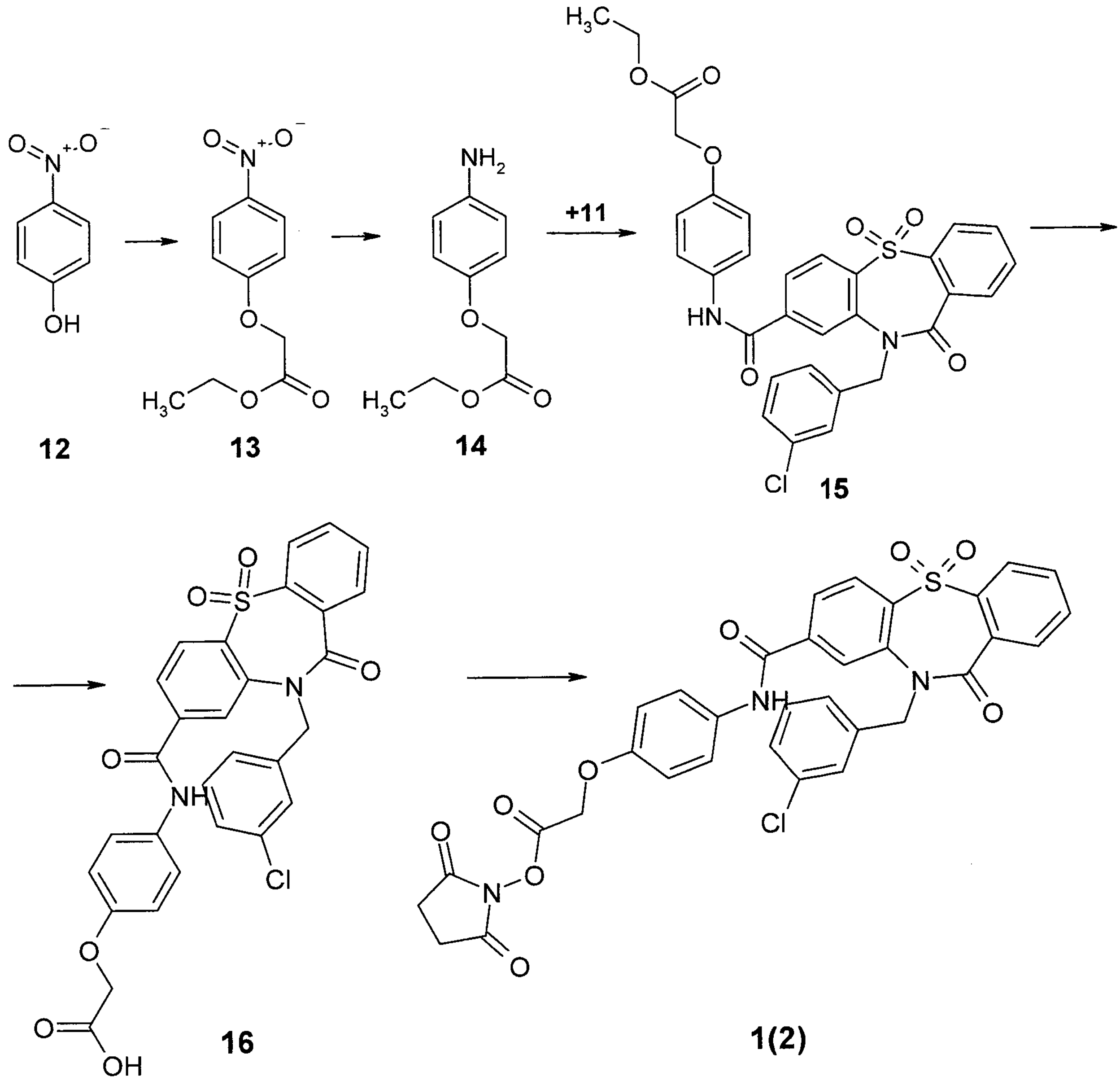


12 13 14 15

16 1(2)

**Scheme 2.**

*p*-Nitrophenol **12** (15 g) was added to a suspension of potassium carbonate (30 g) in acetonitrile (200 ml), stirred for 1 h, following which bromoacetic acid ethyl ester (19.8 g) was dropped. The resultant mixture was stirred for night at 60°C, filtered and evaporated on rotary evaporator. The obtained compound **13** was used further without purification. To a solution of compound **13** (14.3 g) in 50% aqueous acetic acid (200 ml) at 70°C powder Fe (10 g) was added in small portions at such a rate that the reaction mixture was boiling. Then the reaction mixture was refluxed for additional 15 min, cooled and water (500 ml) was added. The mixture was extracted with ethyl acetate (3x150 ml), combined extracts were washed with conc. solution of $NaHCO_3$, dried, solvent was evaporated on rotary evaporator. It gave compound **14,** yield 75%. Compound **11** (1.9 g) and compound **14** (0.87 g) were stirred in dioxane (50 ml) for 1 h, then triethylamine (1.2 ml) and phosphorus oxychloride (0.89 g) were added and stirred for 3 h at 50°C. Then water (150 ml) was added, the precipitated solid was filtered off, washed with water and dried. It gave compound **15,** yield 60%. To a solution of compound **15** (2.7 g) in 50% aqueous ethanol (20 ml) LiOH (0.93 g) was added and the reaction mixture was stirred for night at room temperature. Then ethanol was evaporated on rotary evaporator, water solution was acidified with 10% HCl to pH=3. The precipitated solid was filtered off, washed with water and dried. The crude product was purified by column chromatography, eluent - chloroform : methanol : triethylamine 10:1:1. It gave compound **16,** yield 11%. To a solution of compound **16** (290 mg) in THF (50 ml) in argon atmosphere at stirring *N*-hydroxysuccinimide (58

g) and dicyclohexylcarbodiimide (104 mg) were added. The mixture was stirred for night at room temperature, the precipitated solid was filtered off, mother liquor was evaporated, residue was purified by flash-chromatography on silica (eluent - ethyl acetate). It gave compound **1(2),** yield 50%. NMR Spectrum of compound **1(2)** is given in Fig. 2.

**Example 3.** 2,5-Dioxopyrrolidin-1-yl ester of 4-{[10-(3-chlorobenzyl)-5,5,11-trioxo-10,11-dihydro-5*H*-dibenzo[*b*,*f*][1,4]thiazepine-7-carbonyl]-amino}-phenylcarboxylic acid **1(3)** was prepared according to Scheme 3 given below.

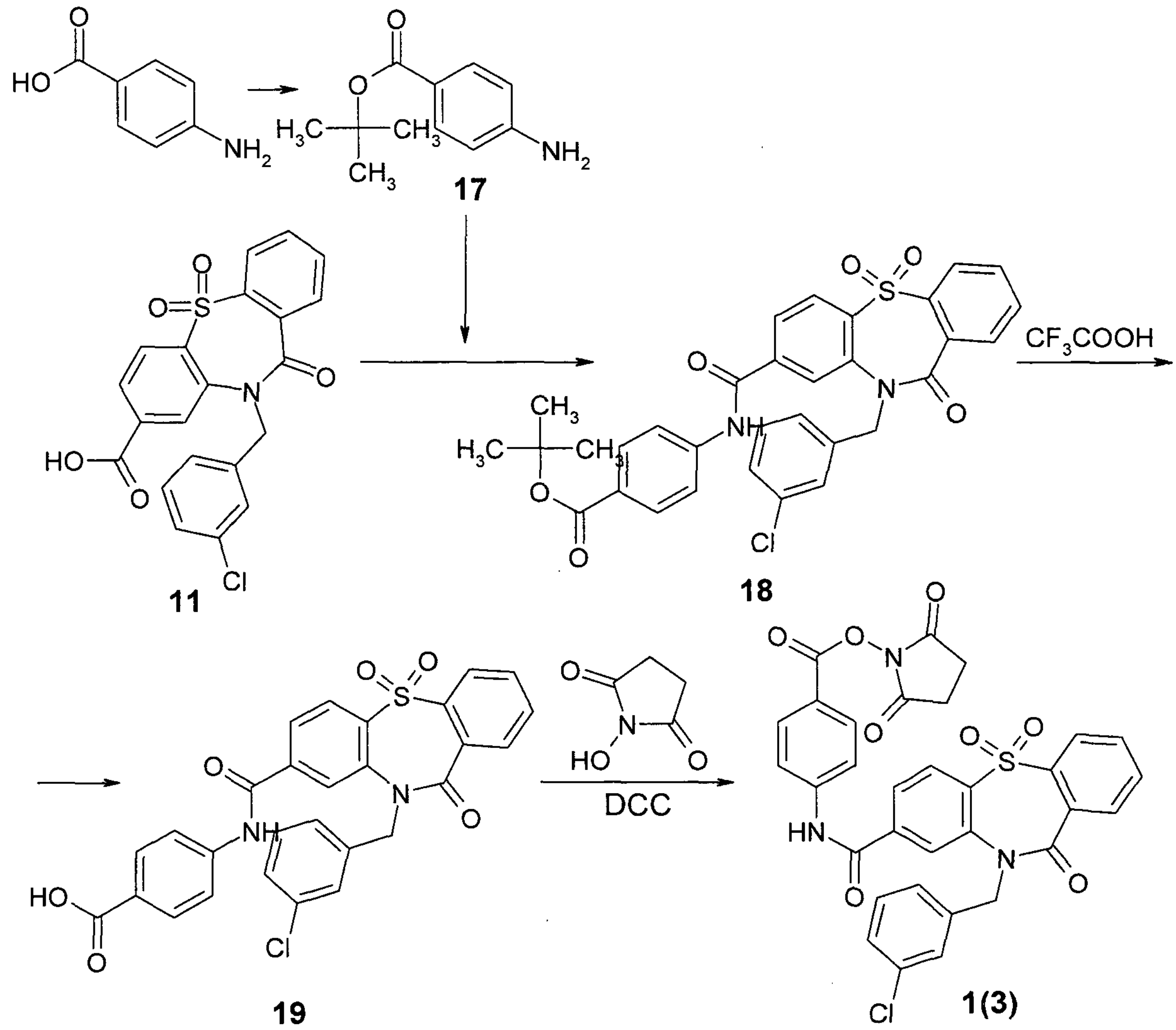


**Scheme 3.**

To a solution of *p*-aminobenzoic acid (1 eq.) in *tert*.-butyl alcohol was added EDC (1.1 eq.). The reaction mixture was refluxed for 18 h. After cooling to 0°C water was added and the resultant mixture was extracted with diethyl ether. Evaporated organic layer was used in the next stage without purification. It gave compound **17,** yield 60%. Compound **11** (2.2 g), *tert*.-butyl ester of p-aminobenzoic acid **17** (0.85 g) and dioxane (50 ml) were stirred together for 1 h, then triethylamine (1.4 ml) and phosphorous oxychloride (1.0 g) were added. The reaction mixture was stirred for 3 h at 50°C, water was added (150 ml), precipitated solid was filtered off, washed with water and dried. It gave compound **18,** yield 60%. Compound **18** was dissolved in trifluoroacetic acid and stirred for night at 50°C. Then the reaction mixture was evaporated to dryness. It gave compound **19,** yield 88%, which was used in the next stage without purification. To a solution of compound **19** (760 mg) in THF (50 ml) in argon atmosphere at stirring N-hydroxysuccinimide (160 mg) and dicyclohexylcarbodiimide (287 mg) were added. The mixture was stirred for night at room temperature, precipitated solid was filtered off, mother liquor was evaporated and purified by flash chromatography on silica (eluent - ethyl acetate). It gave compound **1(4),** yield 50%. NMR Spectrum of compound **1(4)** is given in Fig 3.

**Example 4.** 2,5-Dioxopyrrolidin-1-yl ester 3-[8-(3,4-dimethoxyphenylcarbamoyl)-5,5,11-trioxo-5,11-dihydrodibenzo[*b*,*f*][1,4]thiazepin-10-ylmethyl]-benzoic acid **1(4)** was prepared according to Scheme 4 given below.

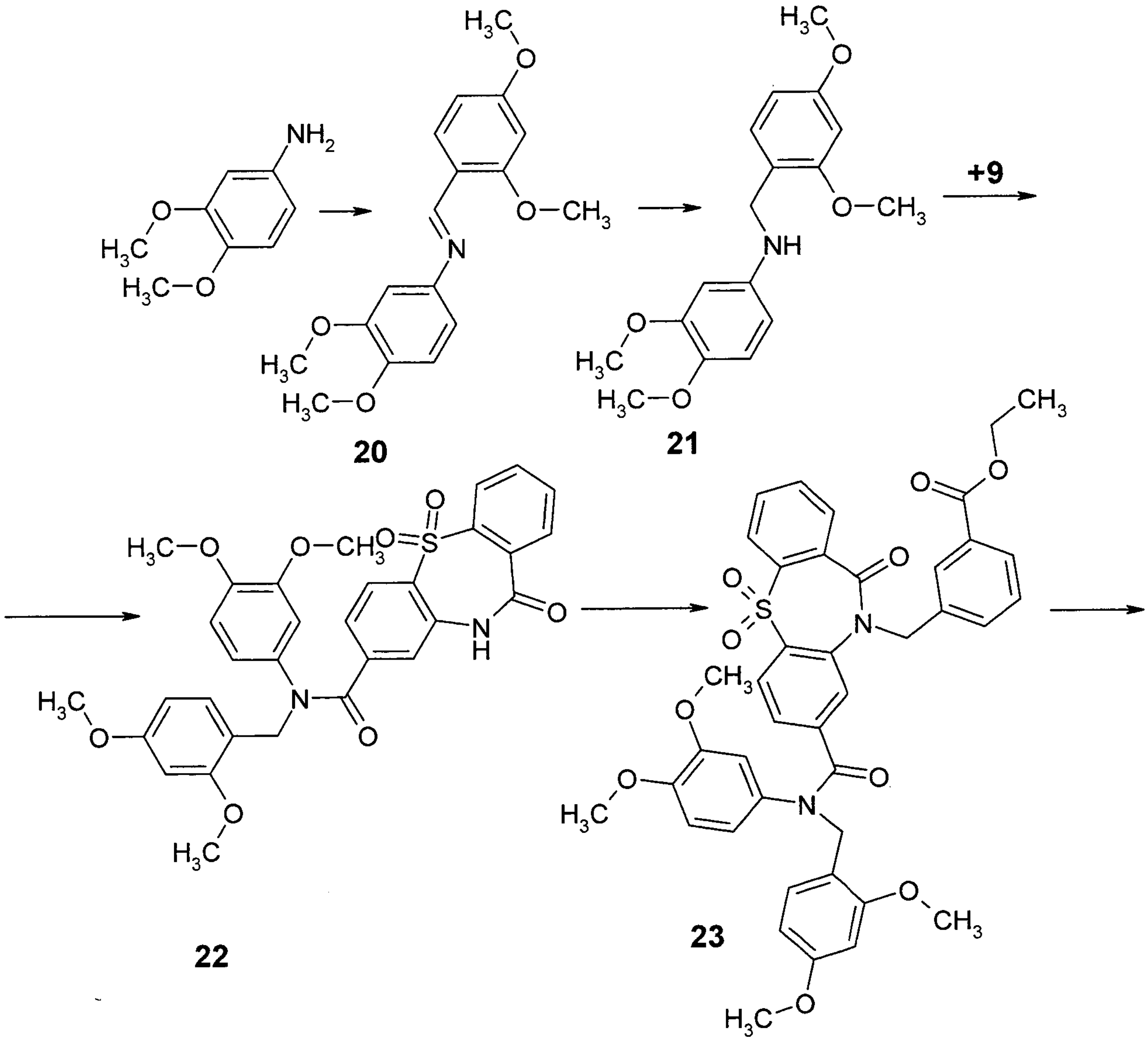


20

21

22

23

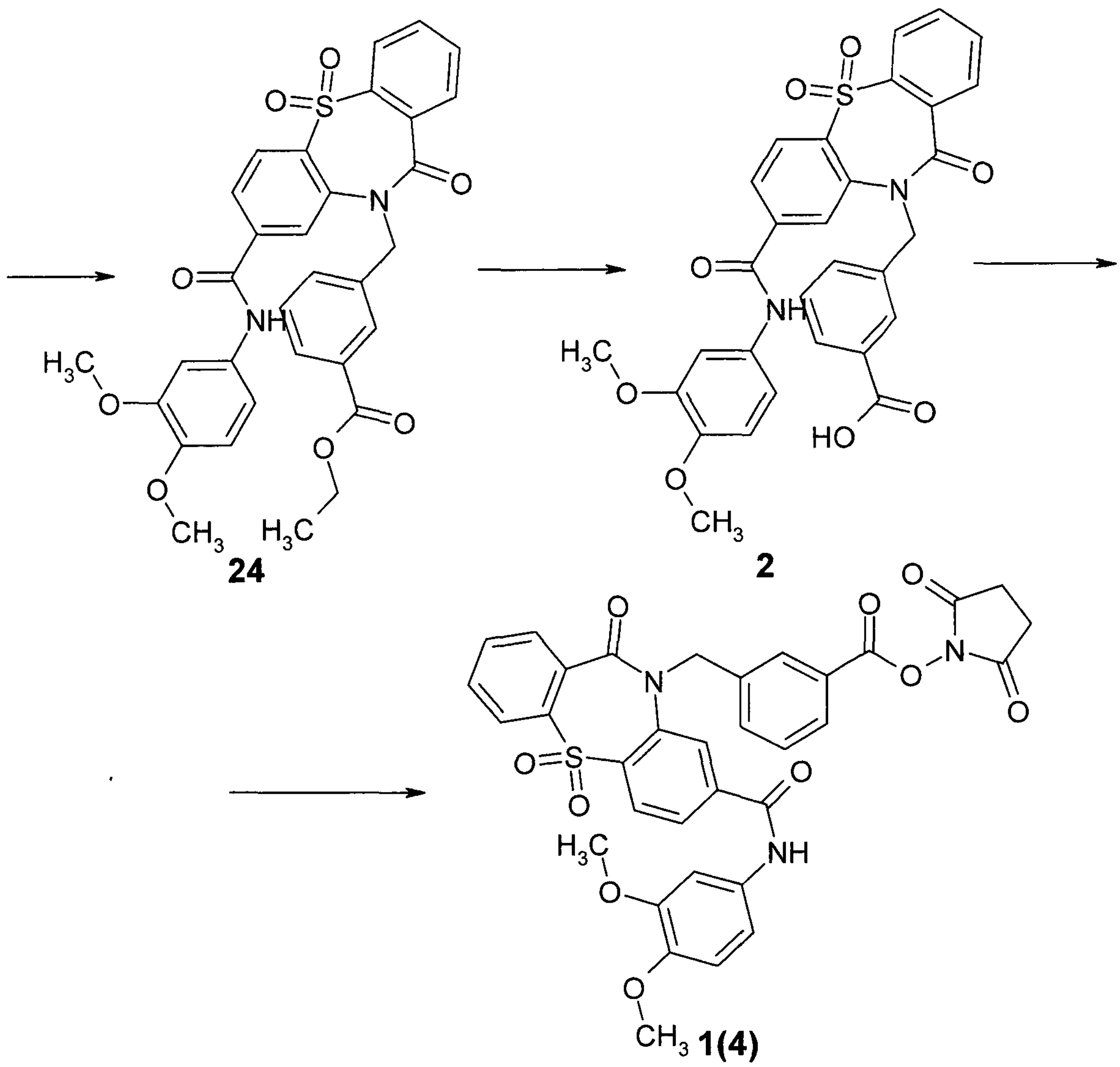

24

2

1(4)

**Scheme 4**.

2,4-Dimethoxybenzaldehyde (10 g) and 3,4-dimethoxyaniline (9.2 g) in toluene (150 ml) were refluxed with Dean-Stark trap until no more water separated. After cooling toluene was evaporated on rotary evaporator, obtained compound **20** was used further without purification. To a solution of compound **20** in methanol (100 ml) at stirring $NaBH_4$ (2.8 g) was added, the resultant mixture was stirred at room temperature for 1 h, methanol was evaporated on rotary evaporator, water was added (100 ml), rendered alkaline with 10% solution of KOH to pH=9, extracted with methylene chloride, dried, evaporated. It gave compound **21,** yield 80%. After stirring for 1 h to a suspension of compound **9** (2.2 g) and compound **21** (3.0 g) in dioxane (50 ml) were added triethylamine (5.5 ml) and phosphorous oxychloride (2.0 g). The resultant mixture was stirred for 3 h at 50°C, water (150 ml) was added, precipitated solid was filtered off, washed with water and dried. It gave compound **22,** yield 60%. To a solution of compound **22** (3.0 g) in DMF (30 ml) potassium carbonate (1.7 g) and ethyl 3-bromomethylbezoate (1.3 g) were added. The mixture was stirred at 50°C for night, DMF was evaporated *in vacuo*, water (70 ml) was added to the residue. Precipitated solid was filtered off, washed with water and dried. It gave compound **23,** yield 90%. To a solution of compound **23** (3.0 g) in methylene chloride (40 ml) trifluoroacetic acid (1.4 g) was added, mixture was refluxed for night. After that the reaction mixture was washed with conc. solution of $NaHCO_3$, dried, methylene chloride was evaporated. The product was purified by column chromatography, eluent - chloroform : methanol 40:1. It gave compound **24,** yield 30%. LiOH (0.25 g) was added to a solution of compound **24** (0.9 g) in 50% aqueous ethanol (20 ml) and the mixture was stirred for night at room temperature. After that ethanol was evaporated on rotary evaporator, aqueous solution was acidified with 10% HCl to pH=3. Precipitated solid was filtered off, washed with water and dried. The product was purified by column chromatography, eluent - chloroform : methanol : triethylamine 10:1:1. It gave compound **25,** yield 20%. To a solution of compound **25** (140 mg) in THF (20

ml) in argon atmosphere at stirring N-hydroxysuccinimide (42 mg) and dicyclohexylcarbodiimide (76 mg) were added. The mixture was stirred for night at room temperature, then precipitated solid was filtered off, mother liquor was evaporated, the product was purified by flash chromatography on silica (eluent - ethyl acetate). It gave compound **1(4),** yield 50%. LCMS spectrum of compound **1(4): (M+1** = **670)** is represented in Fig. 4.

**Example 5.** 2,5-dioxopyrrolidin-1-yl ester of (4-{8-[3-(4-benzylpiperidin-1-yl)-propylcarbamoyl]-5,5,11-trioxo-5,11-dihydro-dibenzo[*b*,*f*][1,4]thiazepin-10-ylmethyl}-phenyl)-acetic acid **1(5)** was prepared according to Scheme 5 given below.

2,4-Dimethoxybenzaldehyde (3.8 g) and compound **26** (5.3 g) in toluene (100 ml) were refluxed with Dean-Stark trap until no water separated. After cooling, toluene was evaporated on rotary evaporator. The obtained compound **27** was used further without purification. To a solution of compound **27** in methanol (50 ml) at stirring $NaBH_4$ (1.3 g) was added, the mixture was stirred at room temperature for 1 h, methanol was evaporated on rotary evaporator, water (50 ml) was added, rendered alkaline with 10% solution of KOH to pH=9, extracted with methylene chloride, dried, evaporated. It gave compound **28,** yield 80%. To a solution of compound **9** (2.4 g) in THF (100 ml) in argon atmosphere at stirring N-hydroxysuccinimide (1.0 g) and dicyclohexylcarbodiimide (1.1 g) were added. The mixture was stirred for night, after that compound **28** (3 g) was added and mixture was stirred for 6 h at 60°C. Precipitated solid was filtered off, mother solution was evaporated on rotary evaporator. The residue was purified by column chromatography, eluent - chloroform : methanol 19:1. It gave compound **29,** yield 70%. A mixture of *p*-tolylacetic acid (10.0 g), *N*-bromosuccinimide (13.0 g) and 2,2'-azabisisobutyronitrile (0.1 g) in carbon tetrachloride (60 ml) was refluxed for 4 h. The mixture was cooled to room temperature, poured out on water (100 ml), precipitated solid was filtered off, and dried. To a solution of the above solid in ethanol (50 ml) at 0°C $SOCl_2$ (3.7 ml) was added, the mixture was stirred at night at room temperature, solvent was evaporated.

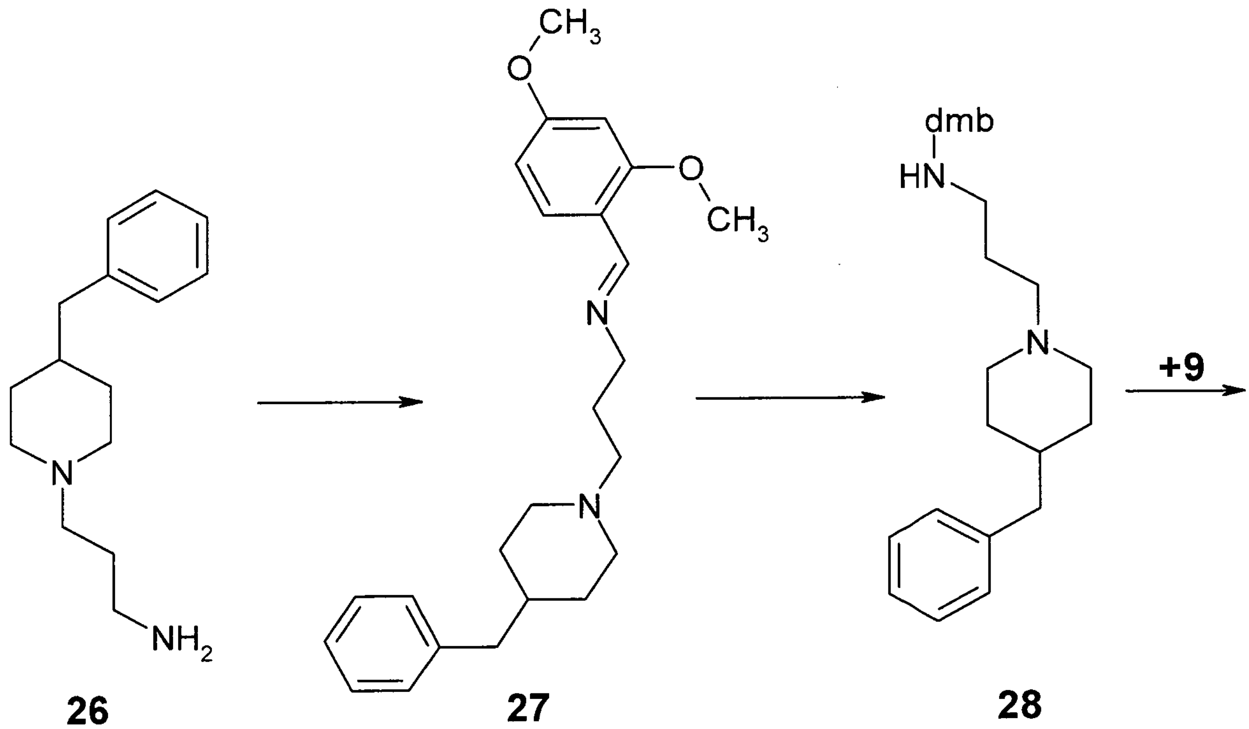


**26** **27** **28**

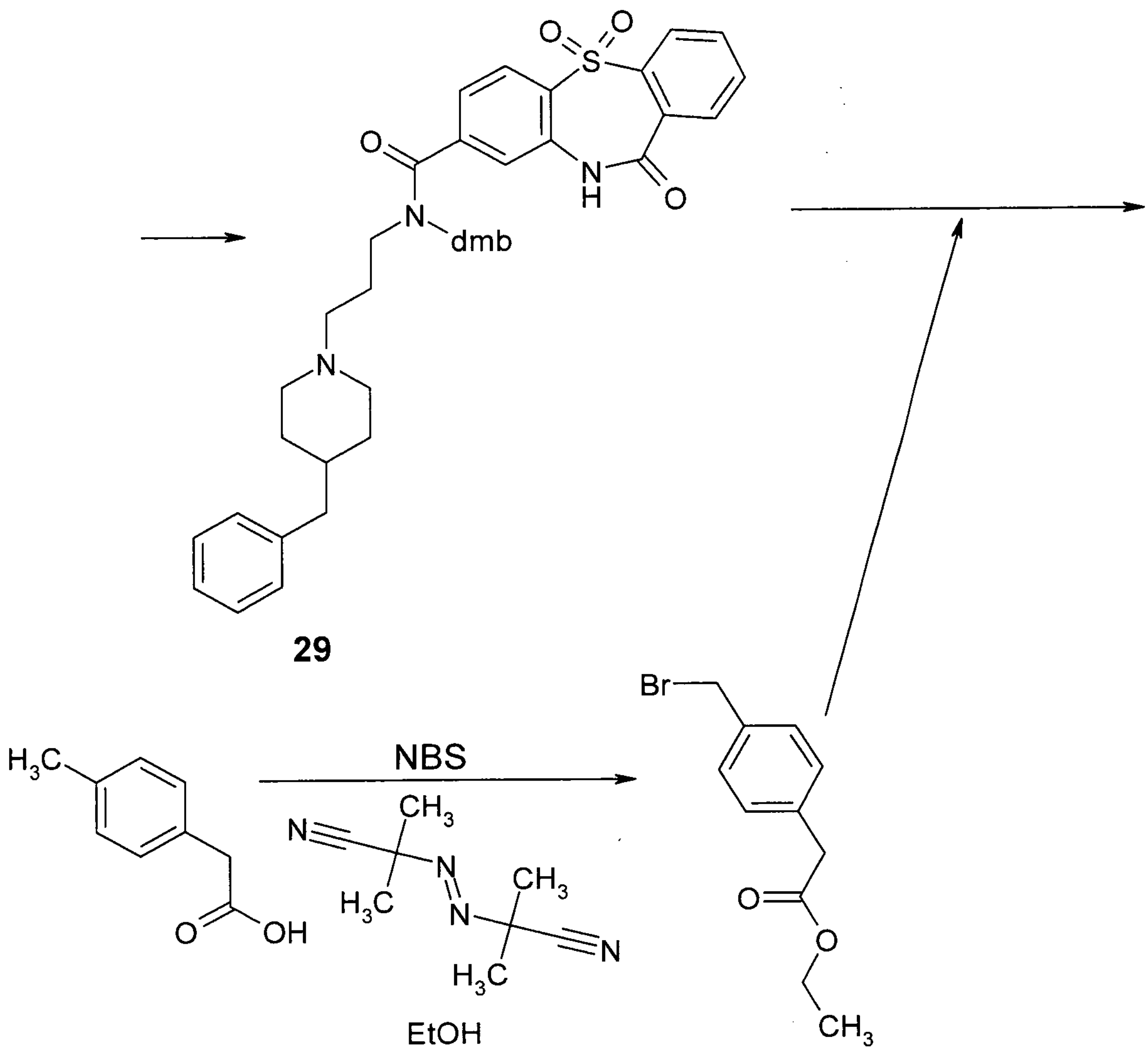


**29**

**30**

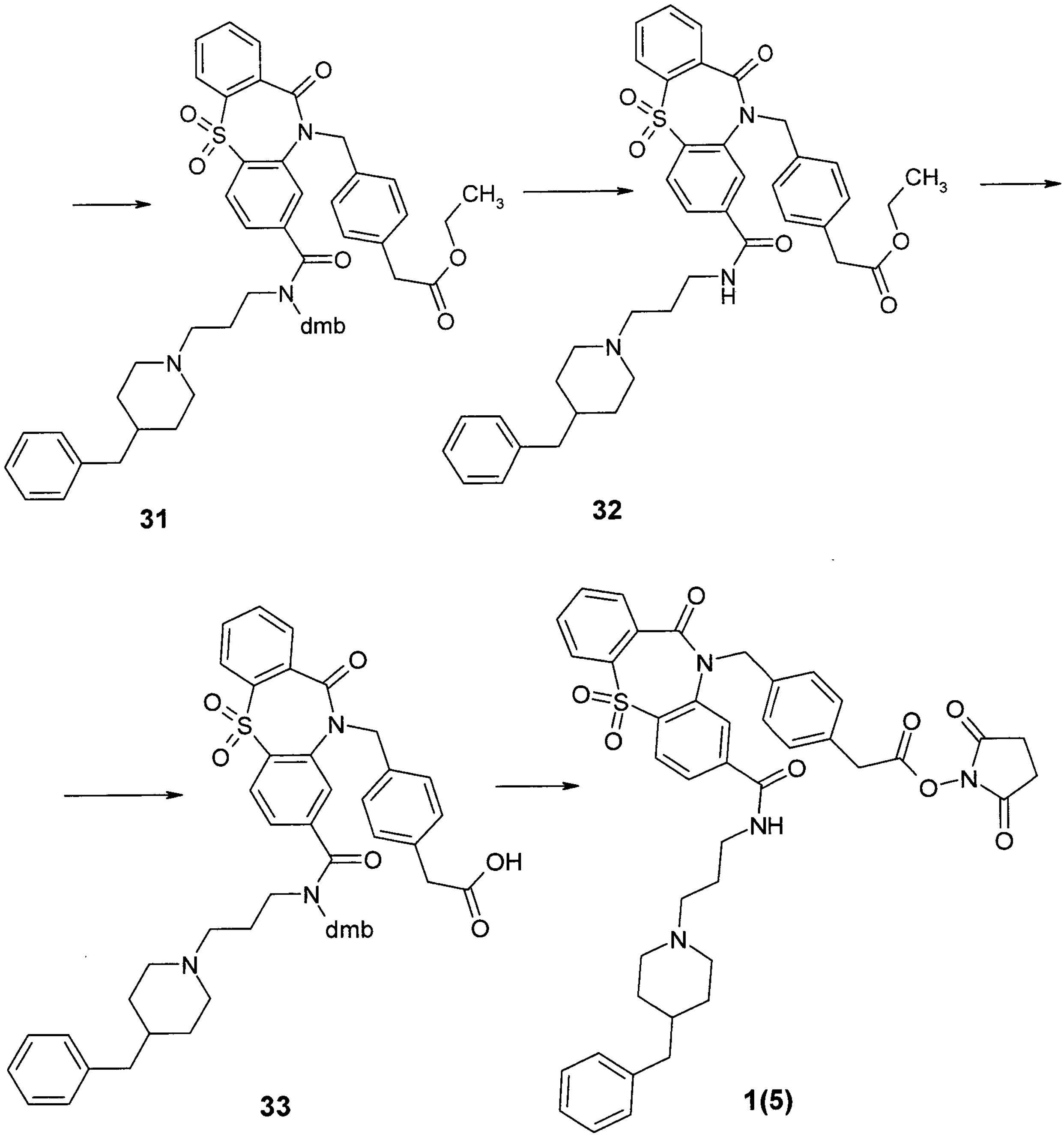

31 32 33 1(5)

**Scheme 5.**

It gave compound **30,** yield 50%. The obtained product was used further without purification. Potassium carbonate (2.2 g) and ethyl ether of *p*-bromomethylphenylacetic acid **30** (2.0 g) were added one after another to a solution of compound **29** (4.5 g) in DMF (50 ml).The mixture was stirred at 50°C for night, DMF was evaporated on rotary evaporator, water (100 ml) was added to the residue. Precipitated solid was filtered off, washed with water and dried. It gave compound **31,** yield 80%. Trifluoroacetic acid (2.0 g) was added to a solution of compound **31** (3.5 g) in methylene chloride (40 ml), the resultant mixture was refluxed for night. After that the mixture was shaken up several times with conc. solution of $NaHCO_3$, dried, methylene chloride was evaporated on rotary evaporator. The product was purified by column chromatography, eluent - chloroform : methanol 40:1. It gave compound **32,** yield 55%. LiOH (0.29 g) was added to a solution of compound **32** (1.6 g) in 50% aqueous ethanol (20 ml) and stirred at room temperature for night. Then ethanol was evaporated on rotary evaporator, water solution was acidified with 10% HCl to pH=3. Precipitated solid was filtered off, washed with water and dried. The product was purified by column chromatography, eluent - chloroform :

methanol : triethylamin 10:1:1. It gave compound **33,** yield 15%. N-hydroxysuccinimide (47 mg) and dicyclohexylcarbodiimide (84 mg) were added to a solution of compound **33** (180 mg) in THF (20 ml), in argon atmosphere and at stirring. Mixture was stirred at room temperature for night, precipitated solid was filtered off, mother solution was evaporated, product was purified by flash chromatography on silica (eluent - ethyl acetate). It gave compound **1(5),** yield 50%. NMR Spectrum of compound **1(5)** is given in Fig. 5.

**Example 6.** 10-(3-chlorobenzyl)-5,5,11-trioxo-10,11-dihydro-5*H*-dibenzo[b,f][1,4]thiazepine-8-carboxylic acid (2-aminoethyl)-amide **1(6),** was prepared according to Scheme 6 given below.

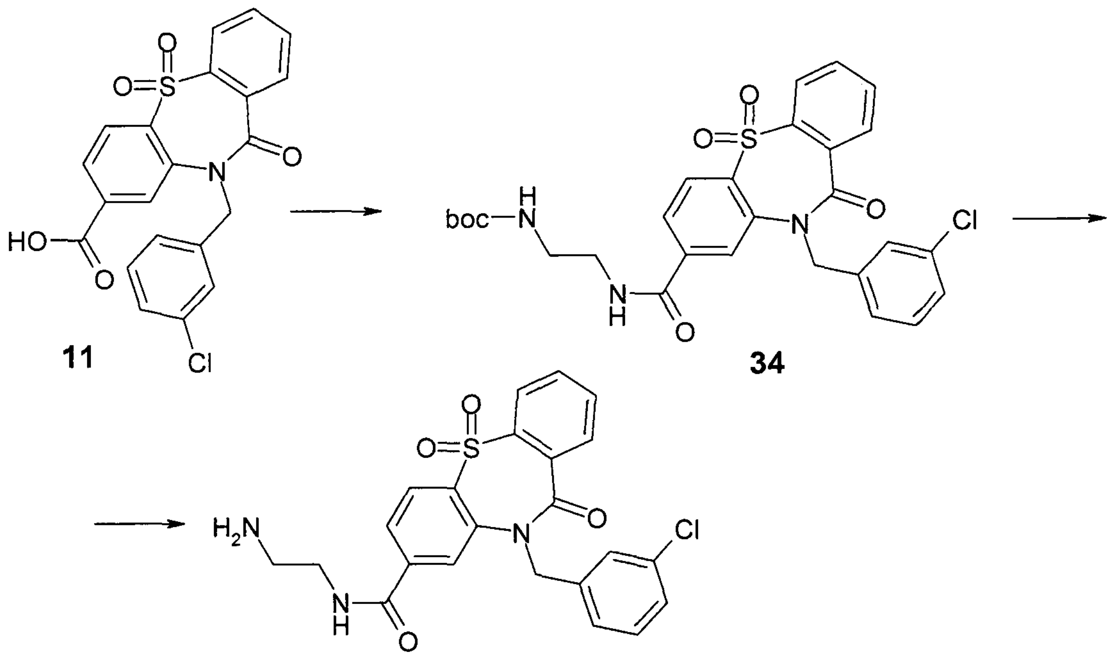

**11** **34**

**1(6)**

**Scheme 6.**

CDI (1.1 eq.) was added to a solution of compound **11** in chloroform, the resultant mixture was stirred at room temperature for 1 h, then *Boc*-ethylene diamine was added and the mixture stirred at room temperature for night. Washed with water, organic layer was evaporated. The product was purified by column chromatography, eluent - chloroform : methanol 20:1. It gave compound **34,** yield 70%.

Compound **34** was stirred in dioxane saturated with HCl at room temperature for 1 h, precipitated solid was filtered off. It gave compound **1(6),** yield 78%. NMR Spectrum of compound **1(6)** is given in Fig. 6.

**Example 7.** 10-{4-[(2-Amino-ethylcarbamoyl)-methyl]-benzyl}-5,5,11-trioxo-10,11-dihydro-5*H*-dibenzo[b,f][1,4]thiazepine-8-carboxylic acid [3-(4-benzyl-piperidin-1-yl)-propyl]-amide **1(7)** was prepared according to Scheme 7 given below.

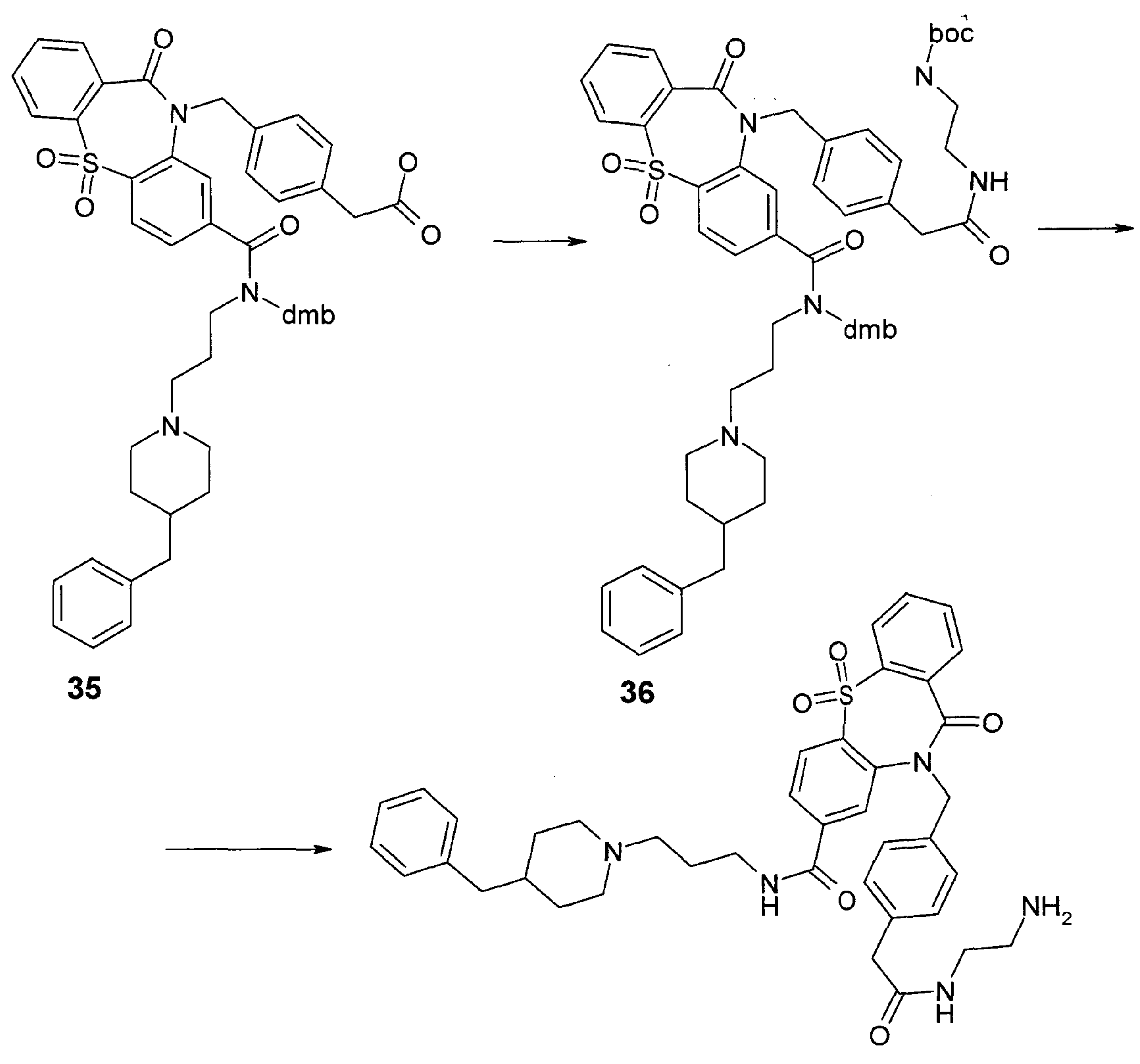

35

36

1(7)

**Scheme 7.**

CDI (1.1 eq.) was added to a solution of compound **35** in chloroform, the resultant mixture was stirred at room temperature for 1 h, then *Boc*-ethylene diamine was added and the mixture was stirred at room temperature for night. Washed with water, organic layer was evaporated. The product was purified by column chromatography, chloroform : methanol (20:1). It gave compound **36,** yield 70%. Compound **36** was stirred in dioxane saturated with HCl at room temperature for 1 h, precipitated solid was filtered off. It gave compound **1(7),** yield 78%. NMR Spectrum of compound **1(7)** is given in Fig. 7.

**Example 8.** 2-(2,5-dioxo-2,5-dihydro-pyrrol-1-yl)-ethyl ester 10-(3-chlorobenzyl)-5,5,11-trioxo-10,11-dihydro-5*H*-dibenzo[*b*,*f*][1,4]thiazepine-8-carboxylic acid **1 (8)** was prepared according to Scheme 8 given below.

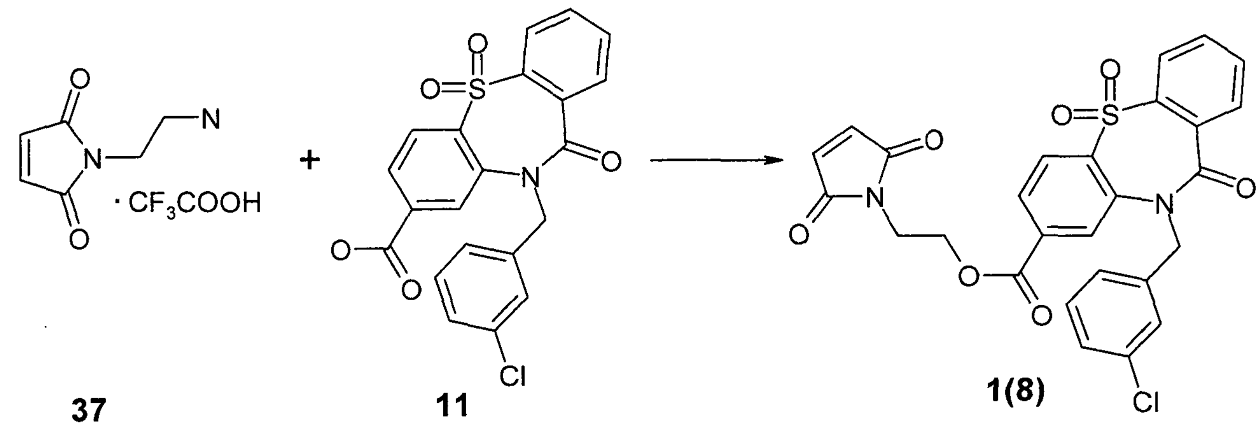

37 11 1(8)

**Scheme 8.**

Dicyclohexylcarbodiimide (1 eq.) and $Et_3N$ (1 eq.) were added at stirring to a solution of compounds **37** and **11** in THF in ratio 1:1. The mixture was stirred for night at 50°C, cooled, precipitated solid was filtered off, mother solution was evaporated, product was purified by HPLC method. It gave compound **1(8),** yield 10%. NMR Spectrum of compound **1(8)** is given in Fig. 8.

**Example 9.** 10-(4-{[2-(2,5-dioxo-2,5-dihydro-pyrrol-1-yl)-ethylcarbamoyl]-methyl}-benzyl)-5,5,11-trioxo-10,11-dihydro-5*H*-dibenzo[b,f][1,4]thiazepine-8-carboxylic acid [3-(4-benzyl-piperidin-1-yl)-propyl]-amide **1(9)** was prepared according to Scheme 9 given below.

Dicyclohexylcarbodiimide (1 eq.) and $Et_3N$ (1 eq.) were added at stirring to a solution of compounds **35** and **37** in THF in ratio 1:1. The mixture was stirred for night at 50°C, cooled, precipitated solid was filtered off, mother solution was evaporated, product was purified by HPLC method. It gave compound **1(9)**, yield 10%. NMR Spectrum of compound **1(9)** is given in Fig. 9.

**35 + 37**

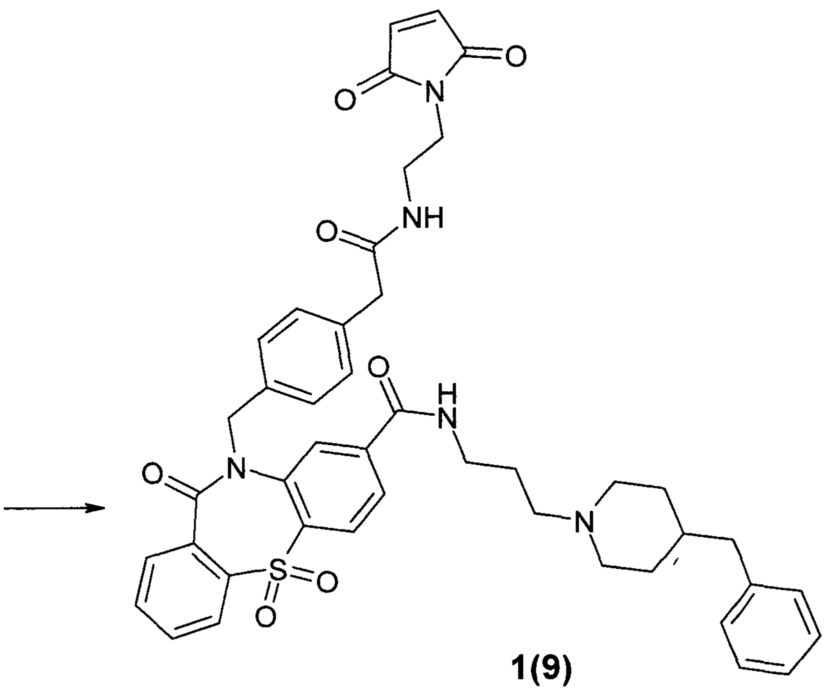

1(9)

**Scheme 9.**

**Example 10.** *N*-[2-({*N*-(methoxycarbonyl)-*N*-[(1,5-dimethoxy-1,5-dioxopentan-2-yl)carbamoyl]-β-alanyl}amino)ethyl]-10-(3-chlorobenzyl)-5,5,11-trioxo-10,11-dihydrodibenzo[*b*,*f*][1,4]thiazepine-7-carboxamide **1(10)** was prepared according to Scheme 10 given below.

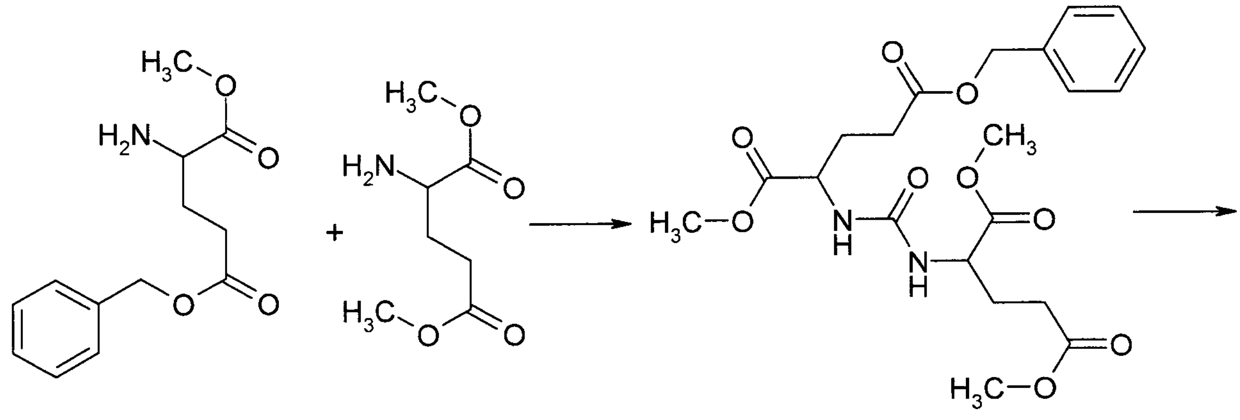

38 39 40

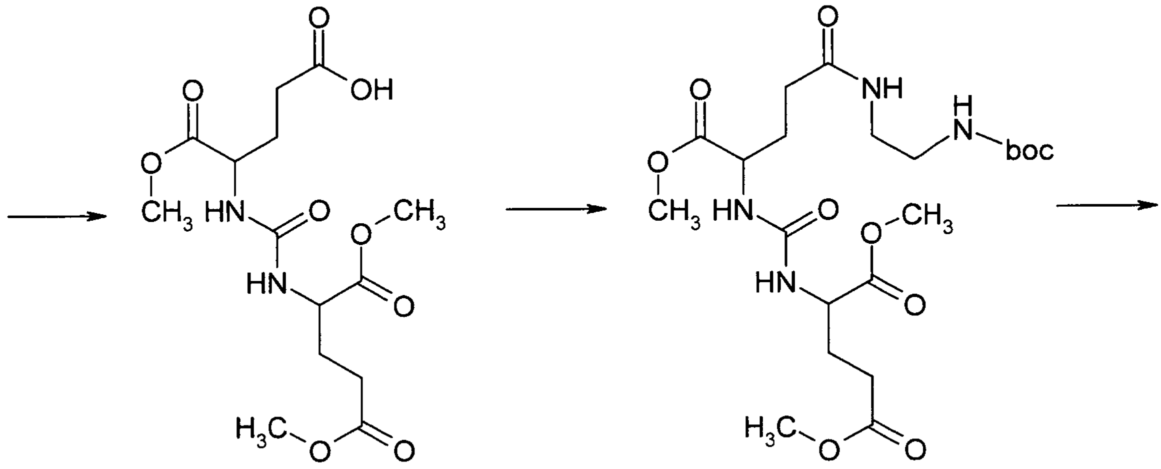

41 42

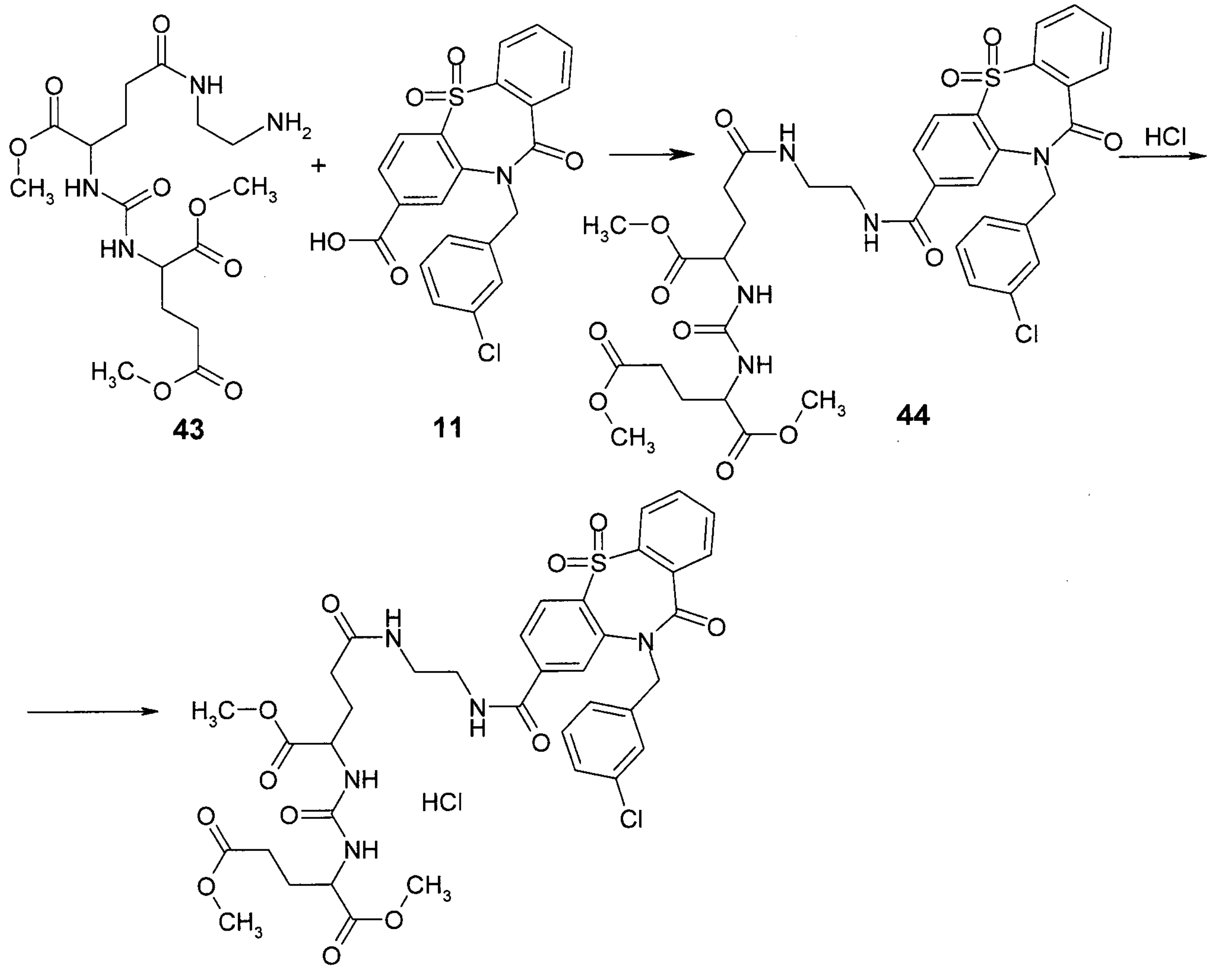


43 11 44

1(10)*HCl

## Scheme 10.

A solution of compound **38** (11 g, 0.038 mol) and DIPEA (12.6 g, 0.098 mol) in $CH_2Cl_2$ was slowly dropped at 0°C to a suspension of triphosgene(4.2 g, 0014 mol) in $CH_2Cl_2$. The mixture was stirred for 30 min at 0°C, then a solution of compound **39** (8.1 g, 0.038 mol) and DIPEA (10.8 g, 0.084 mol) in $CH_2Cl_2$ was added in one portion. The reaction mixture was additionally stirred for 15 min., organic layer was separated, washed with water and subjected to chromatography on silica, eluent-$CHCl_3$ : MeOH (98:2). It gave compound **40,** yield 53%. To a solution of compound **40** (6 g) in ethyl acetate 10% Pd/C (0.6 g) was added. The mixture was hydrogenated in autoclave at 2 atm. for 17 h. The catalyst was filtered off, filtrate was evaporated. It gave 5 g of compound **41,** which was used in the next stage without additional purification. CDI (2.6 g, 0.016 mol) was added to a solution of compound **41** (4.8 g, 0.013 mol) in $CH_2Cl_2$ at room temperature, and in 2 h *Boc*-ethylene diamine (2.2 g, 0.013 mol) was added. The mixture was stirred for 18 h, diluted with water, the product was extracted with $CH_2Cl_2$ and subjected to chromatography on silica, eluent - $CHCl_3$ : MeOH (99:1). It gave 4.4 g of compound **42,** yield 66%. 3N HCl (5eq.) in dioxane was added to a solution of compound **42** (4.4 g) in dioxane. The mixture was stirred at room temperature for night and evaporated to dryness. It gave compound **43,** yield 90%. Compound **43** (0.8 g, 0.002 mol), compound **11** (0.85 g, 0.002 mol), $NEt_3$ (0.4 g, 0.004 mol) and *N*-(dimethylaminopropyl)-*N'*-ethylcarbodiimide hydrochloride (0.4 g, 0.002 mol) were mixed together in THF. The reaction mixture was stirred at 60°C for two days. Then $LiOH{\cdot}H_2O$ (0.5 g, 0.012 mol) and water (2 ml) were added. In 24 h was added acetic acid (0.72 g, 0.012 mol). The reaction mixture was evaporated and the product was purified by HPLC method. It gave compound **44,** yield 7%. NMR spectrum of compound **1(10)** is represented in Fig. 10. Hydrochloride **1(10)*HCl** was prepared by addition of saturated ethyl acetate solution of HCl to compound **1(10).** Precipitated solid was filtered off, dried, and purified by crystallization. Hydrochlorides of compounds preparation of which was described in examples 1-9 and 11-14 were obtained in analogous manner.

**Example 11.** *N*[5]-(2-{[(4-{[7-{[[3-(4-Benzylpiperidin-1-yl)propyl](phenyl)amino]-carbonyl}-5,5,11-trioxo-dibenzo[*b*,*f*][1,4]thiazepin-10(11*H*)-yl]methyl}phenyl)acetyl]-amino}ethyl)-*N*[2]-{[(1,3-dicarboxypropyl)amino]carbonyl}glutamine **1(11)** was prepared according to Scheme 11 given below.

Acid 35 (1.6 g, 0.0024 mol), ester **43** (1 g, 0.0024 mol), $NEt_3$ (0.5 g, 0.0048 mol) and N-(dimethylaminopropyl)-*N'*-ethylcarbodiimide hydrochloride (0.45 g, 0.0024 mol) were mixed together in THF. The reaction mixture was stirred at 60°C for 2 days. Then, without isolating compound **45,** $LiOH{\cdot}H_2O$ (0.6 g, 0.0144 mol) and water (2 ml) were added. In 24 h was added acetic acid (0.86 g, 0.014 mol). The reaction mixture was evaporated and the product was purified by HPLC method. It gave compound **1(11),** yield 10%. NMR spectrum of compound **1(11)** is represented in Fig. 11.

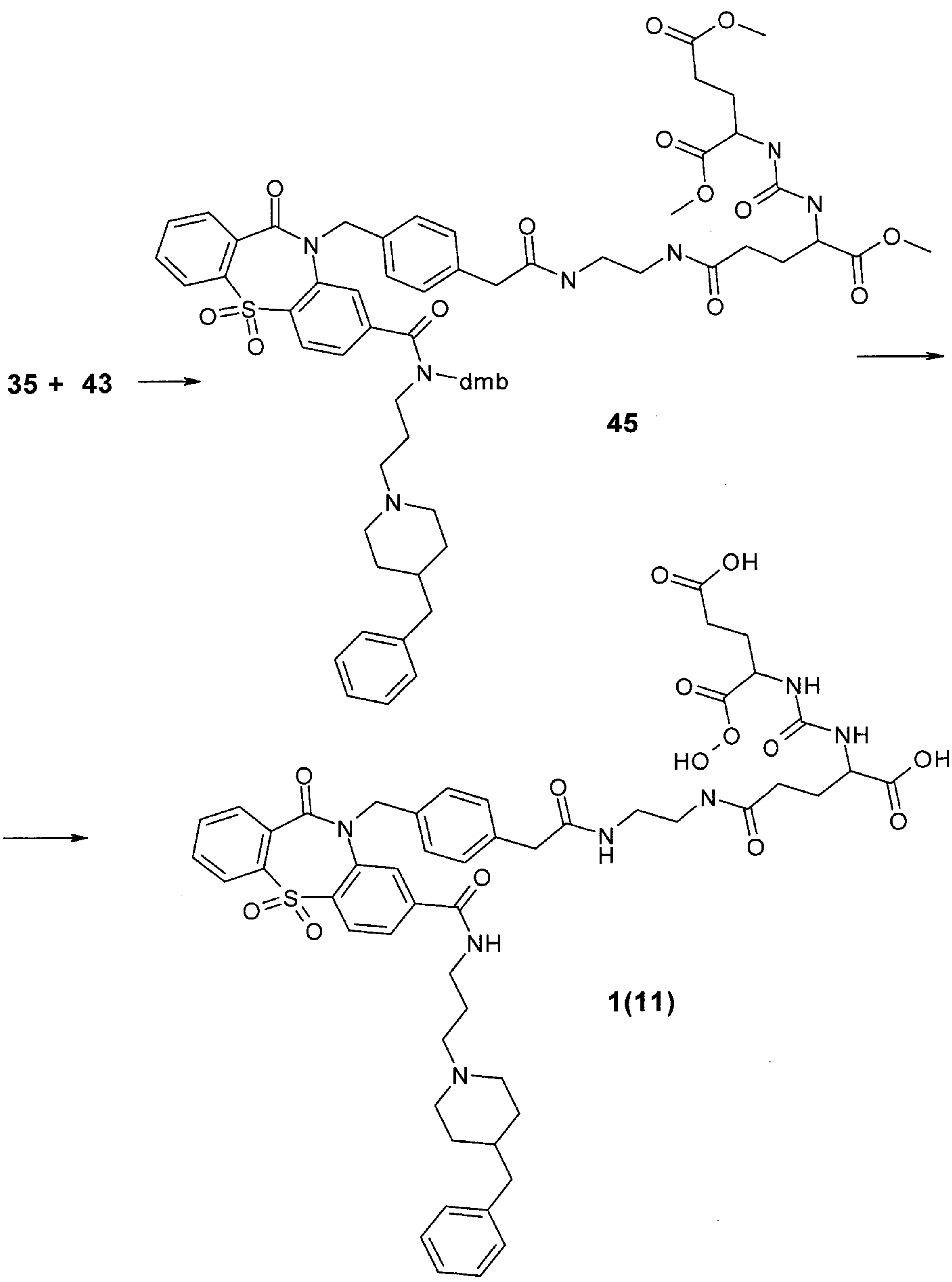

35 + 43

45

1(11)

**Scheme 11.**

**Example 12.** 4-[4-(dimethylamino)phenyl]-*N*-(4-{[(2,5-dioxopyrrolidin-1-yloxy]carbonyl}phenyl)-7,8,9,10-tetrahydro-4*H*-[1]benzothieno[3,2-*f*]pyrrolo[1,2-*a*] [1,4]diazepine-5(6*H*)-carboxamide **2(1)** was prepared according to Scheme 12 given below.

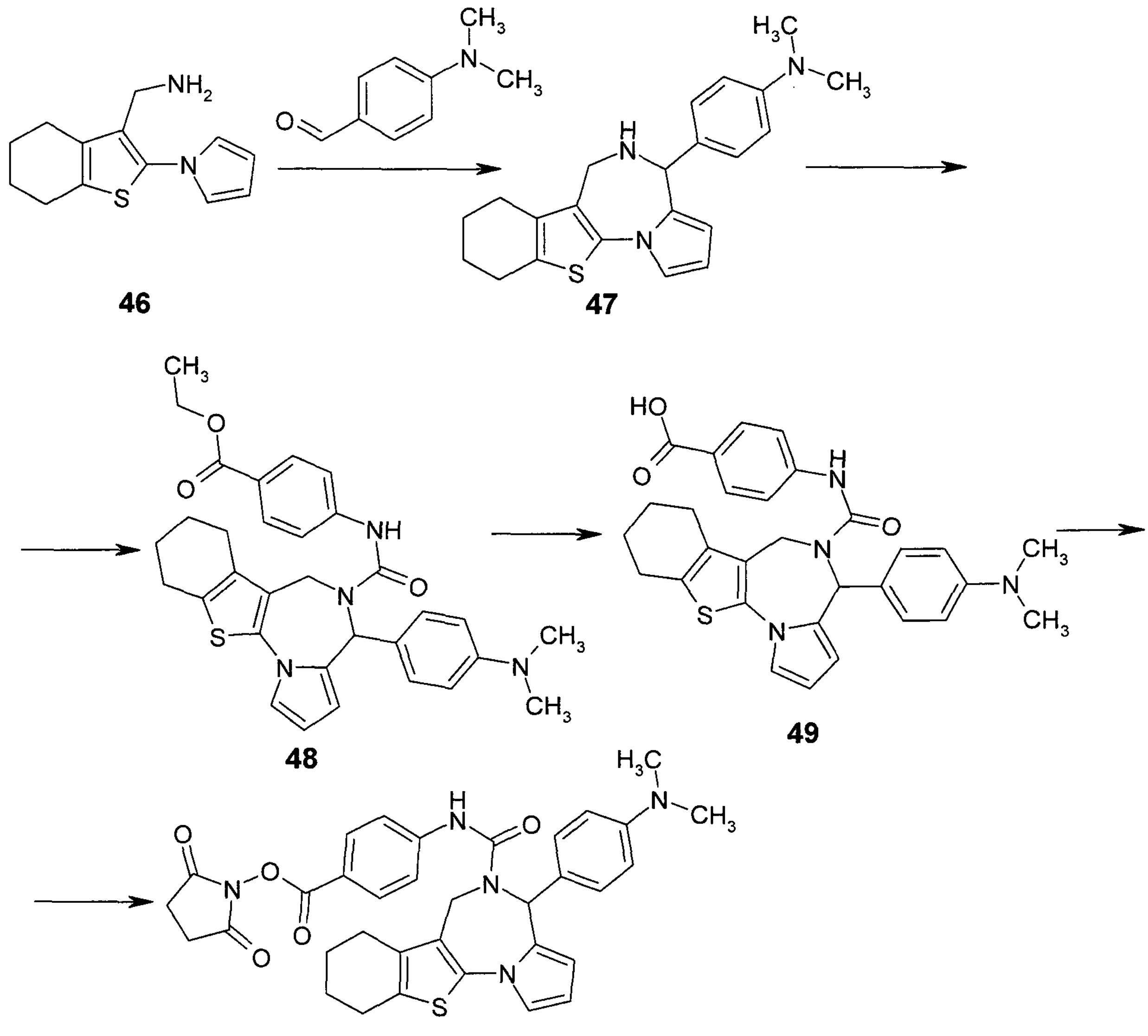

46 47 48 49

2(1)

**Scheme 12.**

A mixture of compound **46** (3.0 g) and 4-dimethylaminobenzaldehyde (1.7 g) in ethanol (40 ml) was refluxed for 10 h, cooled to room temperature, the precipitated solid was filtered off. The precipitate was dissolved in water (50 ml) and rendered alkaline with 10% KOH to pH=10. Precipitated solid was filtered off, washed with water and dried. It gave compound **47,** yield 35%. A solution of compound **47** (0.6 g) and ethyl 4-isocyanatobenzoate (0.32 g) in dioxane (30 ml) was stirred at room temperature for night. Precipitated solid was filtered off. It gave compound **48,** yield 30%. LiOH (32 mg) was added to a solution of compound **48** (140 mg) in 50% aqueous ethanol (5 ml), the reaction mixture was stirred at room temperature for night. Ethanol was evaporated on rotary evaporator, water solution was acidified with 10% HCl to pH=3. Precipitated solid was filtered off, washed with water and dried. It gave compound **49,** yield 60%. *N*-Hydroxysuccinimide (22 mg) and dicyclohexylcarbodiimide (39 mg) were added in argon atmosphere at stirring to a solution of compound **49** (80 mg) in THF (10 ml). The mixture was stirred at room temperature for night, then precipitated solid was filtered off, mother solution was evaporated, the product was purified by flash chromatography on silica (eluent - ethyl acetate). It gave compound **2(1),** yield 50%. LCMS spectrum of compound **1(12)** is represented in Fig. 12 (M+1) = 624.

**Example 13.** 4-[4-(Dimethylamino)phenyl]-*N*-(4-{2-[(2,5-dioxopyrrolidin-1-yl)oxy]-2-oxoethoxy}phenyl)-7,8,9,10-tetrahydro-4*H*-[1]benzothieno[3,2-*f*]pyrrolo[1,2-*a*][1,4]diazepine-5(6*H*)-carboxamide **2(2)** was prepared according to Scheme 13 given below.

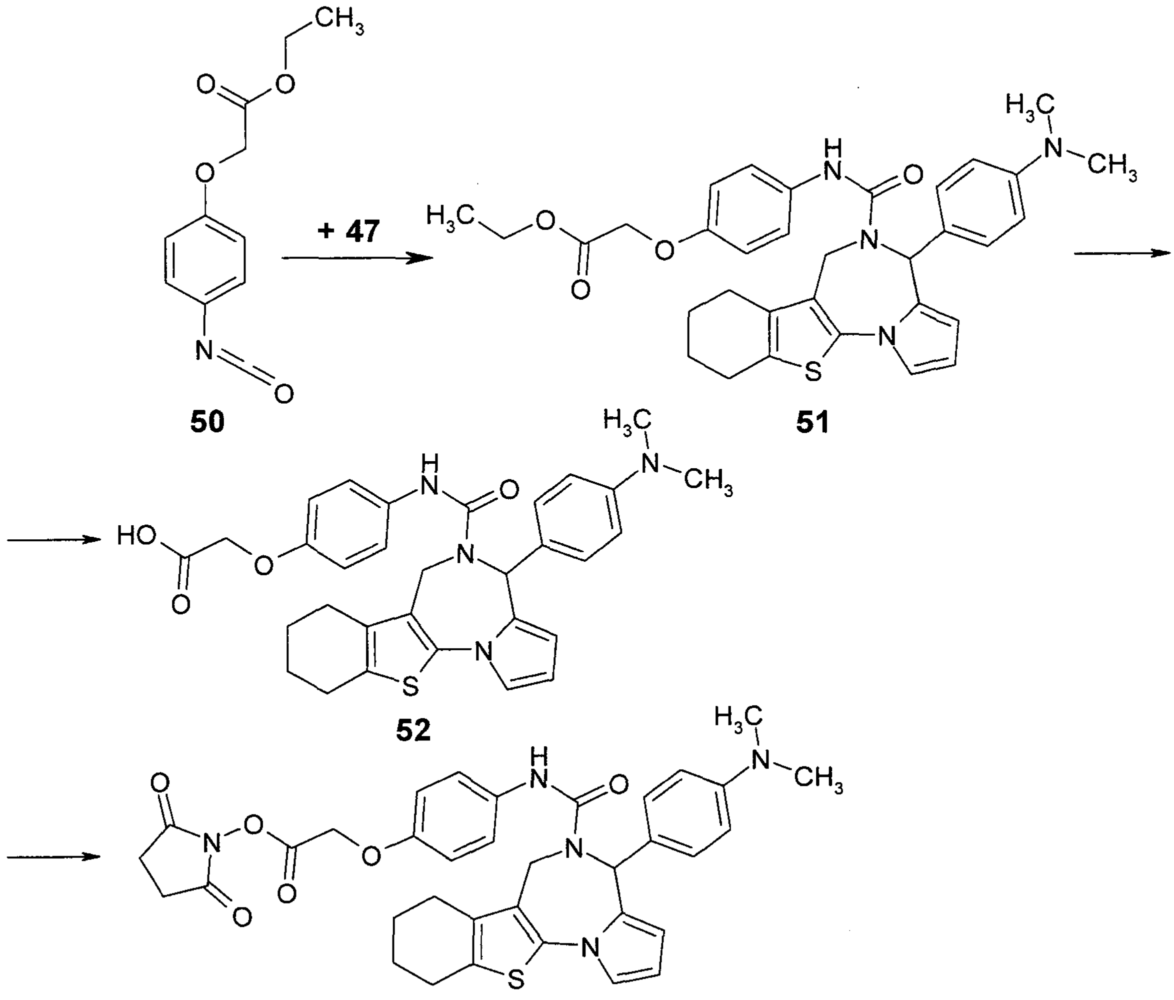


50

51

52

2(2)

**Scheme 13.**

Compound **50** (0.8 g) and compound **47** (0.5 g) were dissolved in dioxane (30 ml) and stirred for night at room temperature. Solvent was evaporated, the residue was purified by column chromatography. Eluent - chloroform : methanol 39:1. It gave compound **51,** yield 5%. LiOH (14 mg) was added to a solution of compound **51** (6 mg) in 50% aqueous ethanol (5 ml), and the resultant mixture was stirred at room temperature for night. After that ethanol was evaporated on rotary evaporator, water solution was acidified with 10% HCl to pH 3. Precipitated solid was filtered off, washed with water and dried. It gave compound **52,** yield 60%. *N*-Hydroxysuccinimide (10 mg) and dicyclohexylcarbodiimide (19 mg) were added at stirring to a solution of compound **52** (50 mg) in THF (10 ml) in argon atmosphere. The mixture was stirred at room temperature for night, then the precipitated solid was filtered off, mother solution was evaporated, the product was purified by flash chromatography on silica (eluent - ethyl acetate). It gave compound **1(13),** yield 50%. NMR Spectrum of compound **2(2)** is represented in Fig. 13.

**Example 14.** 2,5-Dioxopyrrolidin-1-yl *N*-[4-(5-{[(3,4-dimethoxyphenyl)amino]-carbonyl}-5,6,7,8,9,10-hexahydro-4*H*-[1]benzothieno[3,2-*f*]pyrrolo[1,2-*a*][1,4]diazepin-4-yl)phenyl]-*N*-methylglycinate **2(3)** was prepared according to Scheme 14 given below.

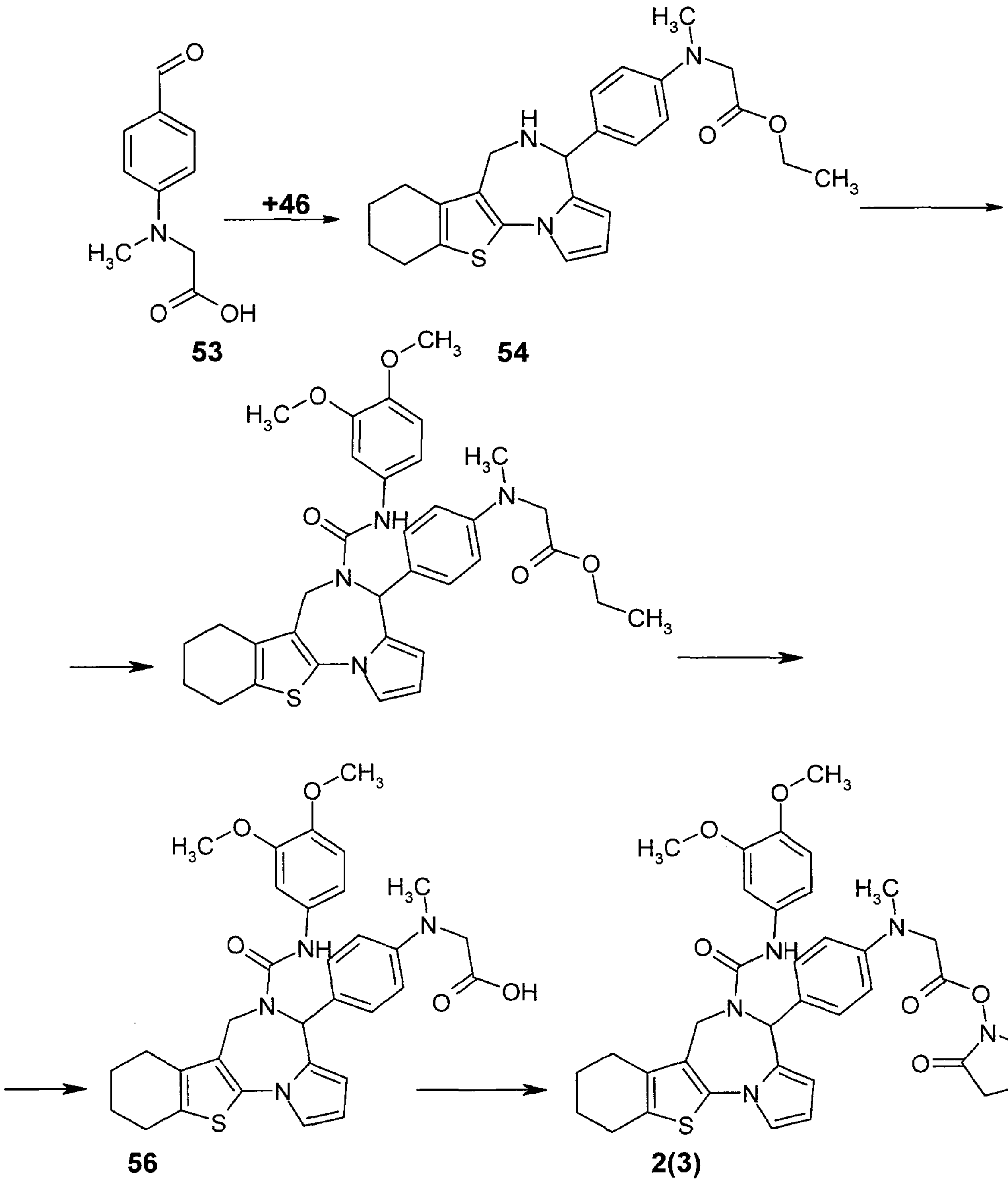


53 54 56 2(3)

**Scheme 14.**

A mixture of compound **46** (1.68 g) and compound **53** (1.45 g) in ethanol (25 ml) was refluxed for 10 h, cooled to room temperature, the precipitated solid was filtered off and dissolved in water (50 ml) and rendered alkaline with 10% KOH to pH 10. Precipitated solid was filtered off, washed with water and dried. It gave compound **54,** yield 19%. A solution of compound **54** (0.54 g) and dimethoxyaniline (0.29 g) in dioxane (10 ml) was stirred for night at room temperature. Then, the solvent was evaporated, the residue was purified by column chromatography. Eluent - chloroform : methanol 39:1. It gave compound **55,** yield 65%. LiOH (32 mg) was added to a solution of compound **55** (500 mg) in 50% aqueous ethanol (5 ml), and the resultant mixture was stirred for night at room temperature. Ethanol was evaporated on rotary evaporator, water solution was acidified with 10% HCl to pH 3. Precipitated solid was filtered off, washed with water and dried. It gave compound **56,** yield 60%. *N*-Hydroxysuccinimide (71 mg) and dicyclohexylcarbodiimide (127 mg) were added at stirring and in argon atmosphere to a solution of compound **56** (360 mg) in THF (10 ml). The resultant

mixture was stirred for night at room temperature, then the precipitated solid was filtered off, mother solution was evaporated, the product was purified by flash chromatography on silica (eluent - ethyl acetate). It gave compound **2(3),** yield 50%. LCMS spectrum of compound **2(3)** is represented in Fig. 14 (M+1) = 684.

**Example 15.** The general method for preparation of modified proteins (conjugates). A protein was modified by a compound of the general formula **1** or **2** in molar ratio from 1:1 to 1:100. To a weight of compound of the general formula **1** or **2** dissolved in DMSO (50 mcl) were slowly added phosphate-buffered saline (PBS, pH 7.4) and a solution of protein in PBS. The final protein concentration in the reaction mixtures was 1 mg/ml. The reaction was carried out at room temperature for 20 h at constant stirring. The reaction mixture was centrifuged, filtrate was applied to a Superdex gel 75 (GE, USA); height of the colomn support - 23 cm; mobil phase - PBS; flow rate - 57.3 cm/h; injection volume - 2.2% of the volume of the carrier. Conjugate purity was determined by HPLC method using affinity column Bio-Monolith Protein A (Agilent, CLLIA).

According to this method the following conjugates, in particular, were obtained:

**CR1(1) -** rituximab **(R)** with compound **1(1),** in ratio 1:10,
**CR1(2) -** rituximab **(R)** with compound **1(2),** in ratio 1:100,
**CR1(3) -** rituximab **(R)** with compound **1(3),** in ratio 1:100,
**CR1 (4) -** rituximab **(R)** with compound **1(4),** in ratio 1:100,
**CR1(5) -** rituximab **(R)** with compound **1(5),** in ratio 1:10,
**CR2(1) -** rituximab **(R)** with compound **2(1),** in ratio 1:100,
**CC1(1) -** cetuximab **(C)** with compound **1(1),** in ratio 1:10,
**CI1(1) -** interferon alfa **(I)** with compound **1(1),** in ratio1:10,
**CM1(1) -** myelin protein **(M)** with compound **1(1),** in ratio 1:10,
**CC1(1) -** complement C1q protein **(C)** with compound **1(1),** in ratio 1:10.

As an example FIG. 15 shows chromatogram of conjugate CR1 (1), indicating assay percentage of more than 98%.

**Example 16.** Determination of conjugate activity towards CD16a receptor. The experiment uses standard materials and equipment for enzyme immunoassay. Into wells of a 96-well plate CD16a was sorbed from a solution of 3.3 μg/ml in PBS in a volume of 75 μl/well. Sorption was carried out at 4°C during 16 h. CD16a Solution was removed from the plate, wells were filled in with 2% solution of bovine serum albumin (BSA) in PBS-p (150 μl/well). Plates were incubated at 37°C for 2 h and three times washed with PBS-p (each time by 300 μl/well). Then the wells were filled with solutions of the conjugates in PBS-P, 50 μl/well in dilutions from 0.25 to 256 μg/ml. To each dilution of the conjugate three wells corresponded in the plate. In order to control non-specific sorption of samples some wells were filled with 2% BSA solution. The plate was incubated 1 h at 37°C in a shaker for ELISA (rotation speed was 180 rpm). The plate was washed five times with 300 μl/well of PBS-p and filled with a solution of a protein modified with horseradish peroxidase (by 75 μl/well in the working dilution, which is given in the description of immunoconjugate). The plate was incubated for 30 minutes, washed five times with 300 μl/well of PBS-p and filled with a solution of TMB (100 μl/well). Depth of enzymatic reaction was estimated visually, it was stopped by the addition of 500 mM solution of sulfuric acid by 50 μl/well. The optical density in the plate wells was measured at 450 nm at room temperature. Analysis of the experimental results was carried out using program GraphPad Prism 5.0

Fig. 16 shows plots of binding for some of the conjugates towards CD16a receptor from which dissociation constants (Table 1) of conjugate complexes with CD16a receptor were calculated (conjugate activity towards CD16a receptor - $K_d$). As can be seen from the Table activity of rituximab conjugates is 10-100 times higher than the activity of the unconjugated rituximab.

**Table 1.** Activity of ($K_d$) rituximab and its conjugates towards CD16a receptor.

| | **R** | Rituximab conjugates | | | | | |
|---|---|---|---|---|---|---|---|
| | | **CR1(4)** | **CR2(1)** | **CR1(3)** | **CR1 (5)** | **CR1(1)** | **CR1(2)** |
| **$K_d$, M** | $1{,}5x10^{-6}$ | $1{,}9x10^{-7}$ | $7{,}4x10^{-8}$ | $6{,}5x10^{-8}$ | $8{,}0x10^{-8}$ | $1{,}3x10^{-8}$ | $1{,}0x10^{-8}$ |

Fig. 17 shows the dependence of binding of interferon alpha and its conjugate with compound **1 (1)** with CD16a receptor. Dependence of optical density at 450 nm on the conjugate concentration used in IFA. As can be seen from Table 2 activity of interferon conjugate is exceeded activity of unconjugated interferon alfa.

**Table 2.** Activity of ($K_d$) interferon alfa **(I),** and its conjugate **CI1(1)** towards CD16a receptor.

| | **I** | **CI1(1)** |
|---|---|---|
| $K_d$, M | $2{,}3x10^{-7}$ | $6{,}0x10^{-8}$ |

**Example 17**. Conjugate effectiveness in test for antibody-dependent (rituximab-dependent) cellular cytotoxicity. Effectiveness of rituximab conjugates in comparison with effectiveness of unconjugated rituximab was estimated in test for antibody-dependent cellular cytotoxicity. CD20-Positive cells of Daudi line were used as target cells (B-cell Burkitt's lymphoma). Pooled peripheral mononuclear blood cells from five healthy donors isolated by standard protocols were used as effector cells.

Dilutions of investigated conjugates and rituximab were prepared in RPMI 1640 medium. 50 μl of solutions of investigated conjugates and rituximab were placed in the wells of 96-well round bottom plate. Cells of Daudi line were resuspended in RPMI 1640 medium up to concentration of $8x10^5$ cells/ml. By 75 μl of this cell suspension was added to the prepared solutions of rituximab in the plate. The plate was incubated in thermostat at 37°C for 50 min. 75 μl of the cell suspension PBMC (thawed immediately before the experiment) was added into the wells to Daudi cells at a ratio of 50:1 (PBMC : cells Daudi). As control solutions RPMI 1640 medium was added (negative control containing effector cells only), as well as a 4% solution of Triton X-100 (positive control containing only target cells). Plates were centrifuged for 3 minutes at 500 rpm. The plates were incubated in thermostat for 8 hours at 37°C.

After incubation, the samples were precipitated by centrifugation at 1200 rpm for 10 minutes. 50 μl of supernatant were transferred into a new plate, where they were mixed with 50 μl of the reaction mixture (set for detection of lactate dehydrogenase, Promega (USA), catalog number G1780). The resulting mixture was incubated at room temperature for 25 minutes, then, the reaction was stopped. Absorption was measured on a spot spectrophotometer at a wavelength of 490 nm.

Cytotoxicity was calculated by the formula (all values are given in absorbance units):

$$\text{cytotoxicity, \%} = 100^*(A - (S_E\text{-}S_T))/(H_E\text{-}S_T),$$

where A -level of signal in the mixture of effector and target cells;
$S_E$ -signal level of effector cells;
$S_T$ - signal level of target cells;
$H_E$ - signal corresponding to the maximum lysis of target cells.

Measurement results of effectiveness of the prepared conjugates was demonstrated by an example of conjugate **CR**1(1) (Fig. 18) CON 4564. The prepared conjugate exhibited increased cytotoxic effect on target cells (Daudi). In particular, a ~ 35% lysis of cells is achieved at rituximab concentration about ~ 1 μg /ml, at the same time, an analogous cytotoxic effect of conjugate **CR1 (1)** CON 4564 is achieved at concentration of ~ 0.001 μg/ml. Similar dependence was observed in the case of conjugate **CR**1(2) (Fig. 19). Thus, the activity of rituximab conjugates is 2-3 orders of magnitude higher than that of rituximab itself.

**Example 18. Preparation of conjugates rituximab CR1(7) and trastuzumab CT1(7).** To 1.5 g of sugar moiety of an antibody in solution of 100 mM sodium acetate, 200 mM sodium chloride, pH 5.5 was added 2.0 g of sodium periodate, the resultant mixture was incubated for 30 min at room temperature in dark at stirring. Compound **1(7)** (1200 mg) was dissolved in DMSO (4 ml). Sodium hydrogen carbonate (8.4 g) was added to a solution of antibody, then compound **1(7)** dissolved in DMSO (1230 μl) was added dropwise and the resultant mixture was incubated for 2 hours.

Purification was carried out on SP-Sepharose (colomn XK26 GE) using stepwise gradient NaCl (buffer 1: 25 mM sodium citrate, pH 4.5. Buffer 2:25 mM sodium citrate, pH ~ 10, 270 mM). Antibody conjugate was concentrated in cell Amicon through membrane Millipore (NMWL 30000, cat. # PLTK07610) to a final conjugate concentration of 10 mg/ml. After filtration Polysorbate 80 was added to conjugate up to final concentration of conjugate **CR1 (7)** or **CT1 (7)** 0.01 %.

**Example 19.** Comparative effectiveness of rituximab (**R**) and trastuzumab (**T**) and their conjugates **CR1(7)**, **CT1(7)** towards CD16a receptor. Investigation of binding of obtained trastuzumab and rituximab conjugates with CD16a receptor was carried out in a manner identical to that described in **example 17**.

Comparative effectiveness and activity ($K_d$) towards CD16a receptor of rituximab (**R**), trastuzumab (**T**) and their conjugates **KP1(7)**, **KT1(7)** in test antibody-dependent cytotoxicity is represented in Fig. 20.

As can be seen from the data obtained activity of the conjugates **CR1(7)**, **CT1 (7)** towards CD16a receptor is 1-2 orders higher in magnitude than the activities of unmodified rituximab (**R**), and trastuzumab (**T**) (Table 3).

**Table 3.** Activity ($K_d$) of rituximab (**R**), trastuzumab (**T**) and their conjugates **CR1(7)**, **CT1(7)** towards CD16a receptor.

| | **R** | **CR1(7)** | **T** | **CT1(7)** |
|---|---|---|---|---|
| $K_d$, M | $1{,}104 \times 10^{-7}$ | $5{,}880 \times 10^{-9}$ | $2{,}341 \times 10^{-7}$ | $3{,}030 \times 10^{-8}$ |

## Industrial applicability

The invention could be used in medicine, veterinary, biochemistry.

## Claims

**1.** A compound exhibiting affinity for CD16a receptor representing 5,5,11-trioxo-10,11-dihydro-5*H*-dibenzo[*b*,*f*][1,4]thiazepine of the general formula **1** or 5,6,7,8,9,10-hexahydro-4*H*-[1]benzothieno[3,2-*f*]pyrrolo[1,2-*a*][1,4]diazepine of the general formula **2**, or pharmaceutically acceptable salt thereof,

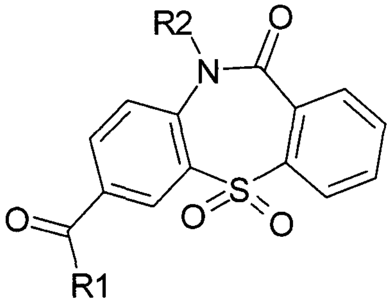

**1**

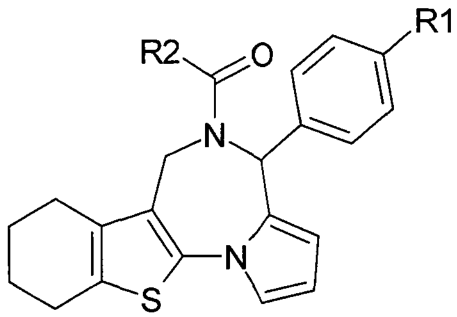

**2**

where R1 is selected from the group representing $(CH_3)_2N$-,

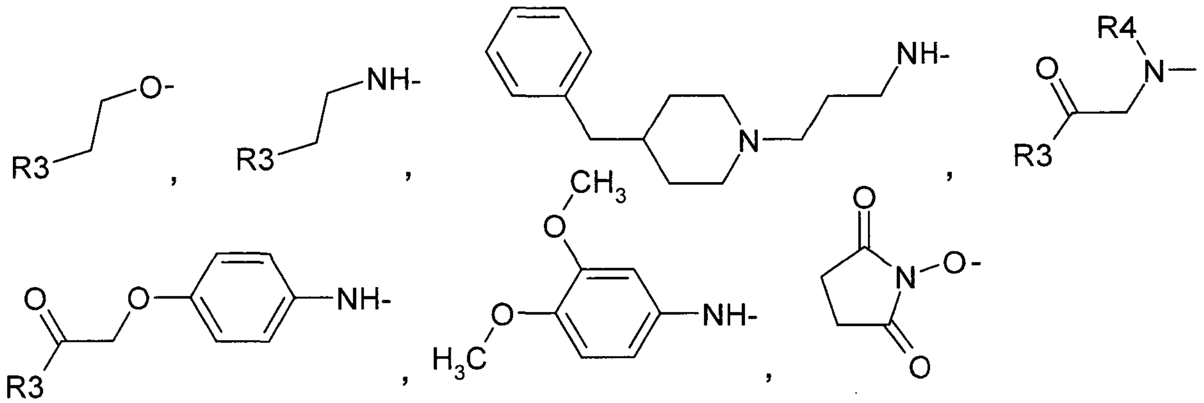

, , , , , ,

R2 is selected from the group representing

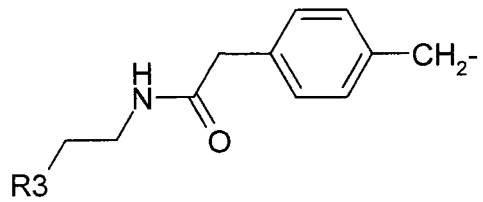
,
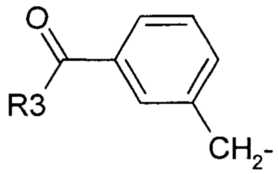
,
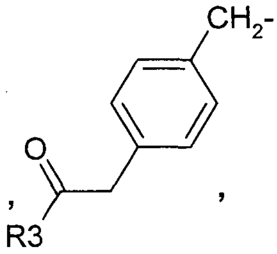
,

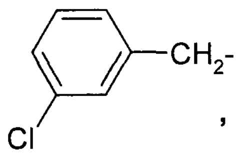
,
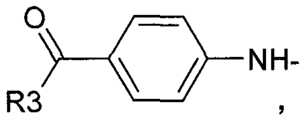
,
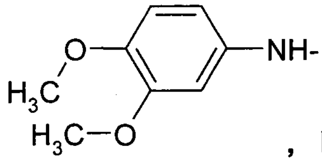
,
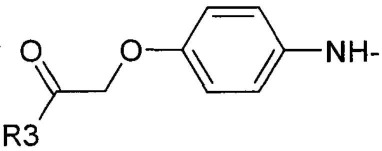

where R3, as a terminal substituent, represents $-NH_2$,

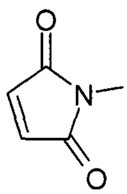
,
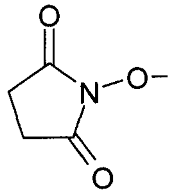
, or
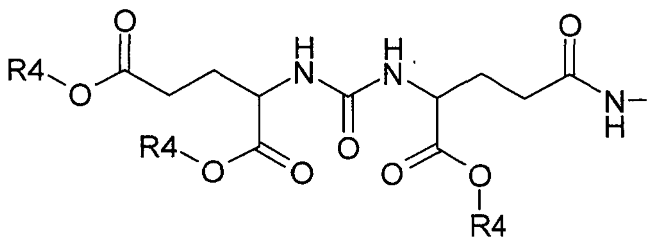
,

R4 represents H or $C_1$-$C_3$alkyl.

**2.** The compound of the general formula **1** according to claim 1, where R1 is selected from the group including:

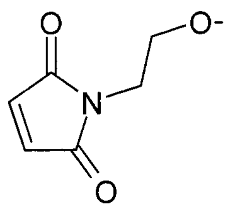
,
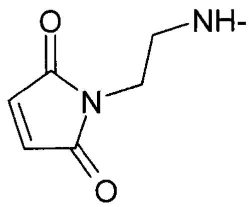
,
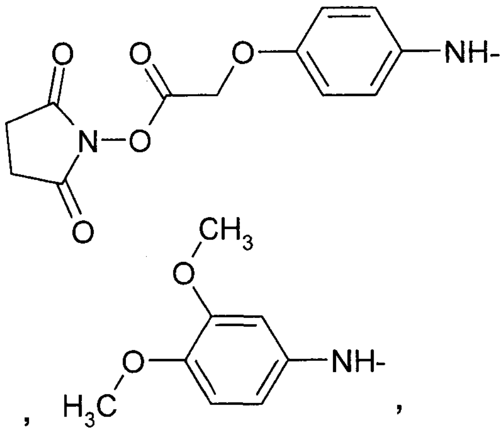
,
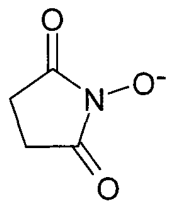
,
,

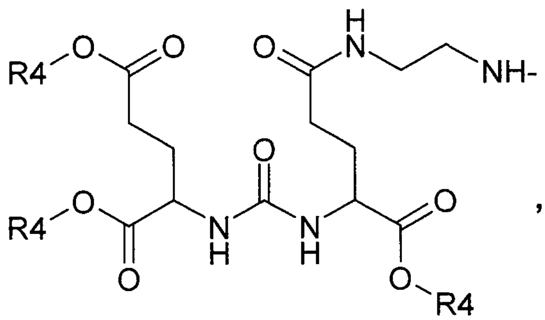

,

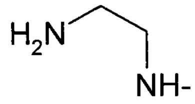

R2 is selected from the group including:

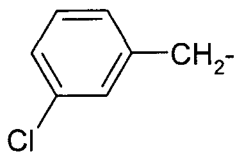

,

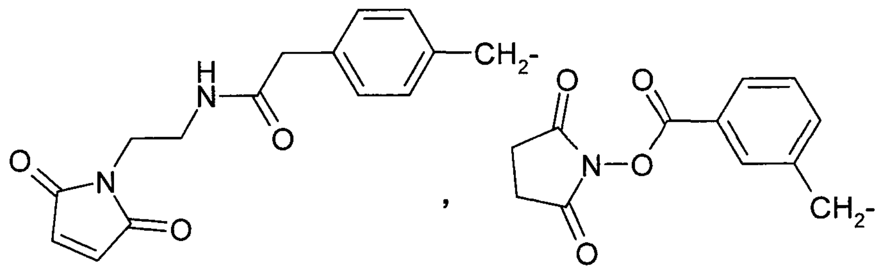

,

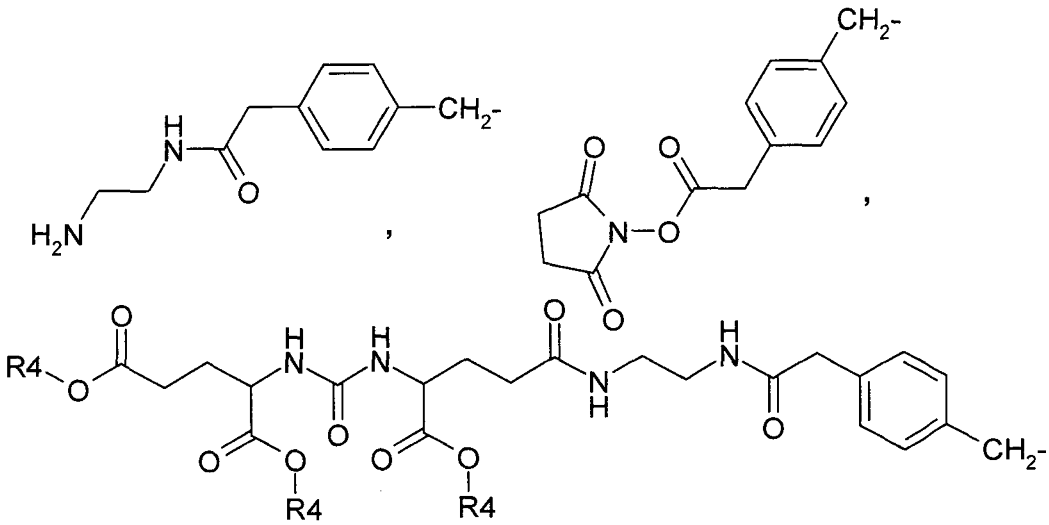

,

,

,

where R4 = H or $C_1$-$C_3$alkyl.

**3.** A compound of the general formula 2 according to claim 1, in which R1 represents $(CH_3)_2N$- or

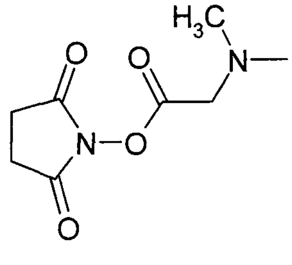

;

R2 is selected from the group including:

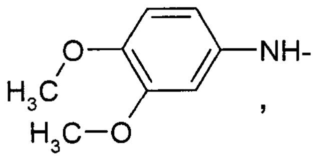 , 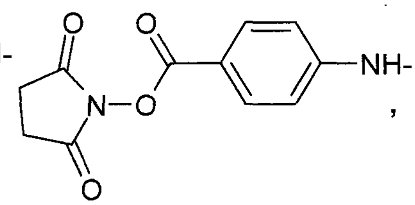 , 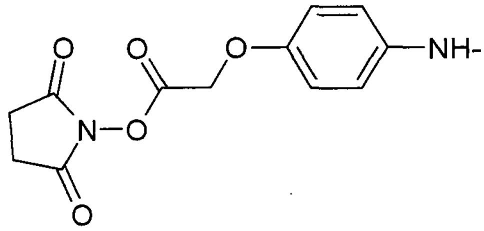

4. Compounds according to claim 1, selected from the group including the following compounds:

2,5-Dioxopyrrolidin-1-yl ester of (3-chlorobenzyl)-5,5,11-trioxo-10,11-dihydro-5*H*-dibenzo[*b*,*f*][1,4]thiazepine-7-carboxylic acid,
2,5-dioxopyrrolidin-1-yl ester of (4-{[10-(3-chlorobenzyl)-5,5,11-trioxo-10,11-dihydro-5*H*-dibenzo[*b*,*f*][1,4]thiazepine-7-carbonyl]-amino}-phenoxy)-acetic acid,
2,5-dioxopyrrolidin-1-yl ester of 4-{[10-(3-chlorobenzyl)-5,5,11-trioxo-10,11-dihydro-5*H*-dibenzo[*b*,*f*][1,4]thiazepine-7-carbonyl]-amino}-phenylcarboxylic acid,
2,5-dioxopyrrolidin-1-yl ester of 3-[8-(3,4-dimethoxyphenylcarbamoyl)-5,5,11-trioxo-5,11-dihydro-dibenzo[*b*,*f*][1,4]thiazepin-10-ylmethyl]-benzoic acid,
2,5-dioxopyrrolidin-1-yl ester of (4-{8-[3-(4-benzylpiperidin-1-yl)-propylcarbamoyl]-5,5,11-trioxo-5,11-dihydrodibenzo[*b*,*f*][1,4]thiazepin-10-ylmethyl}-phenyl)-acetic acid,
10-(3-chlorobenzyl)-5,5,11-trioxo-10,11-dihydro-5*H*-dibenzo[*b*,*f*][1,4]thiazepine-8-carboxylic acid (2-aminoethyl)-amide,
10-{4-[(2-amino-ethylcarbamoyl)-methyl]-benzyl}-5,5,11-trioxo-10,11-dihydro-5*H*-dibenzo[b,f][1,4]thiazepine-8-carboxylic acid [3-(4-benzyl-piperidin-1-yl)-propyl]-amide,
2-(2,5-dioxo-2,5-dihydro-pyrrol-1-yl)-ethyl ester of 10-(3-chlorobenzyl)-5,5,11-trioxo-10,11-dihydro-5*H*-dibenzo[b,f][1,4]thiazepine-8-carboxylic acid,
10-(4-{[2-(2,5-dioxo-2,5-dihydro-pyrrol-1-yl)-ethylcarbamoyl]-methyl}-benzyl)-5,5,11-trioxo-10,11-dihydro-5*H*-dibenzo[b,f][1,4]thiazepine-8-carboxylic acid [3-(4-benzyl-piperidin-1-yl)-propyl]-amide,
*N*-[2-({*N*-(methoxycarbonyl)-*N*-[(1,5-dimethoxy-1,5-dioxopentan-2-yl)carbamoyl]-β-alanyl}amino)ethyl]-10-(3-chlorobenzyl)-5,5,11-trioxo-10,11-dihydrodibenzo[*b*,*f*][1,4]thiazepine-7-carboxamide,
$N^5$-(2-{[(4-{[7-{[[3-(4-benzylpiperidin-1-yl)propyl](phenyl)amino]-carbonyl}-5,5,11-trioxo-dibenzo[*b*,*f*][1,4]thiazepin-10(11*H*)-yl]methyl}phenyl)acetyl]-amino}ethyl)-$N^2$-{[(1,3-dicarboxypropyl)amino]carbonyl}glutamine,
4-[4-(dimethylamino)phenyl]-*N*-(4-{[(2,5-dioxopyrrolidin-1-yloxy]carbonyl}phenyl)-7,8,9,10-tetrahydro-4*H*-[1]benzothieno[3,2-*f*]pyrrolo[1,2-*a*][1,4]diazepine-5(6*H*)-carboxamide,
4-[4-(dimethylamino)phenyl]-*N*-(4-{2-[(2,5-dioxopyrrolidin-1-yl)oxy]-2-oxoethoxy}phenyl)-7,8,9,10-tetrahydro-4*H*-[1]benzothieno[3,2-*f*]pyrrolo[1,2-*a*][1,4]diazepine-5(6*H*)-carboxamide,
2,5-dioxopyrrolidin-1-yl *N*-[4-(5-{[(3,4-dimethoxyphenyl)amino]-carbonyl}-5,6,7,8,9,10-hexahydro-4*H*-[1]benzothieno[3,2-*f*]pyrrolo[1,2-*a*][1,4]diazepin-4-yl)phenyl]-*N*-methylglycinate.

5. A modified protein, active towards CD16a receptor, selected from an antibody or autoantigene, conjugated by a modifying compound exhibiting affinity for CD16a receptor and selected from compound of the general formula 1 or 2 according to claim 1.

6. The modified protein according to claim 5, **characterized in that** antibody represents rituximab.

7. The modified protein according to claim 5, **characterized in that** antibody represents trastuzumab.

8. The modified protein according to claim 5, **characterized in that** antibody represents cetuximab.

9. The modified protein according to claim 5, **characterized in that** autoantigene represents interferon alfa.

10. The modified protein according to claim 5, **characterized in that** autoantigene represents myelin basic protein.

11. The modified protein according to claim 5, **characterized in that** autoantigene represents complement C1q protein.

12. A method for preparation of modified protein as claimed in claim 5, according to which a protein is subjected to

interaction with a compound of the general formula 1 or 2, as claimed in claim1, dissolved in organic solvent, for example, dimethyl sulfoxide at molar ratio in interval from 1:3 to 1:100 in PBS solution (pH 7.4).

**13.** A pharmaceutical composition active towards CD16a receptor in the form of tablets, capsules or injections placed in a pharmaceutically acceptable packing and comprising a modified protein according to claim 5 in therapeutically acceptable amount and pharmaceutically acceptable diluent, carrier or excipient.

**14.** A medicament active towards CD16a receptor in the form of tablets, capsules or injections placed in a pharmaceutically acceptable packing and intended for treating a disease caused by pathologic cells, comprising a modified protein according to claim 5 or pharmaceutical composition according to claim 13 in therapeutically effective amount.

**15.** A method for treatment of disease caused by pathologic cells which could be cured by indirect action on CD16a receptor, according to which therapeutically effective amount of modified protein as claimed in claim 5, or pharmaceutical composition as claimed in claim 13, or medicament as claimed in claim 14 is introduced to a subject.

**16.** The method according to claim 15 of treating autoimmune or oncology disease.

**17.** The method according to claim 16 of treating lymphoma, lymphoid leukemia or breast cancer.

**18.** The method according to claim 16 of treating autoimmune polyendocrinopathy of the first type.

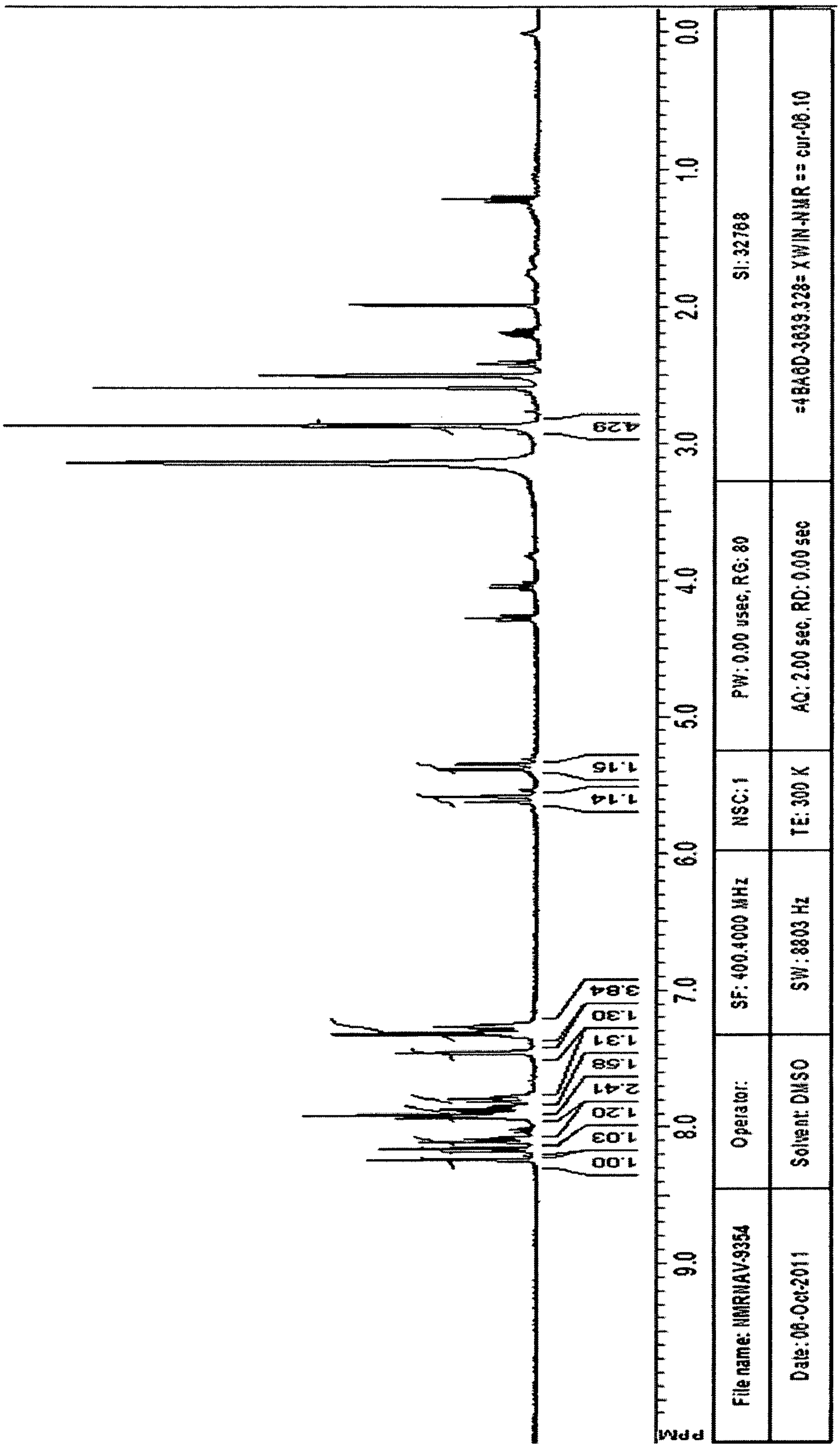
0.0
1.0
2.0
3.0
4.0
5.0
6.0
7.0
8.0
9.0
PPM
4.29
1.16
1.14
3.84
1.30
1.31
1.58
2.41
1.20
1.03
1.00
File name: NMRNAV-9354
Date: 08-Oct-2011
Operator:
Solvent: DMSO
SF: 400.4000 MHz
SW: 8803 Hz
NSC: 1
TE: 300 K
PW: 0.00 usec, RG: 80
AQ: 2.00 sec, RD: 0.00 sec
SI: 32768
=4BA8D-3639.328= XWIN-NMR == cur-08.10

Fig. 1.

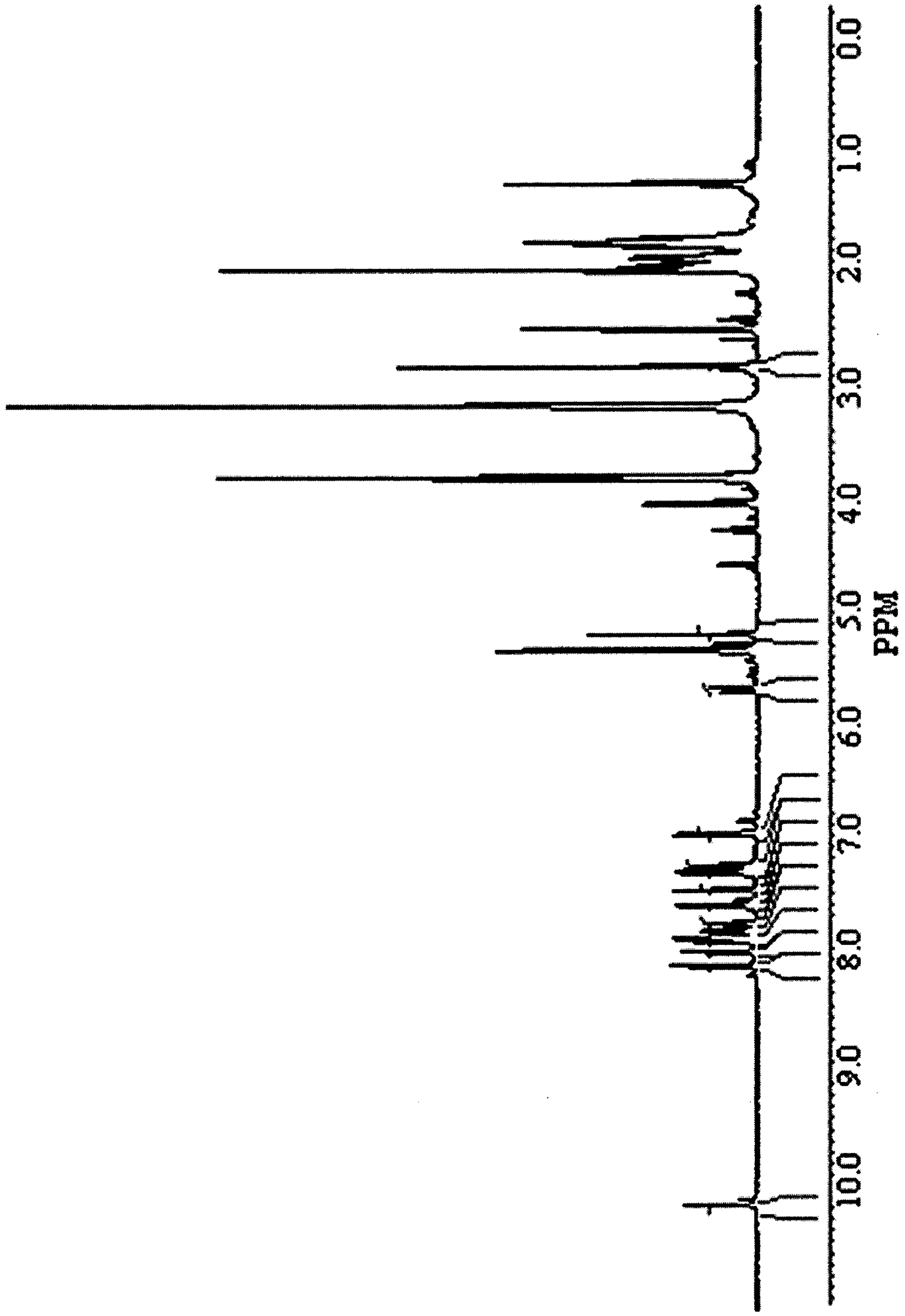


Fig. 2.

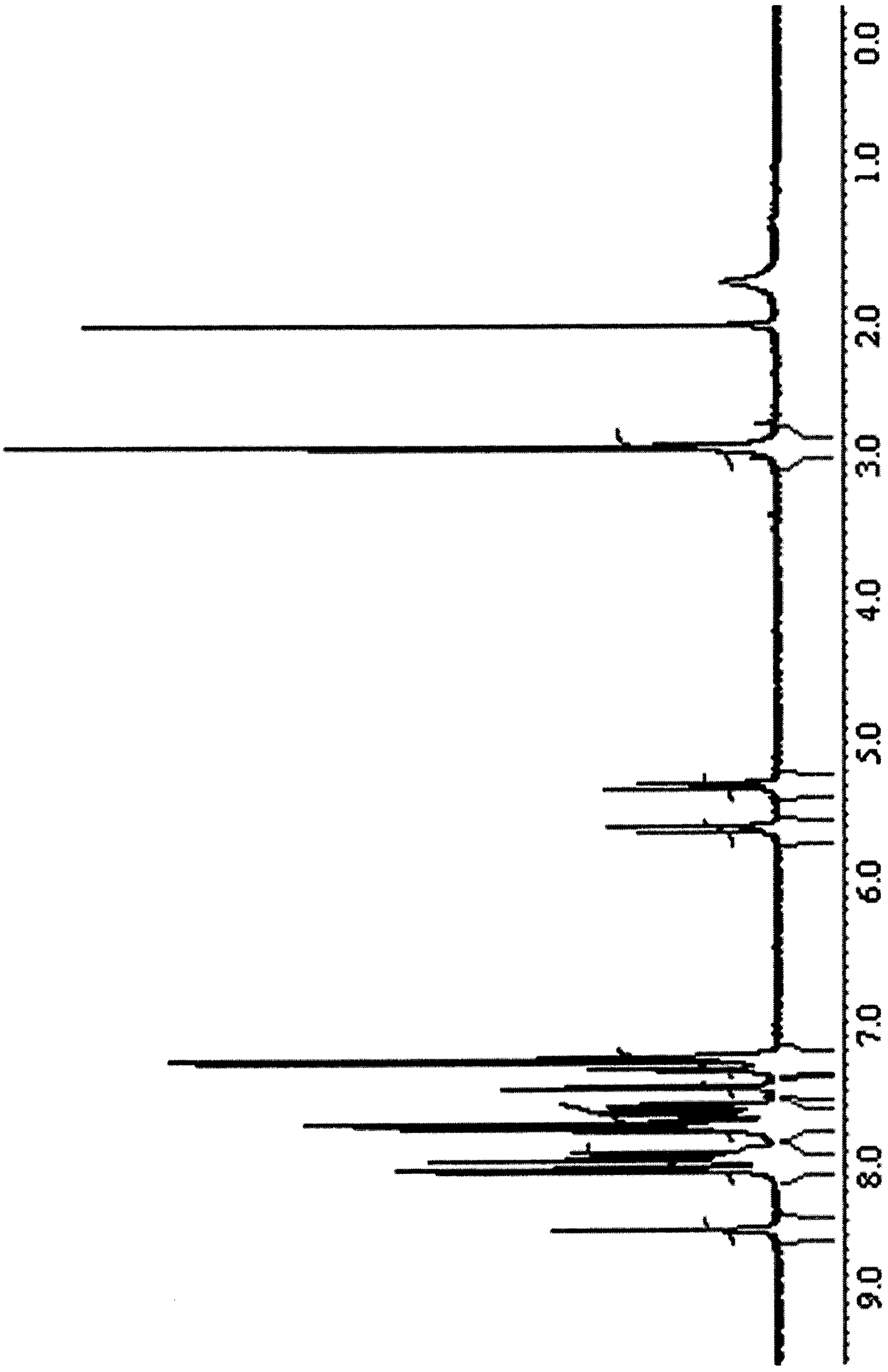


Fig. 3.

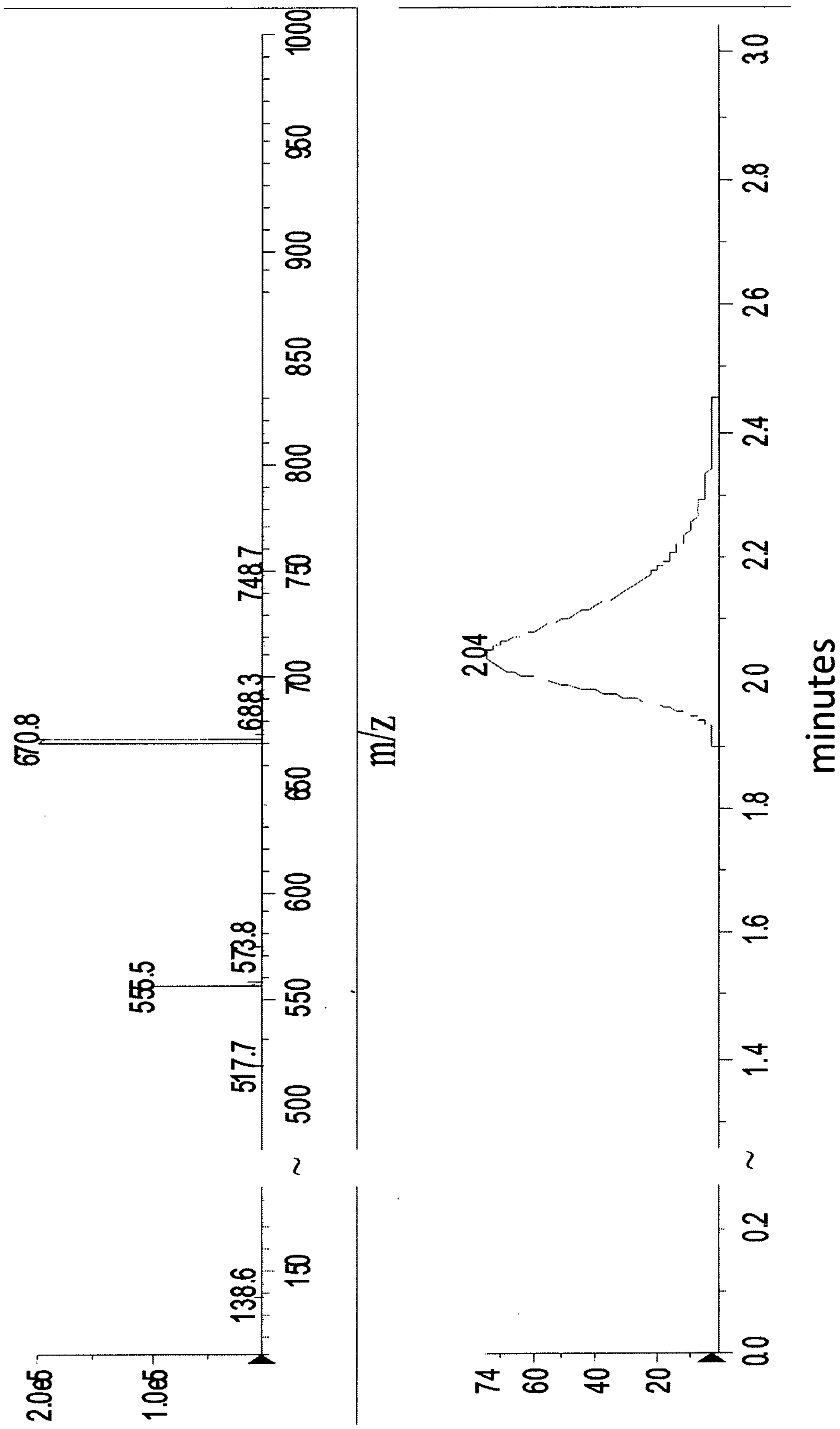
670.8
688.3
555.5
573.8
517.7
748.7
138.6
m/z
2.04
minutes
2.0e5
1.0e5
74
60
40
20

Fig. 4.

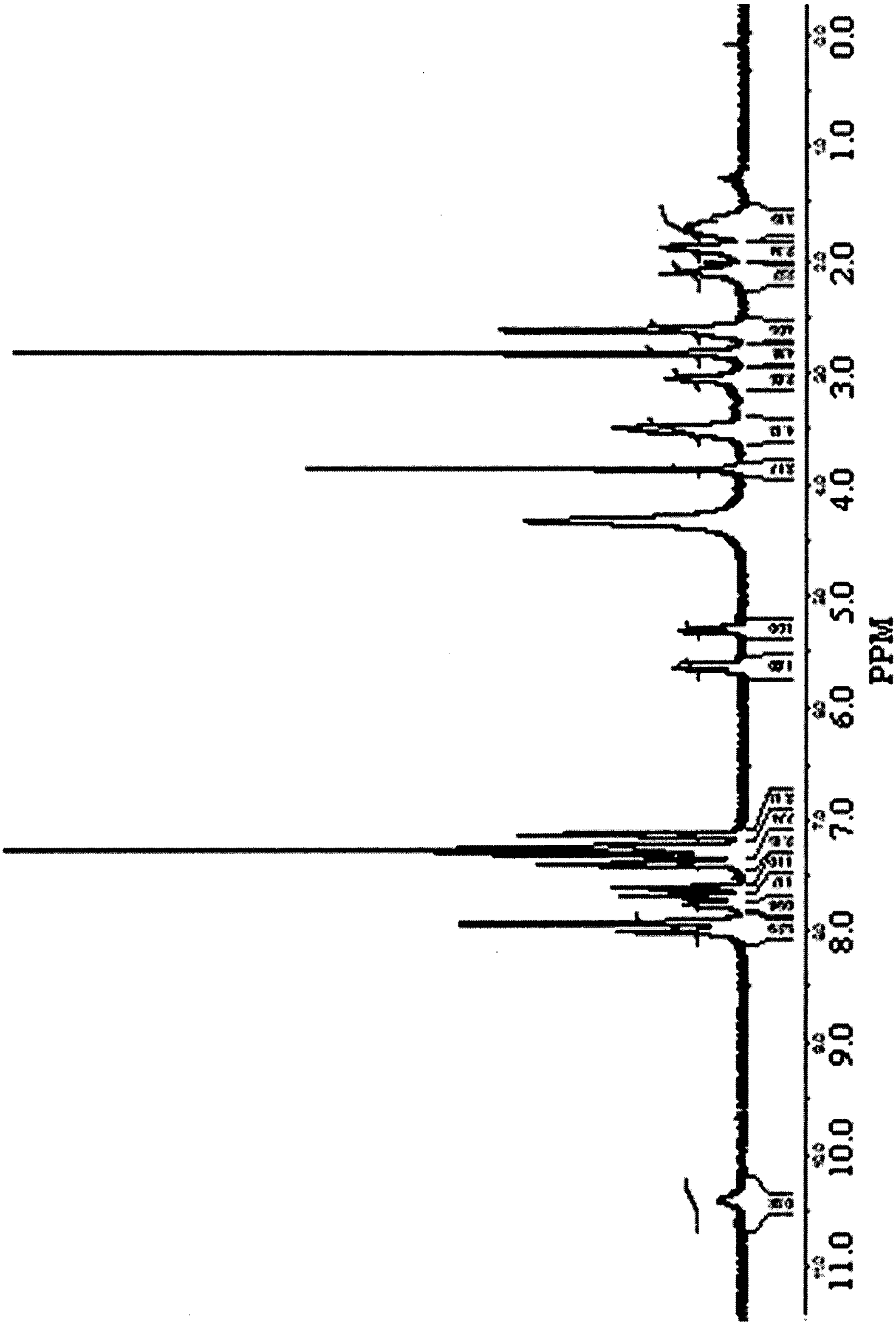


Fig. 5.

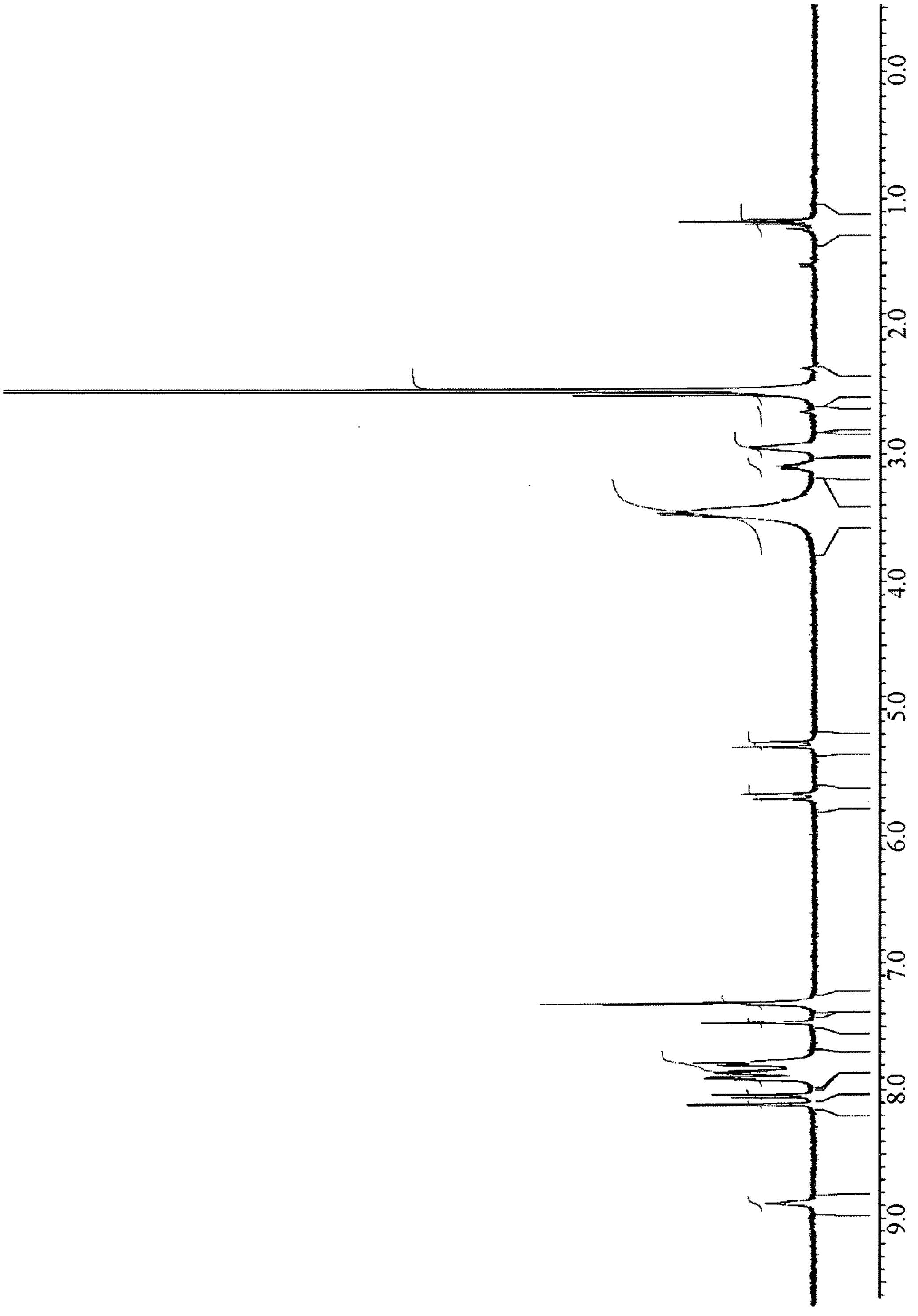


Fig. 6.

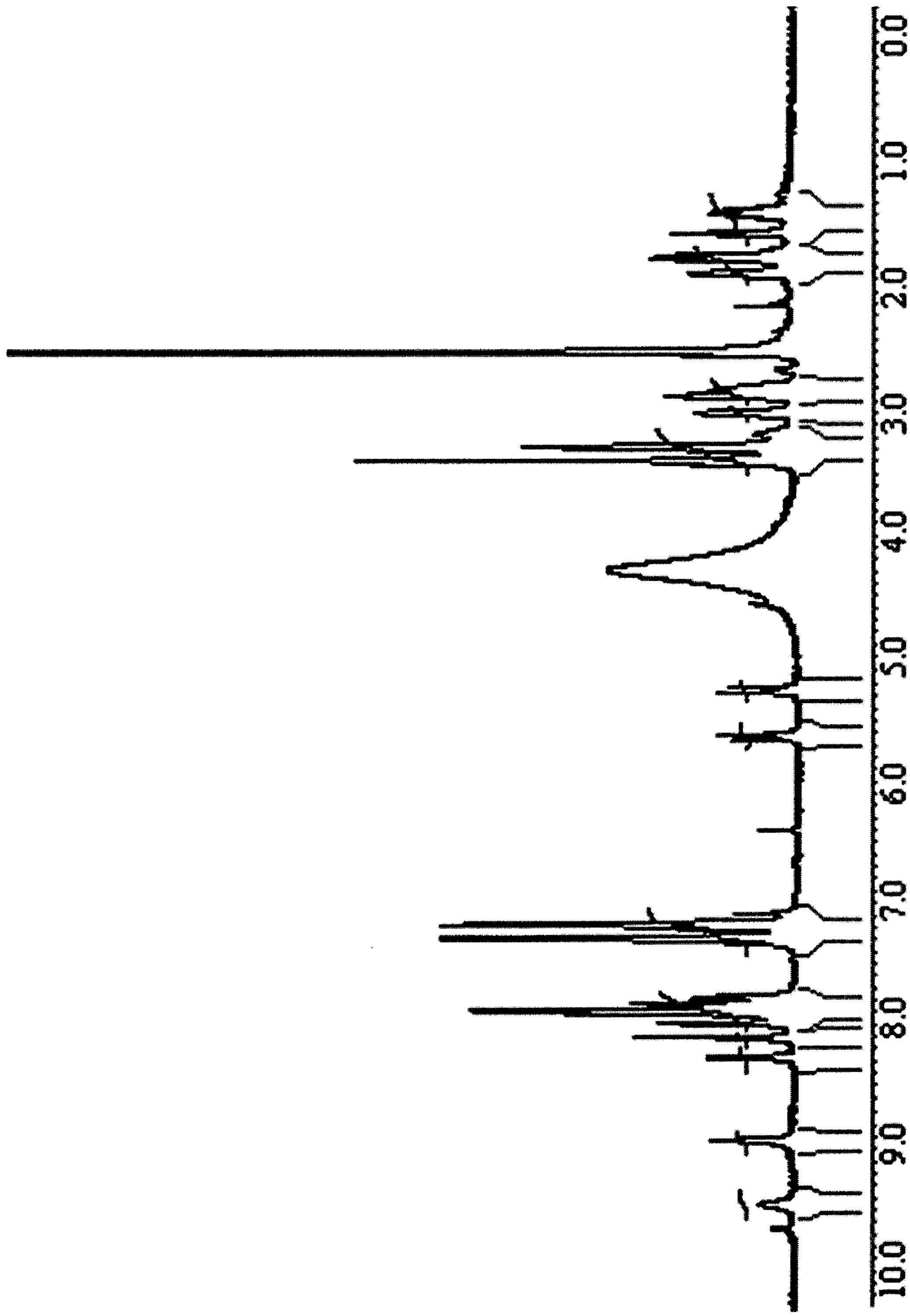


Fig. 7.

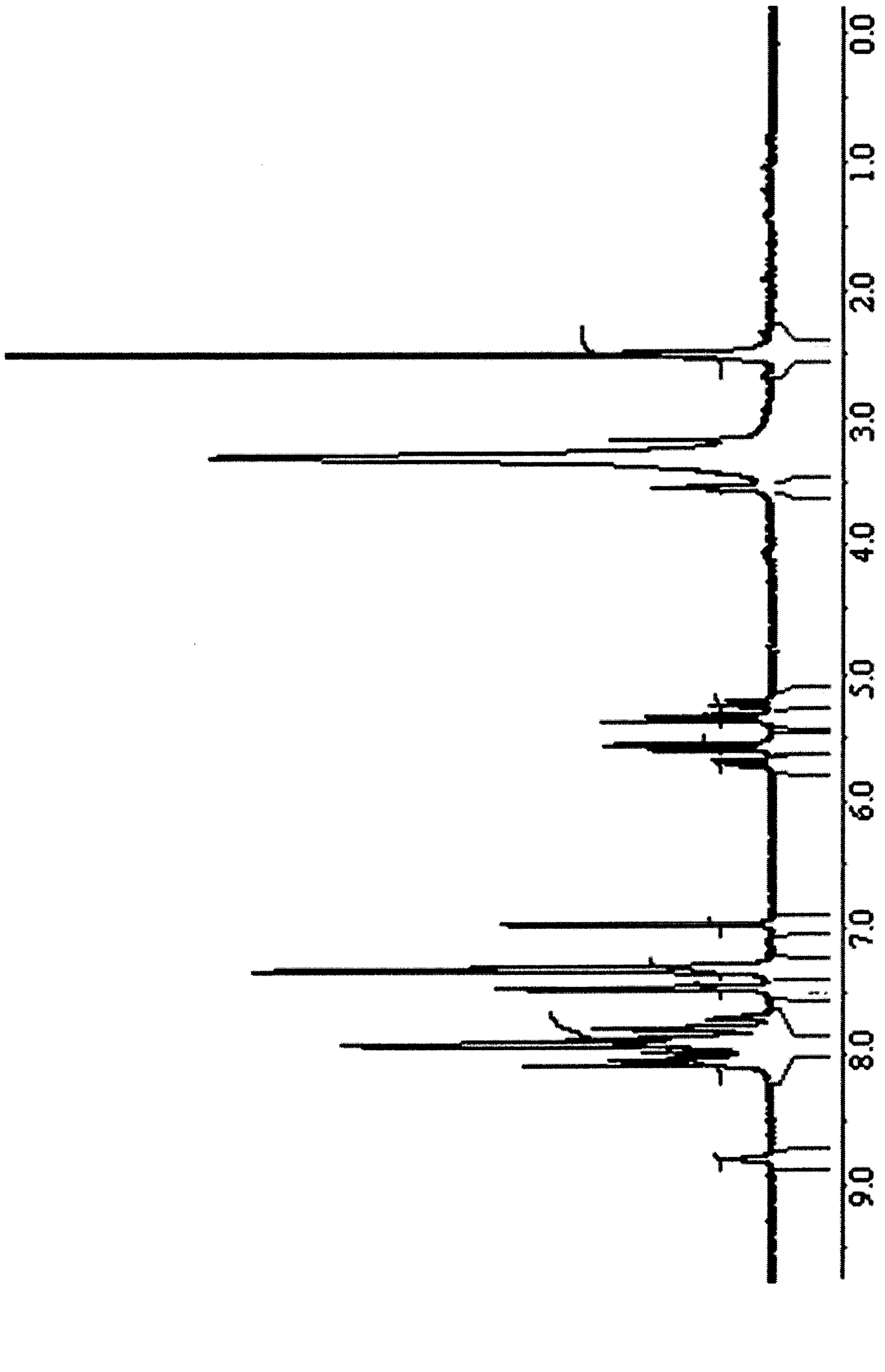


Fig. 8.

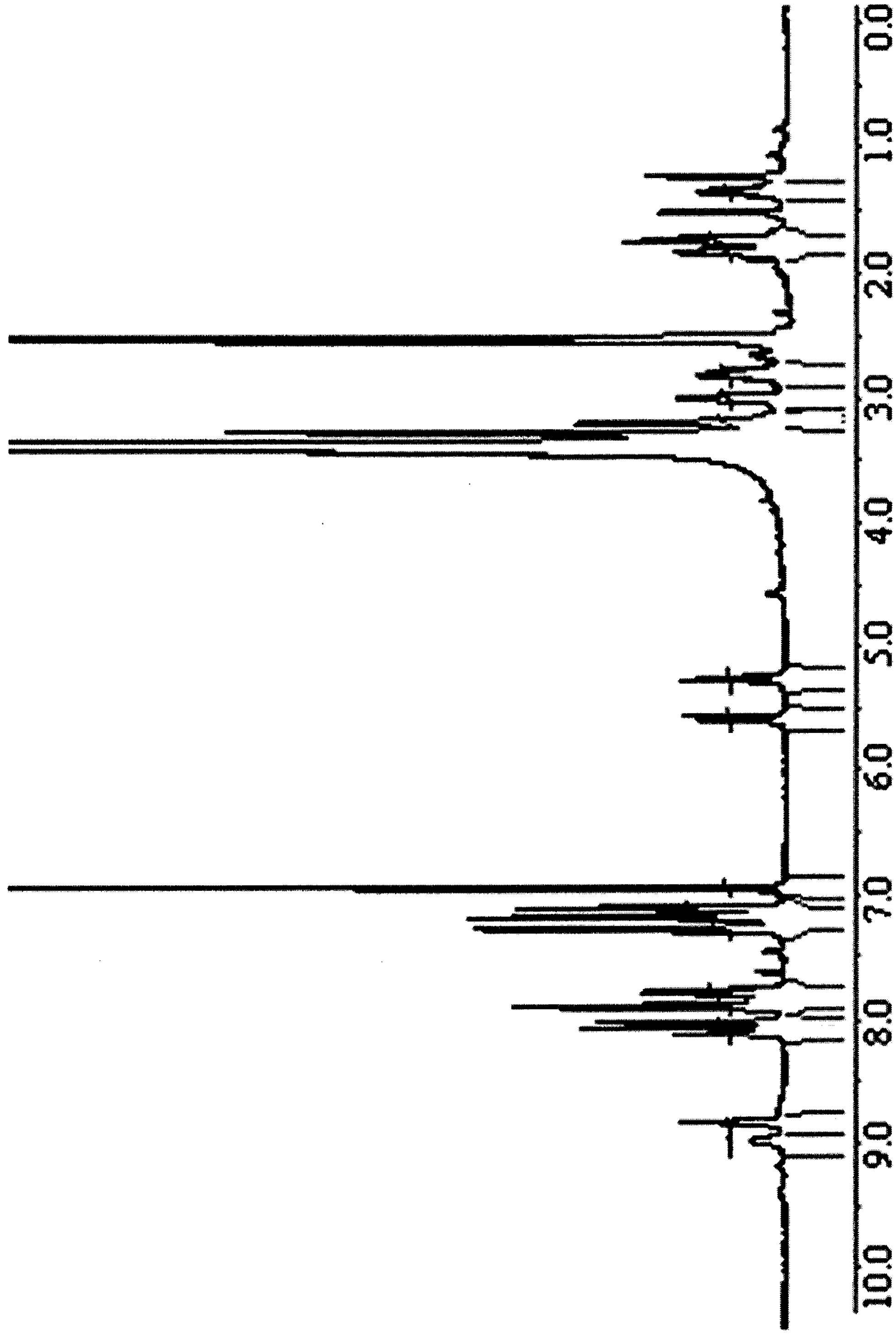


Fig. 9.

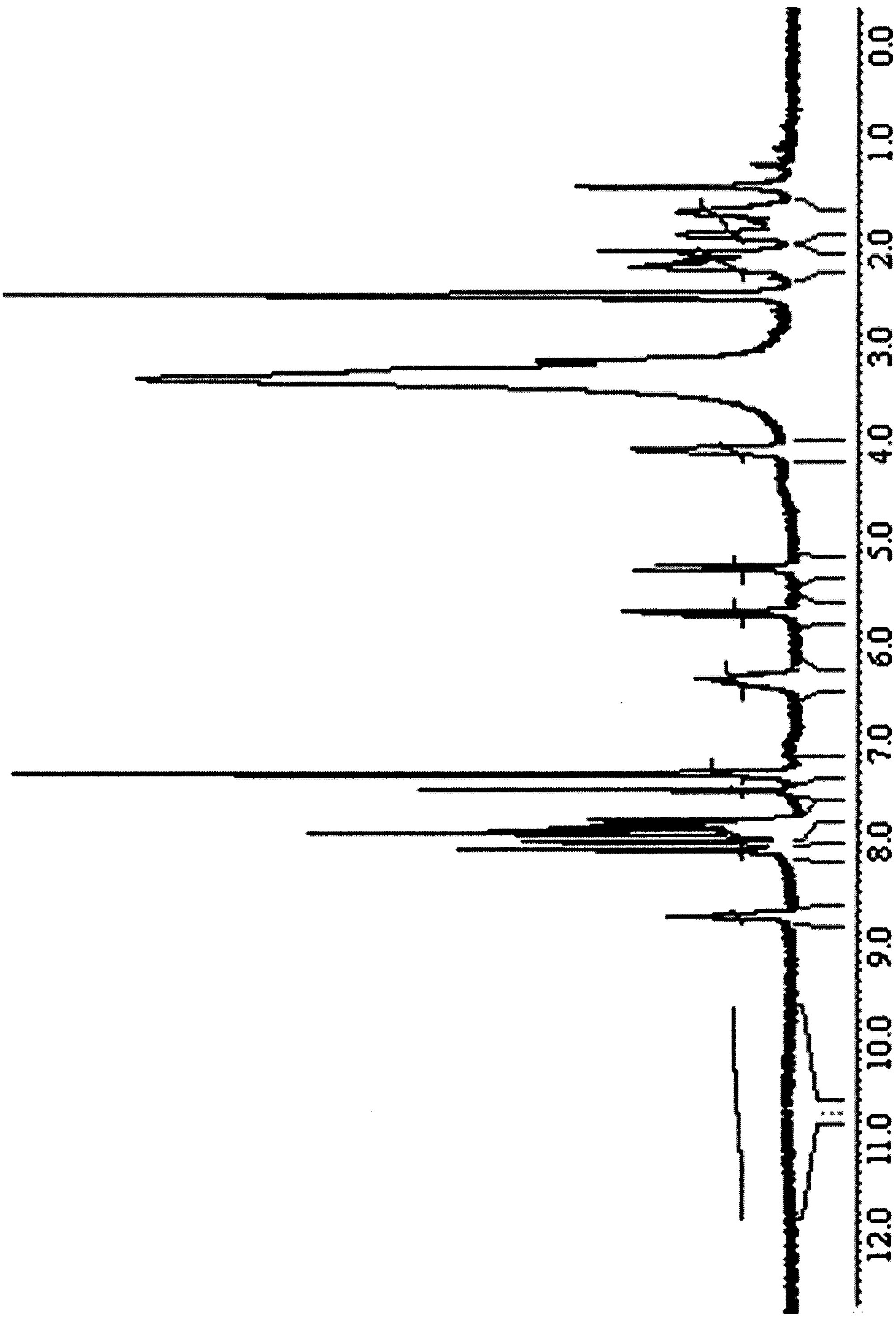


Fig. 10.

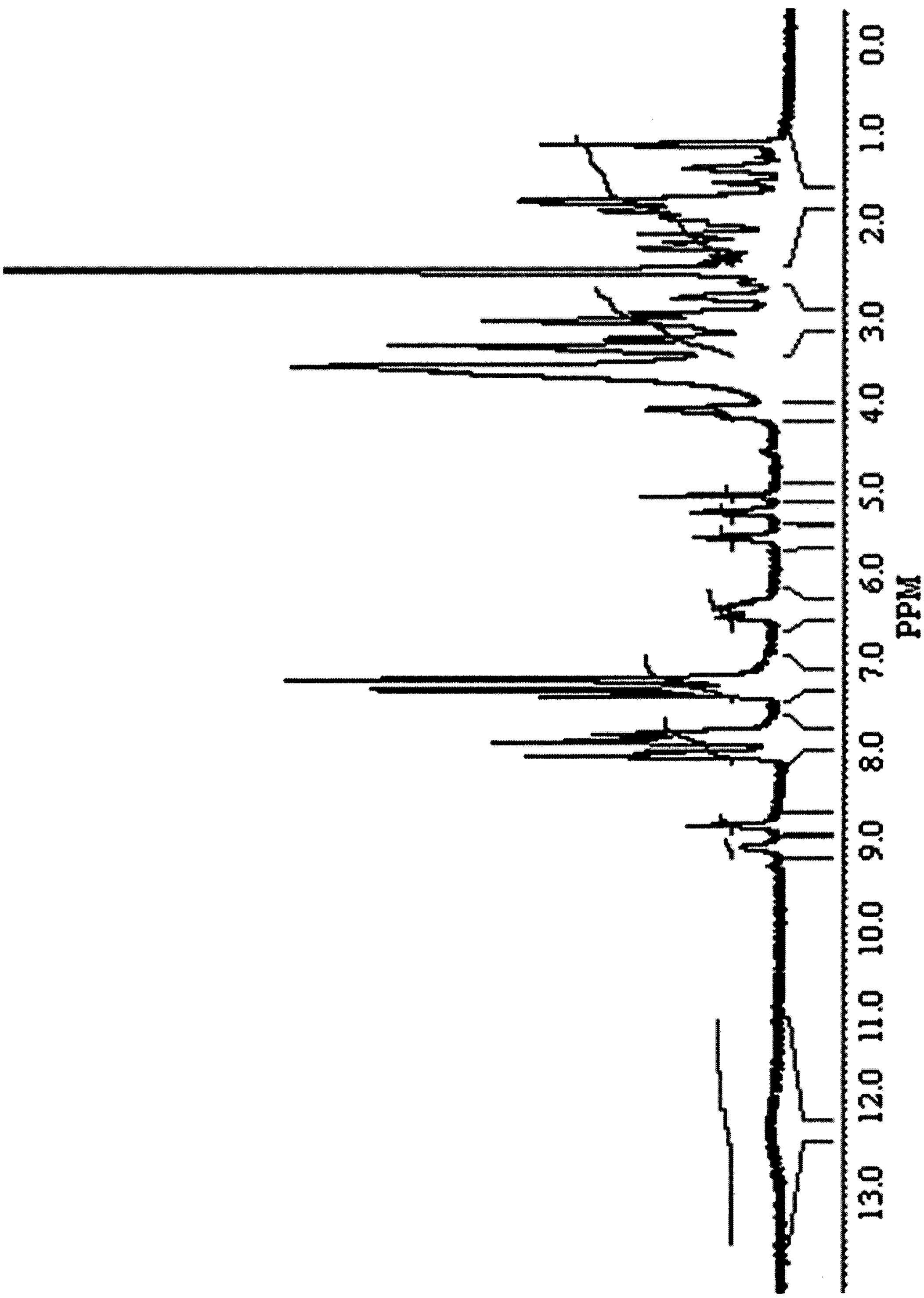


Fig. 11.

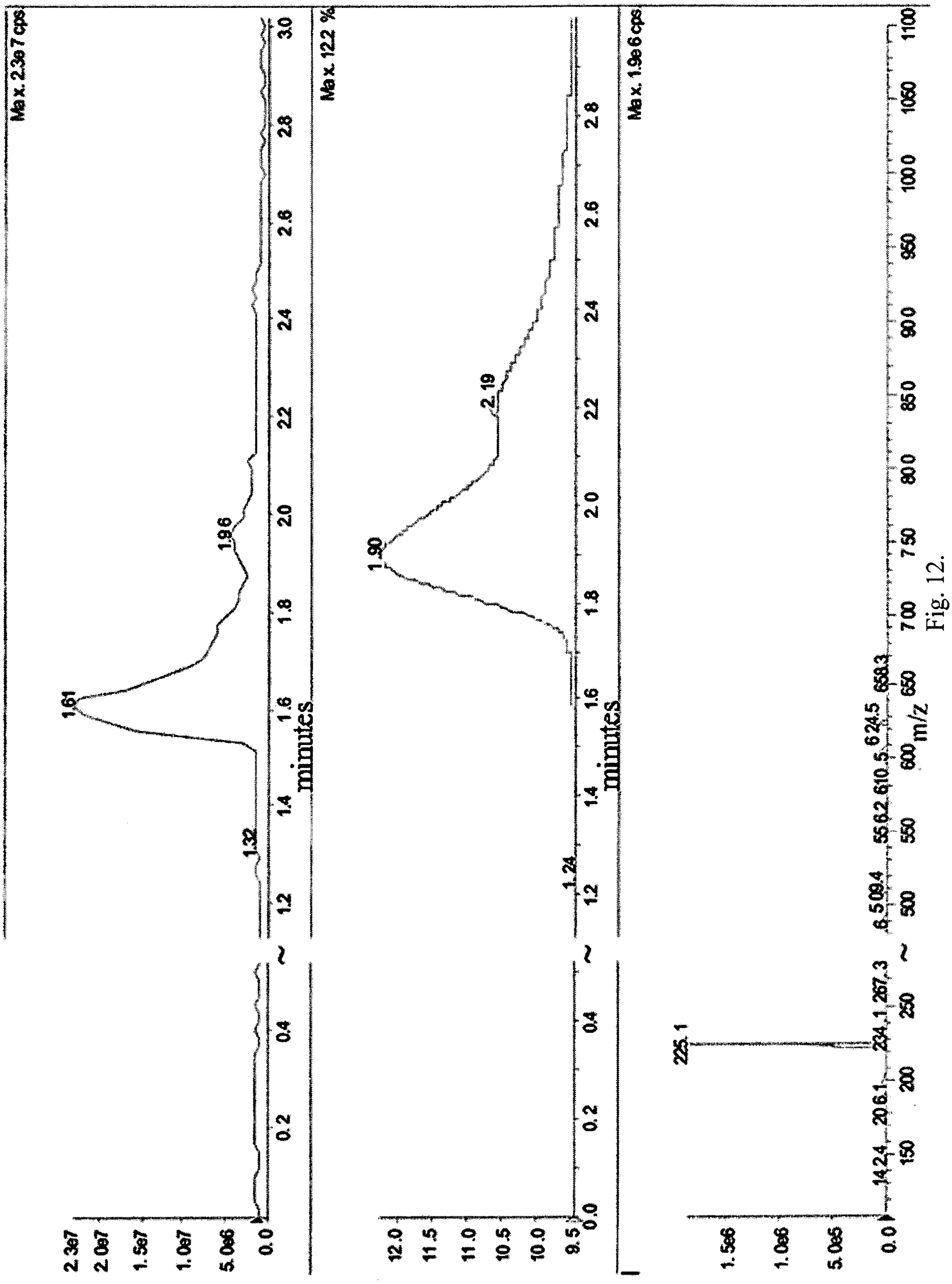
Max. 2.3e7 cps
1.61
1.96
1.32
minutes
Max. 12.2 %
1.90
2.19
1.24
minutes
Max. 1.9e6 cps
225.1
m/z


Fig. 12.

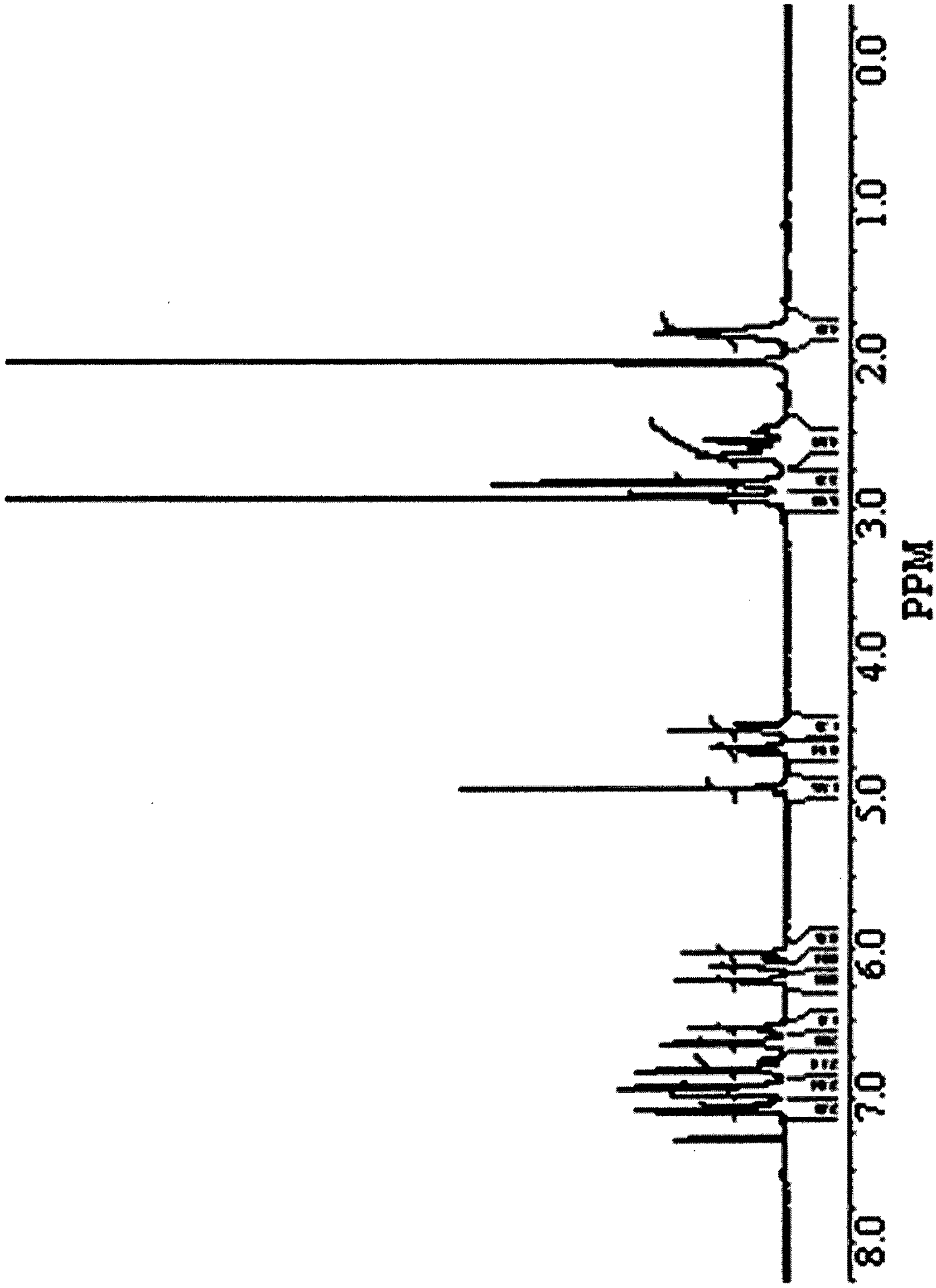


Fig. 13.

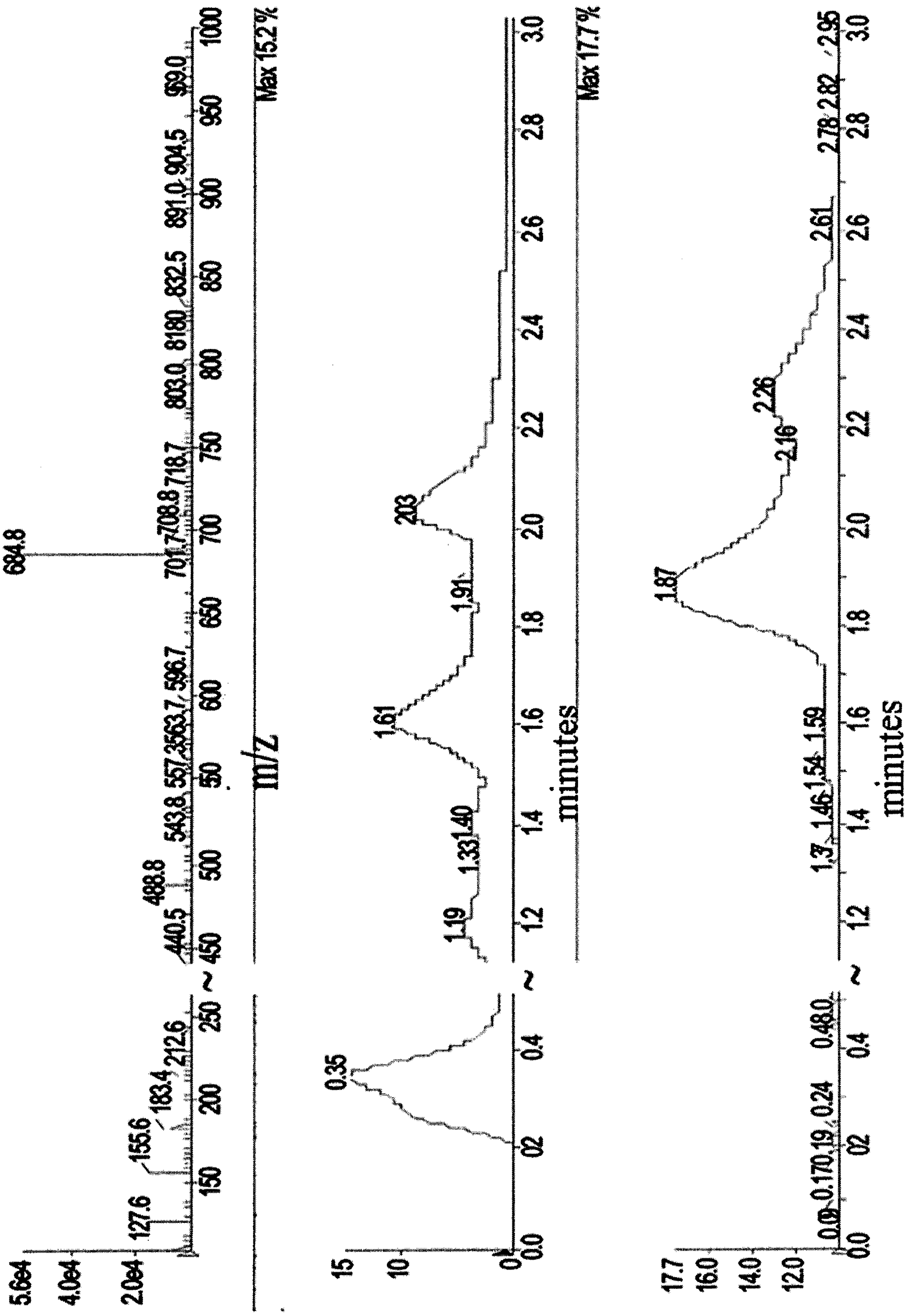


Fig. 14.

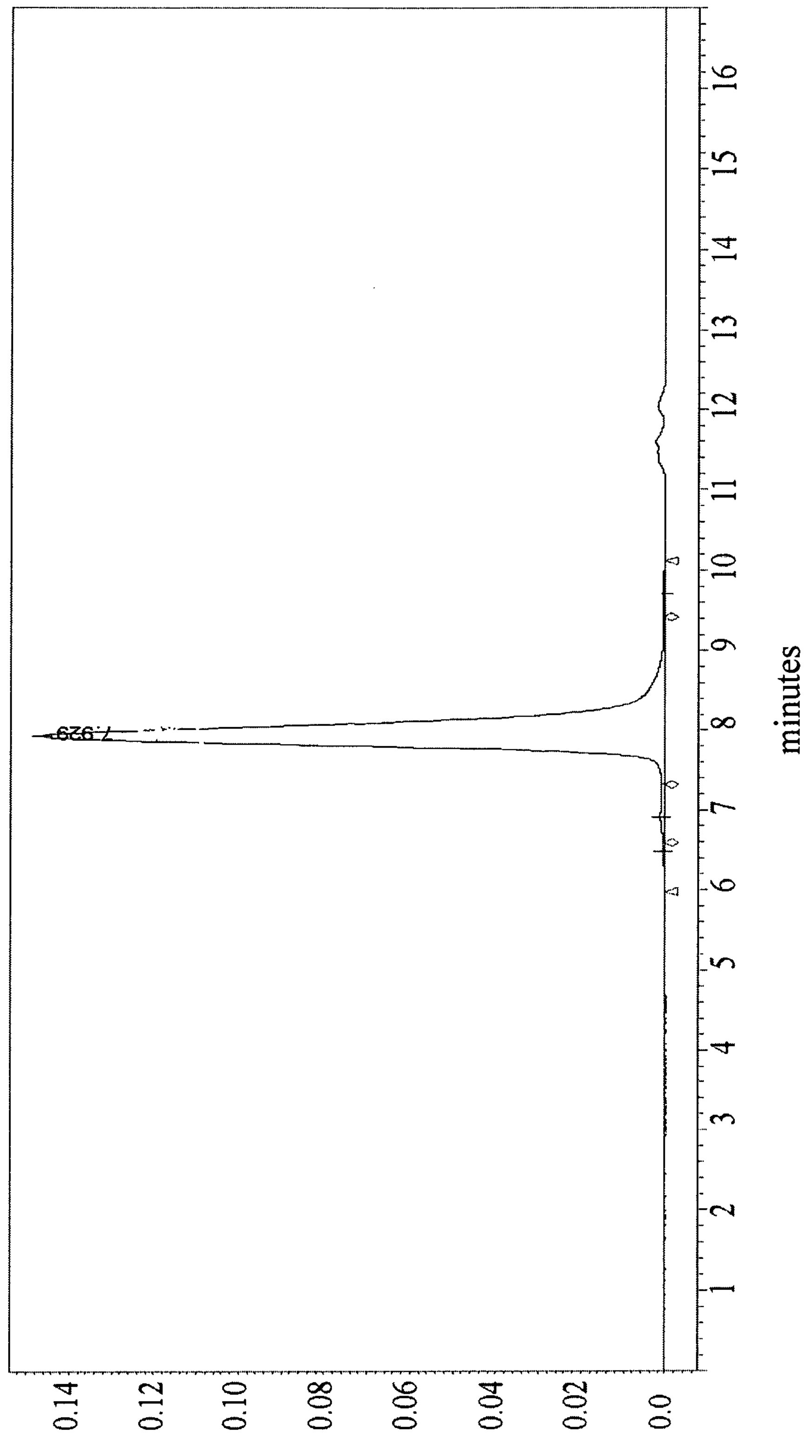
7.929
0.14
0.12
0.10
0.08
0.06
0.04
0.02
0.0
1
2
3
4
5
6
7
8
9
10
11
12
13
14
15
16
minutes

Fig. 15.

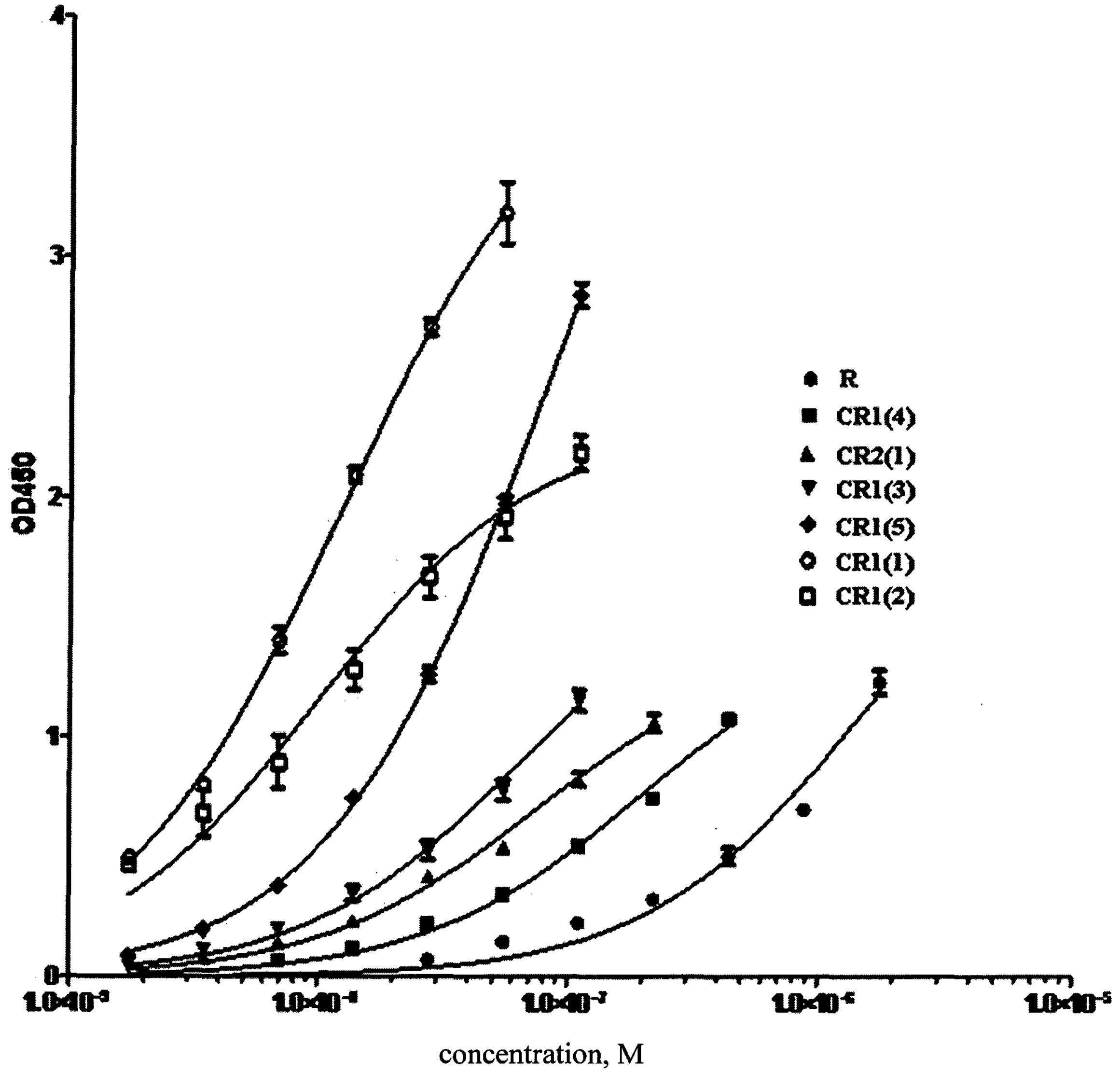
OD450
4
3
2
1
0
R
CR1(4)
CR2(1)
CR1(3)
CR1(5)
CR1(1)
CR1(2)
1.0×10-9
1.0×10-8
1.0×10-7
1.0×10-6
1.0×10-5
concentration, M

Fig. 16.

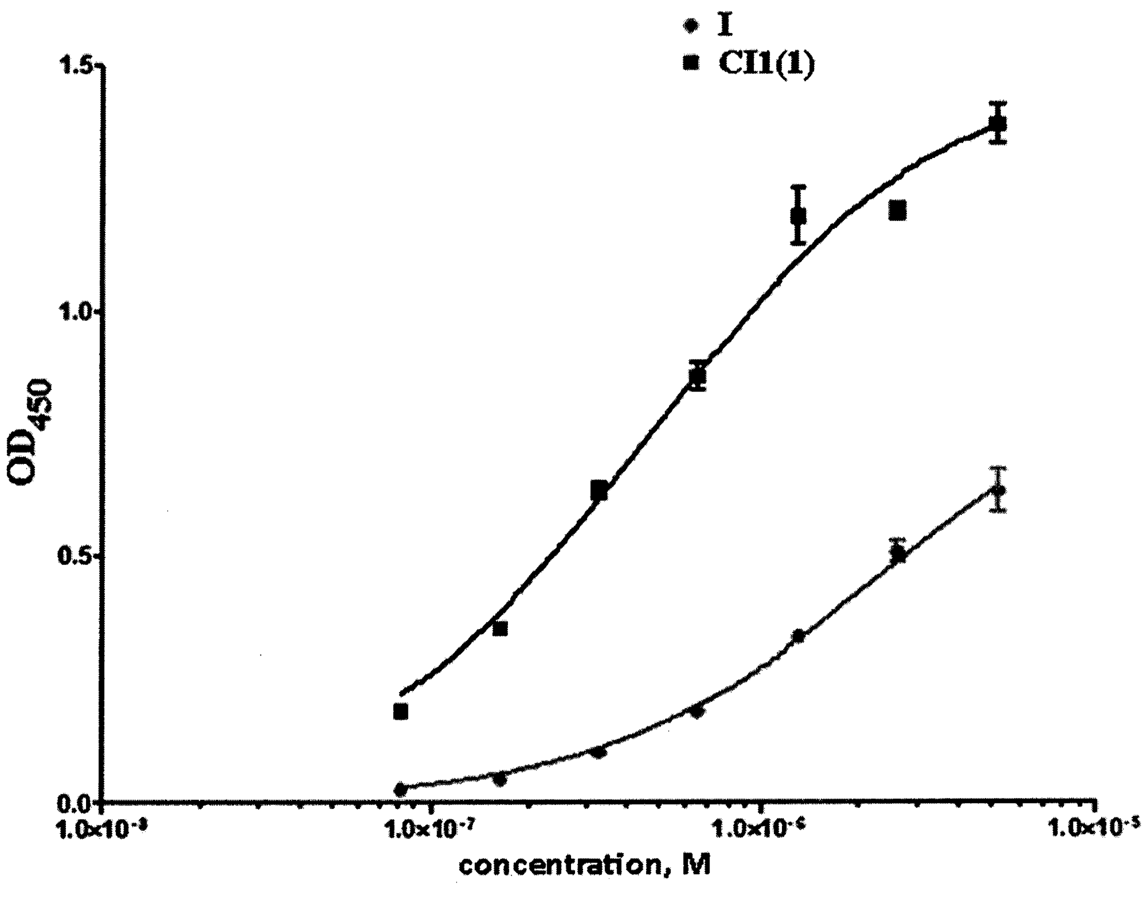
I
CI1(1)
1.5
1.0
0.5
0.0
OD450
1.0×10-8
1.0×10-7
1.0×10-6
1.0×10-5
concentration, M

Fig. 17.

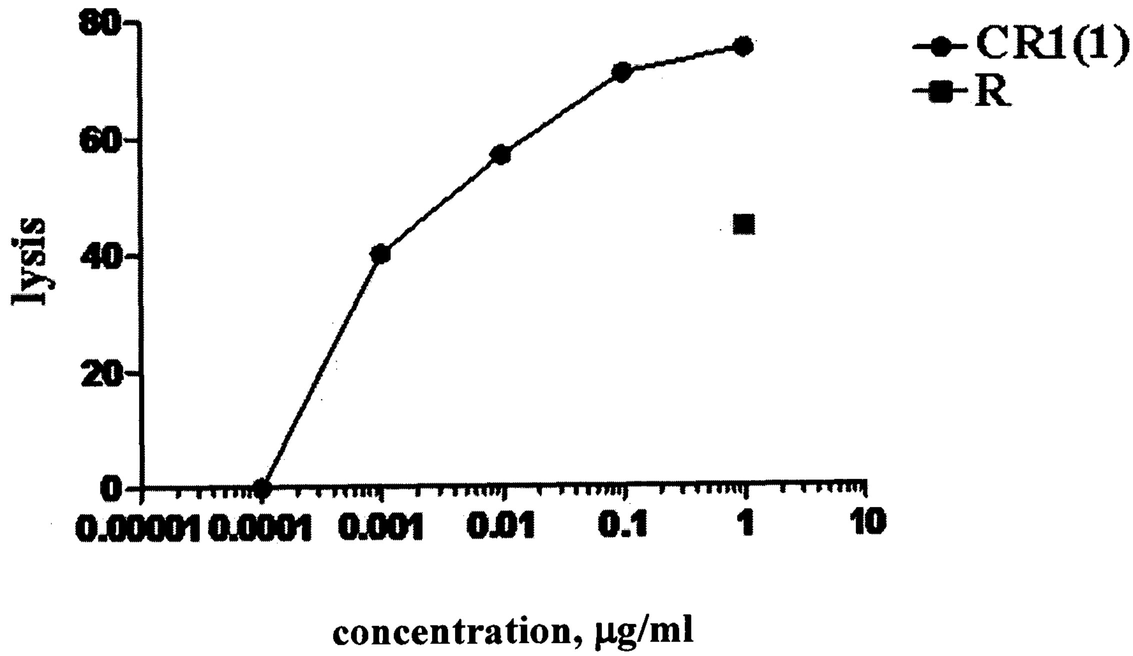
80
60
40
20
0
lysis
0.00001
0.0001
0.001
0.01
0.1
1
10
concentration, µg/ml
CR1(1)
R

Fig. 18.

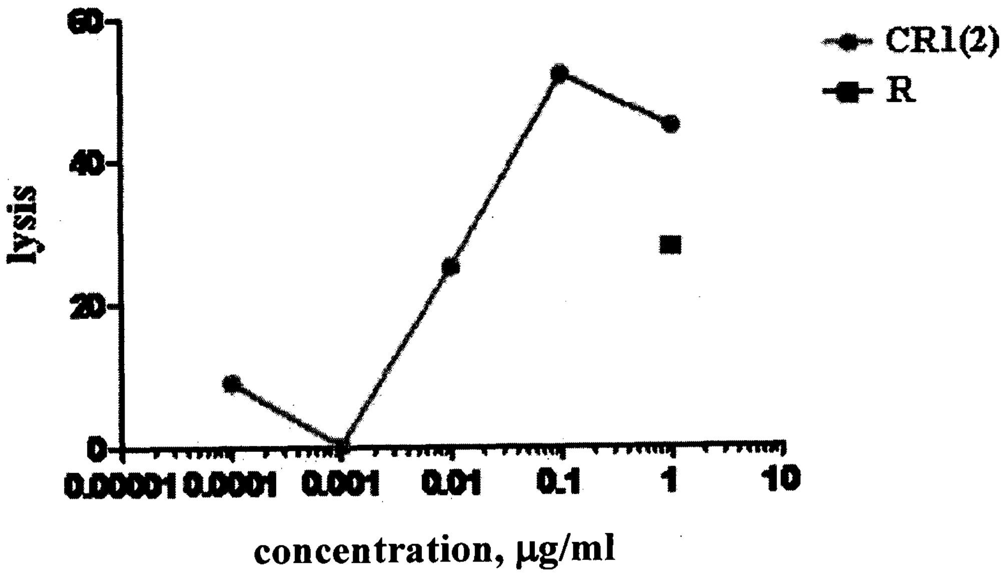
60
40
20
0
lysis
0.00001
0.0001
0.001
0.01
0.1
1
10
concentration, µg/ml
CR1(2)
R

Fig.19.

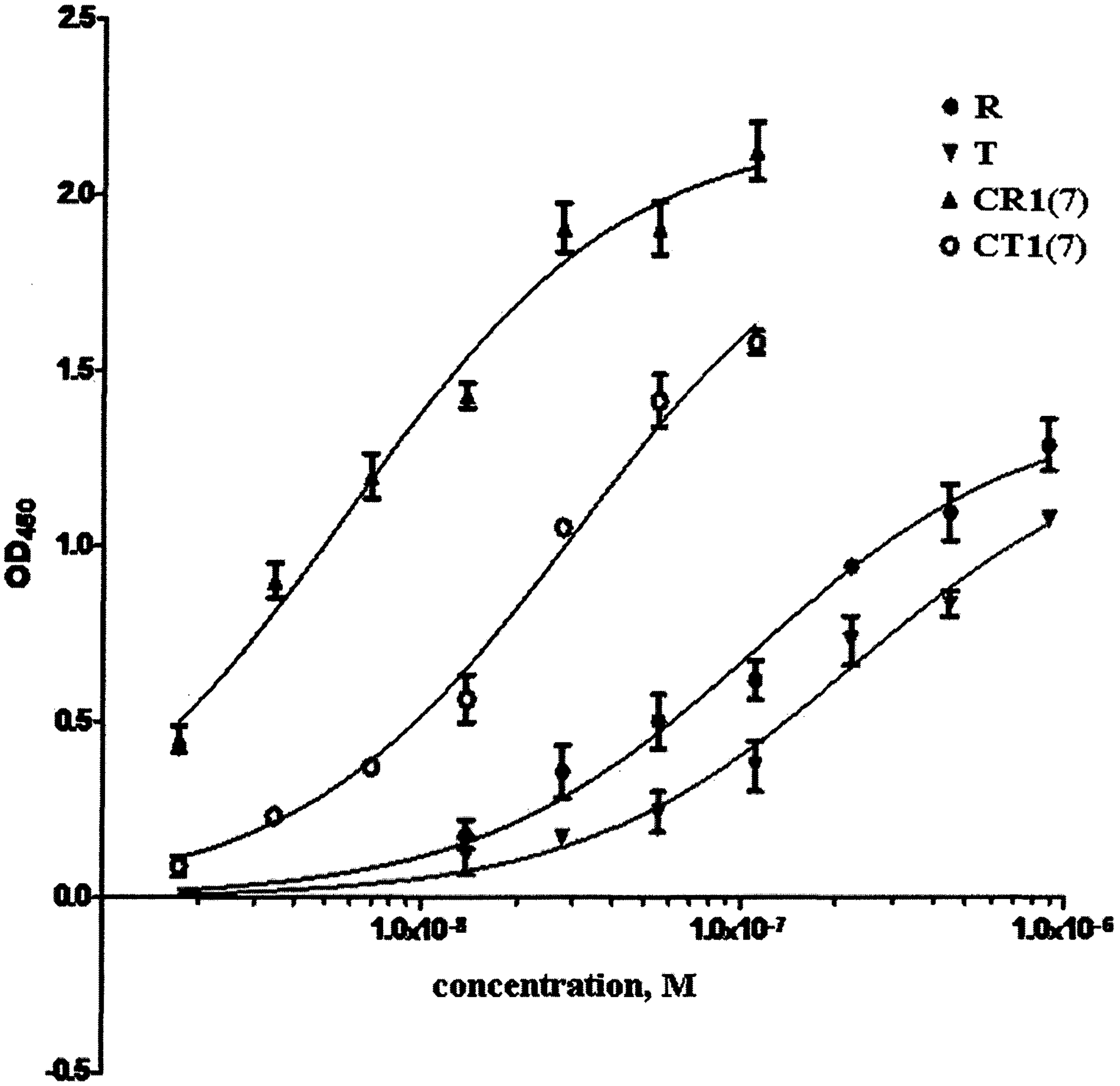

R
T
CR1(7)
CT1(7)
2.5
2.0
1.5
1.0
0.5
0.0
-0.5
OD450
1.0x10-8
1.0x10-7
1.0x10-6
concentration, M


Fig. 20.

**INTERNATIONAL SEARCH REPORT**

International application No.
PCT/RU 2014/000015

A. CLASSIFICATION OF SUBJECT MATTER
See supplemental sheet

According to International Patent Classification (IPC) or to both national classification and IPC

B. FIELDS SEARCHED

Minimum documentation searched (classification system followed by classification symbols)
C07D 281/16, 495/14, C07K 14/555, C12P 21/08, A61K 31/554, 31/5517, 38/02, A61P 35/00, 37/00

Documentation searched other than minimum documentation to the extent that such documents are included in the fields searched

Electronic data base consulted during the international search (name of data base and, where practicable, search terms used)
STN, PAJ, Espacenet, DWPI, Patentscope, USPTO DB, CIPO (Canada PO), SIPO DB, PatSearch (RUPTO internal)

C. DOCUMENTS CONSIDERED TO BE RELEVANT

| Category* | Citation of document, with indication, where appropriate, of the relevant passages | Relevant to claim No. |
|---|---|---|
| Y | EA 013015 B1 (MENARINI INTERNESHNL OPEREISHNZ LJUKSEMBURG S.A.) 26.02.2010, the abstract, p. 1-3, examples 2-11, the claims | 1-5,8-11,13-18 |
| Y | WO 2007/047737 A1 (ACADIA PHARMACEUTICALS INC. et al.) 26.04.2007, the claims, par. [0002] -[0004], [0017], [0026]- [0031], example 114, par. [0317], example 118, par. [0325], example 120, par. [0330], scheme 5, par. [0128]-[0129] | 1-5,8-11,13-18 |
| A | [online] REGISTRY via STN, 01.07.2008, RN 1031967-34-6 | 1-4 |
| A | [online] REGISTRY via STN, 29.07.2008, RN 1031571-30-1 | 1-4 |
| Y | RU 2281947 C1 (OBSCHESTVO S OGRANICHENNOI OTVETSTVENNOSTJU «ISSLEDOVATELSKY INSTITUT KHIMICHESKOGO RAZNOOBRAZIYA») 20.08.2006, the abstract, points 1, 15 of the claims, junction I, L | 1-5,8-11,13-18 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 28 April 2014 (28.04.2014) | 26 June 2014 (26.06.2014) |
| Name and mailing address of the ISA/ RU | Authorized officer |
| Facsimile No. | Telephone No. |

Form PCT/ISA/210 (second sheet) (April 2005)

**INTERNATIONAL SEARCH REPORT**

International application No.
PCT/RU 2014/000015

C (Continuation). DOCUMENTS CONSIDERED TO BE RELEVANT

| Category* | Citation of document, with indication, where appropriate, of the relevant passages | Relevant to claim No. |
|---|---|---|
| Y | US 2009/0264300 A1 (NUEVOLUTION A/S) 22.10.2009, the abstract, par. [0107], [0159], [1129]-[0132], [1157], [1159]-[1160], [0716], [0940], table 1.5A, 1.5B | 11-18 |
| Y | WO 2010/081173 A2 (CYTOMX THERAPEUTICS, LLC et al.) 15.07.2010, points 1, 26 of the claims, par. [00011], [00012], [00139], [00140], [00226]-[00228], [00340], [00341] | 11-18 |
| A | US 2011/0124599 A1 (RIGEL PHARMACEUTICALS, INC.) 26.05.2011, the abstract, par. [0013]-[0019], [0040], [0176], [0177], [0042], [0046] -[0066], points 1, 8, 16 of the claims | 11-18 |

Form PCT/ISA/210 (continuation of second sheet) (April 2005)

INTERNATIONAL SEARCH REPORT

International application No.
PCT/RU 2014/000015

***C07D 281/16*** *(2006.01)*
***C07D 495/14*** *(2006.01)*
***C07K 14/555*** *(2006.01)*
***C12P 21/08*** *(2006.01)*
***A61K 31/554*** *(2006.01)*
***A61K 31/5517*** *(2006.01)*
***A61K 38/02*** *(2006.01)*
***A61P 35/00*** *(2006.01)*
***A61P 37/00*** *(2006.01)*

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4975278 A
- WO 201169074 A
- US 20120003248 A
- WO 2005044859 A
- EA 015009


### Non-patent literature cited in the description


- **GRILLO-LOPEZ A.-J et al.** *Semin. Oncol.,* 1999, vol. 26, 66-73
- **PAYNE, G.** *Cancer Cell,* 2003, vol. 3, 207-212
- **TRAIL et al.** *Cancer Immunol. Immunother.,* 2003, vol. 52, 328-337
- **SYRIGOS ; EPENETOS.** *Anticancer Research,* 1999, vol. 19, 605-614
- **NICULESCU-DUVAZ ; SPRINGER.** *Adv. Drug Del. Rev.,* 1997, vol. 26, 151-172
- **BERGE S.M. et al.** Pharmaceutical Salts. *J.Pharm.Sci.,* 1977, vol. 66, 1-19
- **R. L. FERRIS et al.** *J. Clinical Oncology,* 01 October 2010, vol. 28 (28), 4390-4399
- **K.-H. HEIDER et al.** *Blood,* 2011, vol. 118, 4159-4168
- **J. A. BOWELS et al.** *Blood,* 2006, vol. 108, 2648-2654